# EUROPEAN PATENT APPLICATION

(11) **EP 4 803 621 A2**
(43) Date of publication of application: **09.09.2026**
(21) Application number: 26173120.2
(22) Date of filing: 02.05.2016
(51) Int. Cl.: C12N 9/16

(54) **PRECISE DELETION OF CHROMOSOMAL SEQUENCES IN VIVO AND TREATMENT OF NUCLEOTIDE REPEAT EXPANSION DISORDERS USING ENGINEERED NUCLEASES**

(30) Priority: 01.05.2015 US 201562155838 P
(62) Document number(s) of the earlier application(s) in accordance with Art. 76 EPC: 21208516.1 / 4 015 633; 16789905.3 / 3 289 076
(71) Applicant: Precision Biosciences, Inc., Durham, NC 27701 (US)
(72) Inventor: SMITH, James, Jefferson, Morrisville 27560 (US); BARTSEVICH, Victor, Cary 27519 (US); JANTZ, Derek, Durham 27703 (US)
(74) Representative: Grund, Martin

(57) **Abstract**

The present invention provides a method of treating a nucleotide repeat expansion disorder comprising delivering a pair of engineered nucleases, or genes encoding engineered nucleases, to the cells of a patient such that the two nucleases excise the nucleotide repeat responsible for the disease permanently from the genome. The invention provides a general method for treating nucleotide repeat expansion disorders and engineered nucleases suitable for practicing the method. The invention further provides vectors and techniques for delivering engineered nucleases to patient cells.

## Description

### CROSS-REFERENCES TO RELATED APPLICATIONS

This patent application claims the benefit of the earlier filing date of U.S. Patent Application No. 62/155,838, filed on May 1, 2015, the contents of which are incorporated by reference herein in its entirety.

### FIELD OF THE INVENTION

The invention relates to the field of molecular biology and recombinant nucleic acid technology. In particular, the invention relates to a method of treating a patient with a nucleotide repeat expansion disorder comprising the removal of the nucleotide repeat region using engineered nucleases.

### BACKGROUND OF THE INVENTION

Nucleotide repeat expansion disorders, which are also known as "nucleotide repeat disorders" or "codon reiteration disorders," are a class of genetic diseases caused by excessive nucleotide repeats in the genome. Nucleotide repeats are common in the human genome and are not normally associated with disease. In some cases, however, the number of repeats expands beyond a stable threshold and can lead to disease, with the severity of symptoms generally correlated with the number of repeats.

Nucleotide repeat expansion disorders generally can be categorized as "polyglutamine" or "non-polyglutamine". Polyglutamine disorders, including Huntington's disease (HD) and several spinocerebellar ataxias, are caused by a CAG (glutamine) repeats in the protein-coding regions of specific genes. Non-polyglutamine disorders are more heterogeneous and can be caused by CAG trinucleotide repeat expansions in non-coding regions, as in Myotonic Dystrophy, or by the expansion of trinucleotide repeats other than CAG that can be in coding or non-coding regions such as the CGG repeat expansion responsible for Fragile X Syndrome. Although many nucleotide repeat expansion disorders involve trinucleotide repeats, some disorders are characterized by repeats of different lengths. For example, Type II myotonic dystrophy (DM2) is caused by a CCTG tetranucleotide repeat expansion in intron 1 of the *ZNF9* gene on chromosome 3. Amyotrophic lateral sclerosis (ALS) and frontotemporal dementia (FTD) are strongly associated with an expansion of the hexanucleotide repeat of GGGGCC in the first intron of the *C9ORF72* gene (between exons 1a and 1b) located on chromosome 9p21. The most common nucleotide repeat expansion disorders are summarized in Table 1.

**Table 1. Common Nucleotide Repeat Expansion Disorders**

| Type | Gene | Nucleotide Repeat | Normal Repeats | Pathogenic Repeats |
|---|---|---|---|---|
| DRPLA (Dentatorubropallidoluysian atrophy) | ATN1 or DRPLA | CAG | 6-35 | 49-88 |
| HD (Huntington's disease) | HTT (Huntingtin) | CAG | 6-35 | 36-250 |
| SBMA (Spinal and bulbar muscular atrophy) | AR | CAG | 9-36 | 38-62 |
| SCA1 (Spinocerebellar ataxia Type 1) | ATXN1 | CAG | 6-35 | 49-88 |
| SCA2 (Spinocerebellar ataxia Type 2) | ATXN2 | CAG | 14-32 | 33-77 |
| SCA3 (Spinocerebellar ataxia Type 3 or Machado-Joseph disease) | ATXN3 | CAG | 12-40 | 55-86 |
| SCA6 (Spinocerebellar ataxia Type 6) | CACNA1A | CAG | 4-18 | 21-30 |
| SCA7 (Spinocerebellar ataxia Type 7) | ATXN7 | CAG | 7-17 | 38-120 |
| SCA17 (Spinocerebellar ataxia Type 17) | TBP | CAG | 25-42 | 47-63 |
| FRAXA (Fragile X syndrome) | FMR1 | CGG | 6-53 | 230+ |
| FXTAS (Fragile X-associated tremor/ataxia syndrome) | FMR1 | CGG | 6-53 | 55-200 |
| FRAXE (Fragile XE mental retardation) | AFF2 or FMR2 | CCG | 6-35 | 200+ |
| FRDA (Friedreich's ataxia) | FXN | GAA | 7-34 | 100+ |
| DM1 (Myotonic dystrophy type I) | DMPK | CTG | 5-37 | 50+ |
| DM 2 (Myotonic dystrophy type II) | ZNF9 | CCTG | 11-26 | 75-11000 |
| SCA8 (Spinocerebellar ataxia Type 8) | OSCA or SCA8 | CTG | 16-37 | 110-250 |
| SCA12 (Spinocerebellar ataxia Type 12) | PPP2R2B or SCA12 | nnn On 5' end | 7-28 | 66-78 |
| ALS (Amyotrophic lateral sclerosis) and frontotemporal dementia (FTD) | C9ORF72 | GGGGCC | 20-30 | 100+ |

Nucleotide repeat expansion disorders are dynamic in the sense that the number of repeats can vary from generation-to-generation or even from cell-to-cell in the same individual. Repeat expansion is believed to be caused by polymerase "slipping" during DNA replication. Tandem repeats in the DNA sequence can "loop out" while maintaining complementary base pairing between the parent strand and daughter strands. If the loop structure is formed from the daughter strand, the number of repeats will increase. Conversely, if the loop structure is formed from the parent strand, the number of repeats will decrease. It appears that expansion is more common than reduction. In general, the larger the expansion is, the more likely it is to cause disease or increase the severity of disease. Thus, nucleotide repeat expansion disorders are subject to "anticipation," meaning the severity of symptoms and/or age of onset worsen through successive generations of affected families due to the expansion of these repeats from one generation to the next.

**Fragile X Syndrome.** Fragile X syndrome, also known as Martin-Bell syndrome or Escalante's syndrome, is a genetic disorder resulting from an expansion of the CGG trinucleotide repeat in the *FMR1* gene on the X chromosome. It results in a variety of intellectual disabilities ranging from mild to severe as well as physical characteristics such as an elongated face, large or protruding ears, and large testes (macroorchidism), and behavioral characteristics such as stereotypic movements (*e.g.*, hand-flapping) and social anxiety. Nearly half of the children with fragile X syndrome meet the criteria for a diagnosis of autism. The expanded CGG trinucleotide repeat responsible for Fragile X syndrome is located in the 5' untranslated region (UTR) of the *FMR1* gene which encodes the fragile X mental retardation protein (FMRP), which is required for normal neural development. In unaffected individuals, the *FMR1* gene has 6-53 repeats of the CGG codon, most commonly 29 or 30 repeats (Santoro et al. (2012), Annu. Rev. Pathol. Mech. Dis. 7:219-45). Alleles with 54-200 repeats are generally non-symptomatic but are considered to be indicative of a familial predisposition to the disease. Individuals affected with fragile X syndrome generally have >200 repeats of the CGG codon, which results in methylation of the *FMR1* promoter, silencing of the gene, and a failure to produce FMR1 protein. FMR1 is an mRNA-binding protein that is highly expressed in brain and testes. It is believed to be responsible for transporting certain mRNAs from the nucleus to neuronal synapses. In the absence of FMR1 protein, synapses do not form appropriately, leading to decreased cognitive capacity and developmental impairment. The incidence of fragile X syndrome is about 1 in every 3600 males and 1 in 4000-6000 females, with approximately 98% of cases attributable to an expanded CGG trinucleotide repeat in the 5'UTR and the remainder due to mutations in the *FMR1* gene itself.

**Huntington's Disease.** Huntington's disease (HD) is a neurodegenerative disorder caused by the expansion of a CAG trinucleotide repeat in the Huntingtin (*HTT*) gene. Symptoms of the disease vary between individuals, but usually involve a gradual decline in neuromuscular function. Symptoms can begin at any age but most frequently begin between ages 35 and 44. It has been observed that symptoms typically begin earlier in life with successive generations due to the increase in trinucleotide repeat number from one generation to the next. The earliest symptoms are often subtle problems with mood, cognition, and/or muscle coordination. As the disease advances, uncoordinated body movements become more apparent, along with a decline in mental abilities and erratic behavioral symptoms. Physical abilities gradually worsen until movement becomes difficult and patients begin to exhibit dementia. Complications such as pneumonia, heart disease, and physical injury reduce life expectancy to around twenty years from the point that symptoms begin. About 6% of cases start before the age of 21 years with an akinetic-rigid syndrome. The fast-progressing variant is classified as juvenile, akinetic-rigid, or Westphal variant HD.

The *HTT* gene is located on the short arm of chromosome 4. The N-terminal region of the HTT protein contains a tract of glutamine (Q) amino acids encoded by the CAG trinucleotide. Unaffected individuals have 6-35 repeats of the trinucleotide whereas affected individuals typically have >35 repeats, with higher numbers of repeats associated with increased severity and earlier onset of disease. The HTT protein is expressed at the highest levels in neurons and testes. The exact function of the protein is unknown, as is the mechanism by which the CAG trinucleotide repeat affects function and can cause disease. The mutant (expanded) form of the gene acts in a dominant manner. Thus, symptoms are not attributed to the production of an insufficient amount of the HTT protein but, rather, to the presence of a toxic form of the protein with an expanded polyglutamine tract (Walker (2007), Lancet 369:218-28). Early effects of HD include the significant loss of neurons in the basal ganglia. Other affected area of the brain include the substantia nigra, layers 3, 5 and 6 of the cerebral cortex, the hippocampus, purkinje cells in the cerebellum, lateral tuberal nuclei of the hypothalamus and parts of the thalamus, all of which exhibit reductions in size due to neuron loss. Striatal spiny neurons appear to be the most susceptible to HD, particularly those with projections towards the external globus pallidus.

**Friedreich's Ataxia.** Friedreich's ataxia (FA) is an autosomal recessive inherited disease that results from expansion of an intronic GAA trinucleotide repeat in the frataxin (*FXN*) gene. The disease presents initially as poor coordination but causes progressive damage to the nervous system until the patient requires a wheelchair for mobility. FA affects motor control but does not affect cognition. Its incidence in the general population is roughly 1 in 50,000.

The disorder is caused by a GAA trinucleotide repeat expansion in the *FXN* gene encoding the mitochondrial protein frataxin. Frataxin is an iron-binding protein responsible for forming iron-sulfur clusters. Frataxin deficiency results in the failure to produce several iron-sulfur cluster-containing proteins involved in electron transport in the mitochondria, as well as the accumulation of toxic levels of iron. Cell damage associated with pathology occurs primarily in the spinal cord and peripheral nerves. This includes sclerosis and degeneration of dorsal root ganglion, spinocerebellar tracts, lateral corticospinal tracts, and posterior columns (Marmolino (2011), Brain Research Reviews 67:311-330).

The *FXN* gene is located on Chromosome 9. The GAA trinucleotide repeat associated with FA is located in the first intron of the gene. Unaffected individuals typically have 7-34 copies of the repeat whereas affected individuals frequently have >100 copies. Because the repeat is not located in the coding sequence, it does not affect the amino acid sequence of the protein. Rather, an elevated number of repeats results in gene silencing through the formation of heterochromatin (Delatyckik et al. (2000), J. Med. Genet. 37:1-8). This reduces the expression level of the *FXN* gene and the extent of gene silencing increases with GAA repeat number.

**Myotonic Dystrophy.** Myotonic dystrophy (MD) is an autosomal-dominant form of muscular dystrophy. It is characterized by wasting of the muscles, cataracts, heart conduction defects, endocrine changes, and myotonia. There are two main types of MD. Type 1 (DM1), which is also called Steinert disease, has a severe congenital form and an adult-onset form. Type 2 (DM2), also called proximal myotonic myopathy, is rarer than DM1 and generally manifests with milder signs and symptoms. Both forms of the disease can present in patients of any age.

DM1 is caused by a CAG trinucleotide repeat expansion in the 3' UTR of the *DMPK* gene encoding dystrophia myotonica-protein kinase. The exact cellular function of the protein is unknown but it is expressed in muscle, heart, and brain tissues. Because the CAG trinucleotide repeat expansion is in a non-coding portion of the gene, it does not affect the amino acid sequence of the protein. Rather, the expanded repeat in the untranslated portion of the mRNA appears to cause *DMPK* mRNA and mRNA-binding/processing proteins to aggregate in the nucleus of the cell leading to cellular dysfunction and death. It is not known if cytotoxicity is attributable directly to mRNA aggregation or if, more likely, aggregating *DMPK* transcripts sequester mRNA splicing factors resulting in the mis-splicing of other pre-mRNAs. Alternatively, or in addition, the CAG repeat expansion in the *DMPK* gene may alter the expression of neighboring genes. *DMPK* is located in a gene-rich region of Chromosome 19.

DM2 is caused by a CCTG tetranucleotide repeat expansion in Intron 1 of the *ZNF9* gene on Chromosome 3. DM2 is a *tetra*nucleotide repeat expansion disorder. *ZNF9* encodes zinc-finger protein-9, an RNA-binding protein involved in regulating a number of genes involved in cholesterol homeostasis. The *ZNF9* repeat expansion is in a non-coding region that does not change the amino acid sequence of the protein. Like DM1, the pathogenesis of DM2 is generally attributed to mRNA aggregation, which is believed to sequester splicing factors necessary for normal cellular function.

**Amyotrophic Lateral Sclerosis and Frontotemporal Dementia.** The occurrence of amyotrophic lateral sclerosis (ALS) and frontotemporal dementia (FTD) is strongly associated with a *hexa*nucleotide repeat expansion of GGGGCC in the first intron of the *C9ORF72* gene (between exons 1a and 1b) located on chromosome 9p21. The function of the protein encoded by *C9ORF72* is largely unknown, but it has been predicted to function as a guanine nucleotide exchange factor for small GTPases. Unaffected individuals typically have up to 30 hexanucleotide repeats in *C9ORF72*, whereas affected individuals can have hundreds of repeats. Although the repeat expansion is present in a non-coding portion of the gene, the mutation interferes with normal expression of the protein made by *C9ORF72* and can result in the formation of nuclease RNA foci.

The use of engineered meganucleases for the treatment of nucleotide repeat expansion disorders was previously disclosed in WO 2004/067753. In that publication, the authors disclose the possibility of using an engineered meganuclease to target a double-strand DNA break to a trinucleotide repeat such that it promotes homologous recombination between the two chromosomes (interchromosomal recombination) or between individual repeats on the same chromosome (intrachromosomal recombination). The latter approach, which consists of targeting a DNA break to the repeat itself (or immediately adjacent to the repeat) such that individual trinucleotides recombine with one another to effectively reduce the number of repeats, has been demonstrated in experimental systems using zinc-finger nucleases and TALENs (Richard (2015), Trends Genet. 31(4): 177-86). These previously disclosed methods involve a single DNA break induced by a single endonuclease and require homologous recombination to correct the disorder. As homologous recombination is not the dominant mechanism of DNA break repair in many cell types, the previously disclosed methods are extremely inefficient and are, therefore, unlikely to have significant clinical benefit because the gene is not "corrected" in enough cells. Therefore, there remains a need for methods to treat nucleotide repeat expansion disorders that do not require homologous recombination.

### SUMMARY OF THE INVENTION

The present invention depends, in part, upon the recognition and application of the fact that double-strand breaks at two loci in mammalian chromosomes can efficiently promote repair of the chromosome with resultant deletion of the intervening sequence. The mechanism is distinct from that used in prior art methods, in which a single double-stranded break has been used in conjunction with provision of a donor sequence lacking the desired deletion, such that DNA repair mediated by homologous recombination between the cleaved chromosome and the donor sequence would result in deletion of the desired sequence in the chromosome, Furthermore, the present invention depends, in part, upon the recognition and application of the fact that the generation of two double-stranded breaks with complementary 3' overhangs can result in a relatively high percentage of precise deletions of the intervening sequences, apparently independent of homologous recombination with a sister chromosome or donor sequence, without exonuclease degradation of the overhangs, and bypassing mechanisms such as non-homologous end-joining.

Thus, the present invention provides treatments for nucleotide repeat expansion disorders in which the region of the chromosome comprising the disease-causing repeat expansion is excised from the genome using a pair of site-specific endonucleases. We have shown that genomic DNA sequences, including trinucleotide, hexanucleotide, and tetranucleotide repeat expansion sequences, can be deleted from the genome by introducing a pair of engineered endonucleases that recognize and cut DNA sites flanking the sequence of interest. The fragment of a chromosome between such a pair of targeted DNA-breaks is lost from the genome, resulting in the elimination of the disease-causing sequence. The resulting cell, and its progeny, will have a normal phenotype due to the elimination of the expanded repeat.

The invention can utilize site-specific, rare-cutting endonucleases that are engineered to recognize DNA sequences in the locus of interest. Methods for producing engineered, site-specific endonucleases are known in the art. For example, zinc-finger nucleases (ZFNs) can be engineered to recognize and cut pre-determined sites in a genome. ZFNs are chimeric proteins comprising a zinc finger DNA-binding domain fused to the nuclease domain of the FokI restriction enzyme. The zinc finger domain can be redesigned through rational or experimental means to produce a protein which binds to a pre-determined DNA sequence ~18 basepairs in length. By fusing this engineered protein domain to the FokI nuclease, it is possible to target DNA breaks with genome-level specificity. ZFNs have been used extensively to target gene addition, removal, and substitution in a wide range of eukaryotic organisms (reviewed in Durai et al. (2005), Nucleic Acids Res. 33, 5978). Likewise, TAL-effector nucleases (TALENs) can be generated to cleave specific sites in genomic DNA. Like a ZFN, a TALEN comprises an engineered, site-specific DNA-binding domain fused to the FokI nuclease domain (reviewed in Mak et al. (2013), Curr. Opin. Struct. Biol. 23:93-9). In this case, however, the DNA binding domain comprises a tandem array of TAL-effector domains, each of which specifically recognizes a single DNA basepair. A limitation that ZFNs and TALENs have for the practice of the current invention is that they are heterodimeric, so that the production of a single functional nuclease in a cell requires co-expression of two protein monomers. Because the current invention requires *two* nucleases, one to cut on either side of the nucleotide repeat, this would necessitate co-expressing *four* ZFN or TALEN monomers in the same cell. This presents significant challenges in gene delivery because traditional gene delivery vectors have limited carrying capacity. It also introduces the possibility of "mis-dimerization" in which the monomers associate inappropriately to make unintended dimeric endonuclease species that might recognize and cut off-target locations in the genome. This can, potentially, be minimized by generating orthogonal obligate heterodimers in which the FokI nuclease domains of the four monomers are differentially engineered to dimerize preferentially with the intended partner monomer.

Compact TALENs are an alternative endonuclease architecture that avoids the need for dimerization (Beurdeley et al. (2013), Nat. Commun. 4:1762). A Compact TALEN comprises an engineered, site-specific TAL-effector DNA-binding domain fused to the nuclease domain from the I-TevI homing endonuclease. Unlike FokI, I-TevI does not need to dimerize to produce a double-strand DNA break so a Compact TALEN is functional as a monomer. Thus, it is possible to co-express two Compact TALENs in the same cell to practice the present invention.

Engineered endonucleases based on the CRISPR/Cas9 system are also know in the art (Ran et al. (2013), Nat. Protoc. 8:2281-2308; Mali et al. (2013), Nat. Methods 10:957-63). A CRISPR endonuclease comprises two components: (1) a caspase effector nuclease, typically microbial Cas9; and (2) a short "guide RNA" comprising a ~20 nucleotide targeting sequence that directs the nuclease to a location of interest in the genome. By expressing multiple guide RNAs in the same cell, each having a different targeting sequence, it is possible to target DNA breaks simultaneously to multiple sites in in the genome. Thus, CRISPR/Cas9 nucleases are suitable for the present invention. The primary drawback of the CRISPR/Cas9 system is its reported high frequency of off-target DNA breaks, which could limit the utility of the system for treating human patients (Fu et al. (2013), Nat. Biotechnol. 31:822-6).

In the preferred embodiment of the invention, the DNA break-inducing agent is an engineered homing endonuclease (also called a "meganuclease"). Homing endonucleases are a group of naturally-occurring nucleases which recognize 15-40 base-pair cleavage sites commonly found in the genomes of plants and fungi. They are frequently associated with parasitic DNA elements, such as group 1 self-splicing introns and inteins. They naturally promote homologous recombination or gene insertion at specific locations in the host genome by producing a double-stranded break in the chromosome, which recruits the cellular DNA-repair machinery (Stoddard (2006), Q. Rev. Biophys. 38: 49-95). Homing endonucleases are commonly grouped into four families: the LAGLIDADG family, the GIY-YIG family, the His-Cys box family and the HNH family. These families are characterized by structural motifs, which affect catalytic activity and recognition sequence. For instance, members of the LAGLIDADG family are characterized by having either one or two copies of the conserved LAGLIDADG motif (see Chevalier et al. (2001), Nucleic Acids Res. 29(18): 3757-3774). The LAGLIDADG homing endonucleases with a single copy of the LAGLIDADG motif form homodimers, whereas members with two copies of the LAGLIDADG motif are found as monomers.

I-CreI (SEQ ID NO: 1) is a member of the LAGLIDADG family of homing endonucleases which recognizes and cuts a 22 basepair recognition sequence in the chloroplast chromosome of the algae *Chlamydomonas reinhardtii*. Genetic selection techniques have been used to modify the wild-type I-CreI cleavage site preference (Sussman et al. (2004), J. Mol. Biol. 342: 31-41; Chames et al. (2005), Nucleic Acids Res. 33: e178; Seligman et al. (2002), Nucleic Acids Res. 30: 3870-9, Arnould et al. (2006), J. Mol. Biol. 355: 443-58). More recently, a method of rationally-designing mono-LAGLIDADG homing endonucleases was described which is capable of comprehensively redesigning I-CreI and other homing endonucleases to target widely-divergent DNA sites, including sites in mammalian, yeast, plant, bacterial, and viral genomes (WO 2007/047859).

As first described in WO 2009/059195, I-CreI and its engineered derivatives are normally dimeric but can be fused into a single polypeptide using a short peptide linker that joins the C-terminus of a first subunit to the N-terminus of a second subunit (Li et al. (2009), Nucleic Acids Res. 37:1650-62; Grizot et al. (2009), Nucleic Acids Res. 37:5405-19). Thus, a functional "single-chain" meganuclease can be expressed from a single transcript. By delivering genes encoding two different single-chain meganucleases to the same cell, it is possible to simultaneously cut two different sites. This, coupled with the extremely low frequency of off-target cutting observed with engineered meganucleases makes them a preferred endonuclease for the present invention.

Thus, in one aspect, the invention provides a method for promoting precise deletion of a locus flanked by a pair of direct repeat sequences in a chromosome in a population of eukaryotic cells, by (a) introducing into the cells a first engineered nuclease protein and a second engineered nuclease protein; or (b) introducing into the cells at least a first nucleic acid encoding the first engineered nuclease and a second nucleic acid encoding the second engineered nuclease, wherein the first engineered nuclease and the second engineered nuclease are expressed in the cells *in vivo*; wherein the pair of direct repeat sequences includes a first direct repeat sequence 5' of the locus on a first DNA strand of the chromosome and a second direct repeat sequence 3' of the locus on the first DNA strand of the chromosome, and each of the first direct repeat sequence and the second direct repeat sequence consists of the same nucleotide sequence of 2-4 basepairs; wherein the first engineered nuclease recognizes the first direct repeat sequence and cleaves the first strand of the chromosome at either the 3' or 5' end of the first direct repeat sequence on the first strand, and cleaves the second strand of the chromosome at either the 3' or 5' end of the first direct repeat sequence on the second strand, thereby producing either a first 3' or 5' overhang; wherein the second engineered nuclease recognizes the second direct repeat sequence and cleaves the first strand of the chromosome at either the 3' or 5' end of the second direct repeat sequence on the first strand, and cleaves the second strand of the chromosome at either the 3' or 5' end of the second direct repeat sequence on the second strand, thereby producing either a second 3' or 5' overhang which is complementary to the first 3' or 5' overhang; and wherein ligation of the first 3' or 5' overhang to the second 3' or 5' overhang promotes a precise deletion of the locus between the pair of direct repeats. For the avoidance of doubt, any given nuclease will always cut at only one of the 3' or 5' end of the direct repeat, and will always produce only one of 3' or 5' overhangs. However, some nucleases will always cut at 3' ends and produce 3' overhangs whereas other nucleases will always cut at 5' ends and produce 5' overhangs. In order to promote precise deletions, it is necessary for the first nuclease and second nuclease to produce complementary overhangs of equal length.

In some embodiments, the first and/or second engineered nuclease is an engineered meganuclease, single chain meganuclease, compact TALEN, CRISPR, or zinc finger nuclease. Thus, in some embodiments, the first and/or second nuclease is an engineered LAGLIDADG meganuclease or single chain LAGLIDADG meganuclease and cleavage of the chromosome produces a 4 basepair 3' overhang. In other embodiments, the first and/or second nuclease is an engineered GIY-YIG meganuclease and cleavage of the chromosome produces a 2 basepair 3' overhang. In other embodiments, the first and/or second nuclease is an engineered compact TALEN and cleavage of the chromosome produces a 2 basepair 3' overhang. In other embodiments, the first and/or second nuclease is an engineered CRISPR and cleavage of the chromosome produces a 2-4 basepair 5' overhang.

In some embodiments, ligation of the first 3' or 5' overhang and the second 3' or 5' overhang promotes a precise deletion of the locus between the pair of direct repeats in at least 20%, 25%, 30%, 35% or 40% of the population of cells.

In some embodiments, one or both ends of the deleted sequence is present in an exon, and the precise deletion results in deletion of a total length of exon sequences which is a multiple of three basepairs.

In some embodiments, the locus to be precisely deleted includes a nucleotide repeat expansion which causes a nucleotide repeat expansion disorder. In some embodiments the nucleotide repeat expansion disorder is chosen from Dentatorubropallidoluysian atrophy (DRPLA), Huntington's disease (HD), Spinal and bulbar muscular atrophy (SBMA), Spinocerebellar ataxia Type 1 (SCA1), Spinocerebellar ataxia Type 2 (SCA2), Spinocerebellar ataxia Type 3 or Machado-Joseph disease (SCA3), Spinocerebellar ataxia Type 6 (SCA6), Spinocerebellar ataxia Type 7 (SCA7), Spinocerebellar ataxia Type 17 (SCA17), Fragile X syndrome (FRAXA), Fragile X-associated tremor/ataxia syndrome (FXTAS), Fragile XE mental retardation (FRAXE), Friedreich's ataxia (FRDA), Myotonic dystrophy type I (DM1), Myotonic dystrophy type II (DM 2), Spinocerebellar ataxia Type 8 (SCA8), Spinocerebellar ataxia Type 12 (SCA12), Amyotrophic lateral sclerosis (ALS) and frontotemporal dementia (FTD).

In some embodiments, one or both of the pair of direct repeats is included in one of the recognition sequences disclosed herein. In addition, in some embodiments, one or both of the first engineered nuclease and second engineered nuclease is one of the engineered meganuclease disclosed herein.

Thus, in one aspect, the present invention provides a method of treating a nucleotide repeat expansion disorder comprising delivering a pair of engineered nucleases, or nucleic acids encoding a pair of engineered nucleases, to the cells of a patient such that the two nucleases excise the nucleotide repeat expansion responsible for the disease permanently from the genome. Thus, the invention provides a general method for treating nucleotide repeat expansion disorders, as well as engineered nucleases suitable for practicing the method. The invention also provides vectors and techniques for delivering engineered nucleases to patient cells.

In another aspect, the present invention provides engineered nucleases, or nucleic acids encoding engineered nucleases, that excise a nucleotide repeat expansion responsible for a nucleotide repeat expansion disorder permanently from the genome. In some embodiments, the engineered nuclease is an engineered meganuclease or engineered single chain meganuclease. In other embodiments, the engineered nuclease is a zinc finger nuclease, TALEN, compact TALEN, CRISPR, or megaTAL nuclease.

In another aspect, the invention provides engineered nucleases that recognize and cleave a recognition sequence within intron 1 of the human frataxin (*FXN*) gene. In some embodiments, the engineered nuclease is an engineered meganuclease or engineered single chain meganuclease. In other embodiments, the engineered nuclease is a zinc finger nuclease, TALEN, compact TALEN, CRISPR, or megaTAL nuclease.

In some embodiments, the recognition sequence is positioned 5' upstream of a GAA repeat region in said intron 1 (SEQ ID NO: 74). In such embodiments, the recognition sequence can comprise any one of SEQ ID NOs: 12-73. In particular embodiments, the recognition sequence comprises SEQ ID NO: 29. In some particular embodiments, the recognition sequence comprises SEQ ID NO: 63. In some such embodiments, the engineered nuclease is an engineered meganuclease or engineered single chain meganuclease. In some particular embodiments, the engineered meganuclease comprises SEQ ID NO: 133 or 134. In other such embodiments, the engineered nuclease is a zinc finger nuclease, TALEN, compact TALEN, CRISPR, or megaTAL nuclease.

In some embodiments, the recognition sequence is positioned 3' downstream of a GAA repeat region in said intron 1 (SEQ ID NO: 96). In some such embodiments, the recognition sequence can comprise any one of SEQ ID NOs: 75-95. In particular embodiments, the recognition sequence comprises SEQ ID NO: 89. In some embodiments, the engineered nuclease is an engineered meganuclease or engineered single chain meganuclease. In some embodiments, the engineered meganuclease comprises SEQ ID NO: 135. In other embodiments, the engineered nuclease is a zinc finger nuclease, TALEN, compact TALEN, CRISPR, or megaTAL nuclease.

In another aspect, the invention provides engineered nucleases that recognize and cleave a recognition sequence within the 5' untranslated (UTR) region of the human *FMR1* gene. In some embodiments, the engineered nuclease is an engineered meganuclease or engineered single chain meganuclease. In other embodiments, the engineered nuclease is a zinc finger nuclease, TALEN, compact TALEN, CRISPR, or megaTAL nuclease.

In some embodiments, the recognition sequence is positioned 5' upstream of a CGG repeat region in said 5' UTR region (SEQ ID NO: 3). In one such embodiment, the recognition sequence can comprise SEQ ID NO: 2. In some such embodiments, the engineered nuclease is an engineered meganuclease or engineered single chain meganuclease. In other such embodiments, the engineered nuclease is a zinc finger nuclease, TALEN, compact TALEN, CRISPR, or megaTAL nuclease.

In some embodiments, the recognition sequence is positioned 3' downstream of a CGG repeat region in the 5' UTR region. In such embodiments, the recognition sequence can comprise SEQ ID NO: 4. In some such embodiments, the engineered nuclease is an engineered meganuclease or engineered single chain meganuclease. In other such embodiments, the engineered nuclease is a zinc finger nuclease, TALEN, compact TALEN, CRISPR, or megaTAL nuclease.

In another aspect, the invention provides engineered nucleases that recognize and cleave a recognition sequence within exon 1 of the human *HTT* gene. In some embodiments, the engineered nuclease is an engineered meganuclease or engineered single chain meganuclease. In other embodiments, the engineered nuclease is a zinc finger nuclease, TALEN, compact TALEN, CRISPR, or megaTAL nuclease.

In some embodiments, the recognition sequence is positioned 5' upstream of a CAG repeat region in exon 1 (SEQ ID NO: 7). In such embodiments, the recognition sequence can comprise any one of SEQ ID NOs: 5, 6, or 141. In some such embodiments, the engineered nuclease is an engineered meganuclease or engineered single chain meganuclease. In other such embodiments, the engineered nuclease is a zinc finger nuclease, TALEN, compact TALEN, CRISPR, or megaTAL nuclease.

In some embodiments, the recognition sequence is positioned 3' downstream of a CAG repeat region in exon 1 (SEQ ID NO: 11). In such embodiments, the recognition sequence can comprise any one of SEQ ID NOs: 8-10. In some such embodiments, the engineered nuclease is an engineered meganuclease or engineered single chain meganuclease. In other such embodiments, the engineered nuclease is a zinc finger nuclease, TALEN, compact TALEN, CRISPR, or megaTAL nuclease.

In another aspect, the invention provides engineered nucleases that recognize and cleave a recognition sequence within the 3' UTR region of the human *DMPK* gene. In some embodiments, the engineered nuclease is an engineered meganuclease or engineered single chain meganuclease. In other embodiments, the engineered nuclease is a zinc finger nuclease, TALEN, compact TALEN, CRISPR, or megaTAL nuclease.

In some embodiments, the recognition sequence is positioned 5' upstream of a CTG repeat region in the 3' UTR region (SEQ ID NO: 102). In some such embodiments, the recognition sequence can comprise any one of SEQ ID NOs: 97-101. In some such embodiments, the engineered nuclease is an engineered meganuclease or engineered single chain meganuclease. In other such embodiments, the engineered nuclease is a zinc finger nuclease, TALEN, compact TALEN, CRISPR, or megaTAL nuclease.

In some embodiments, the recognition sequence is positioned 3' downstream of a CTG repeat region in the 3' UTR region (SEQ ID NO: 132). In some such embodiments, the recognition sequence can comprise any one of SEQ ID NOs: 103-131. In some embodiments, the engineered nuclease is an engineered meganuclease or engineered single chain meganuclease. In other embodiments, the engineered nuclease is a zinc finger nuclease, TALEN, compact TALEN, CRISPR, or megaTAL nuclease.

In another aspect, the invention provides engineered nucleases that recognize and cleave a recognition sequence within intron 1 of the human *ZNF9* gene. In some embodiments, the engineered nuclease is an engineered meganuclease or engineered single chain meganuclease. In other embodiments, the engineered nuclease is a zinc finger nuclease, TALEN, compact TALEN, CRISPR, or megaTAL nuclease.

In some embodiments, the recognition sequence is positioned 5' upstream of a CCTG tetranucleotide repeat in intron 1. In other embodiments, the recognition sequence is positioned 3' downstream of a CCTG tetranucleotide repeat in intron 1. In some such embodiments, the engineered nuclease is an engineered meganuclease or engineered single chain meganuclease. In other such embodiments, the engineered nuclease is a zinc finger nuclease, TALEN, compact TALEN, CRISPR, or megaTAL nuclease.

In another aspect, the invention provides isolated polynucleotides comprising a nucleic acid sequence encoding an engineered nuclease of the invention. In some embodiments, the isolated polynucleotide is an mRNA. In some embodiments, the engineered nuclease is an engineered meganuclease or engineered single chain meganuclease, a zinc finger nuclease, TALEN, compact TALEN, CRISPR, or megaTAL nuclease as described herein, or which recognizes and cleaves at least one of the recognition sequences described herein.

In another aspect, the invention provides a recombinant DNA construct comprising a nucleic acid sequence encoding an engineered nuclease of the invention. In some embodiments, the engineered nuclease is an engineered meganuclease or engineered single chain meganuclease, a zinc finger nuclease, TALEN, compact TALEN, CRISPR, or megaTAL nuclease as described herein, or which recognizes and cleaves at least one of the recognition sequences described herein.

In another aspect, the invention provides a viral vector comprising a nucleic acid sequence encoding an engineered nuclease of the invention. In some embodiments, the viral vector is a recombinant adeno-associated virus (AAV) vector. In some embodiments, the engineered nuclease is an engineered meganuclease or engineered single chain meganuclease, a zinc finger nuclease, TALEN, compact TALEN, CRISPR, or megaTAL nuclease as described herein, or which recognizes and cleaves at least one of the recognition sequences described herein.

In another aspect, the invention provides methods for treating a subject having a nucleotide repeat expansion disorder characterized by expansion of a nucleotide repeat in a gene of interest, the method comprising delivering to target cells in the subject: (a) at least a first engineered nuclease protein and a second engineered nuclease protein; or (b) at least a first nucleic acid encoding the first engineered nuclease and a second nucleic acid encoding the second engineered nuclease, wherein the first nucleic acid and second nucleic acid are expressed to produce the first engineered nuclease and the second engineered nuclease in the target cells *in vivo*; wherein the first engineered nuclease recognizes and cleaves a first recognition sequence positioned 5' upstream of the nucleotide repeat in the gene of interest; and wherein the second engineered nuclease recognizes and cleaves a second recognition sequence positioned 3' downstream of the nucleotide repeat in the gene of interest; and wherein an intervening DNA fragment between the first recognition sequence and the second recognition sequence is excised and the number of the nucleotide repeat is reduced in the gene of interest.

In some embodiments of the methods, the engineered nuclease can be an engineered meganuclease or single chain meganuclease, a zinc finger nuclease, TALEN, compact TALEN, CRISPR, or megaTAL nuclease.

In some embodiments of the methods, the first engineered nuclease and the second engineered nuclease generate complementary overhangs which promote direct re-ligation of the gene of interest following excision of the intervening DNA fragment. In some embodiments, the overhangs are at least 2-4 basepairs and are 3' overhangs,

In some embodiments of the methods, the nucleotide repeat is a trinucleotide repeat. In some such embodiments of the method, the trinucleotide repeat is selected from the group consisting of CAG, CGG, CCG, GAA, and CTG.

In some embodiments of the methods, the nucleotide repeat expansion disorder is Friedreich's Ataxia, the trinucleotide repeat is GAA, and the gene of interest is the human frataxin (*FXN*) gene, wherein the trinucleotide repeat is positioned within intron 1 of the *FXN* gene. In some such embodiments of the method, the first recognition sequence is positioned 5' upstream of the trinucleotide repeat in intron 1 (SEQ ID NO: 74), and the second recognition sequence is positioned 3' downstream of the trinucleotide repeat in intron 1 (SEQ ID NO: 96). In some embodiments, the engineered nuclease is an engineered meganuclease or single chain meganuclease.

In some particular embodiments of the foregoing methods for treating Friedreich's Ataxia, the first recognition sequence can comprise any one of SEQ ID NOs: 12-73. In some particular embodiments, the first engineered nuclease is a meganuclease and can comprise SEQ ID NOs: 133 or 134. In other such particular embodiments, the second recognition sequence can comprise any one of SEQ ID NOs: 75-95. In some particular embodiments, the second engineered nuclease is a meganuclease and can comprise SEQ ID NO: 135. In particular embodiments, a first engineered meganuclease comprises SEQ ID NO: 133 and a second engineered meganuclease comprises SEQ ID NO: 135. In some particular embodiments, a first engineered meganuclease comprises SEQ ID NO: 134 and a second engineered meganuclease comprises SEQ ID NO: 135.

In some embodiments of the methods, the nucleotide repeat expansion disorder is Fragile X syndrome, the trinucleotide repeat is CGG, and the gene of interest is the human *FMR1* gene, wherein the trinucleotide repeat is positioned within the 5' UTR region of the *FMR1* gene. In some such embodiments of the methods, the first recognition sequence is positioned 5' upstream of the trinucleotide repeat in the 5' UTR region (SEQ ID NO: 3), and the second recognition sequence is positioned 3' downstream of the trinucleotide repeat in the 5' UTR region. In some such embodiments, the first recognition sequence can comprise SEQ ID NO: 2. In other such embodiments, the second recognition sequence comprises SEQ ID NO: 4. In some embodiments, the engineered nuclease is an engineered meganuclease or single chain meganuclease.

In some embodiments of the methods, the nucleotide repeat expansion disorder is Huntington's disease, the trinucleotide repeat is CAG, and the gene of interest is the human *HTT* gene, wherein the trinucleotide repeat is positioned within exon 1 of the *HTT* gene. In some such embodiments of the methods, the first recognition sequence is positioned 5' upstream of the trinucleotide repeat in exon 1 (SEQ ID NO: 7), and the second recognition sequence is positioned 3' downstream of the trinucleotide repeat in exon 1 (SEQ ID NO: 11). In particular embodiments, the first recognition sequence can comprise any one of SEQ ID NOs: 141, 5, or 6. In some particular embodiments, the second recognition sequence can comprise any one of SEQ ID NOs: 8-10. In some embodiments of the methods, the engineered nuclease is an engineered meganuclease or single chain meganuclease.

In some embodiments of the methods, the nucleotide repeat expansion disorder is Myotonic dystrophy type I (DM1), the trinucleotide repeat is CAG, and the gene of interest is the human *DMPK* gene, wherein the trinucleotide repeat is positioned within the 3' UTR region of the *DMPK* gene. In some such embodiments of the methods, the first recognition sequence is positioned 5' upstream of the trinucleotide repeat in the 3' UTR region (SEQ ID NO: 102), and the second recognition sequence is positioned 3' downstream of the trinucleotide repeat in the 3' UTR region (SEQ ID NO: 132). In some such embodiments of the methods, the first recognition sequence can comprise any one of SEQ ID NOs: 97-101. In other such embodiments of the methods, the second recognition sequence can comprise any one of SEQ ID NOs: 103-131.

In some embodiments of the methods, the nucleotide repeat is a tetranucleotide repeat. In some such embodiments, the nucleotide repeat expansion disorder is Myotonic dystrophy type II (DM2), the tetranucleotide repeat is CCTG, and the gene of interest is the human *ZNF9* gene, wherein the tetranucleotide repeat is positioned within intron 1 of the *ZNF9* gene. In some such embodiments of the methods, the first recognition sequence is positioned 5' upstream of the tetranucleotide repeat in intron 1, and the second recognition sequence is positioned 3' downstream of the tetranucleotide repeat in intron 1. In some embodiments, the engineered nuclease is an engineered meganuclease or a single chain meganuclease.

In some embodiments of the methods, the invention provides an engineered meganuclease that recognizes and cleaves a recognition sequence within intron 1 of the human frataxin (*FXN*) gene, wherein the engineered meganuclease comprises a first subunit and a second subunit, wherein the first subunit binds to a first recognition half-site of the recognition sequence and comprises a first hypervariable (HVR1) region, and wherein the second subunit binds to a second recognition half-site of the recognition sequence and comprises a second hypervariable (HVR2) region.

In some particular embodiments, the recognition sequence comprises SEQ ID NO: 34 (*i.e.*, the FXN 3-4 recognition sequence). In some such embodiments, the first subunit can comprise an amino acid sequence having at least 80%, at least 85%, at least 90%, at least 95%, or more, sequence identity to residues 7-153 of any one of SEQ ID NOs: 155-159, and/or the second subunit can comprise an amino acid sequence having at least 80%, at least 85%, at least 90%, at least 95%, or more, sequence identity to residues 198-344 of any one of SEQ ID NOs: 155-159. In some particular embodiments, the HVR1 region can comprise residues 24-79 of any one of SEQ ID NOs: 155-159. In some particular embodiments, the HVR2 region can comprise residues 215-270 of any one of SEQ ID NOs: 155-159. In some particular embodiments, the first subunit can comprise residues 7-153 of any one of SEQ ID NOs: 155-159. In some particular embodiments, the second subunit can comprise residues 198-344 of any one of SEQ ID NOs: 155-159. In some embodiments, the engineered nuclease is a single-chain meganuclease comprising a linker, wherein the linker covalently joins the first subunit and the second subunit. In some particular embodiments, the engineered meganuclease can comprise the amino acid sequence of any one of SEQ ID NOs: 155-159.

In some particular embodiments, the recognition sequence comprises SEQ ID NO: 89 (*i.e.*, the FXN 5-6 recognition sequence). In some such embodiments, the first subunit can comprise an amino acid sequence having at least 80%, at least 85%, at least 90%, at least 95%, or more, sequence identity to residues 198-344 of any one of SEQ ID NOs: 170-172, or residues 7-153 of SEQ ID NO: 173, and/or the second subunit can comprise an amino acid sequence having at least 80%, at least 85%, at least 90%, at least 95%, or more, sequence identity to residues 7-153 of any one of SEQ ID NOs: 170-172, or residues 198-344 of SEQ ID NO: 173. In some particular embodiments, the HVR1 region can comprise residues 215-270 of any one of SEQ ID NOs: 170-172, or residues 24-79 of SEQ ID NO: 173. In some particular embodiments, the HVR2 region can comprise residues 24-79 of any one of SEQ ID NOs: 170-172, or residues 215-270 of SEQ ID NO: 173. In some particular embodiments, the first subunit comprises residues 198-344 of any one of SEQ ID NOs: 170-172, or residues 7-153 of SEQ ID NO: 173. In some particular embodiments, the second subunit comprises residues 7-153 of any one of SEQ ID NOs: 170-172, or residues 198-344 of SEQ ID NO: 173. In some embodiments, the engineered nuclease is a single-chain meganuclease comprising a linker, wherein the linker covalently joins the first subunit and the second subunit. In some particular embodiments, the engineered meganuclease comprises the amino acid sequence of any one of SEQ ID NOs: 170-173

In some particular embodiments, the recognition sequence comprises SEQ ID NO: 29 (*i.e.*, the FXN 1-2 recognition sequence). In some such embodiments, the first subunit can comprise an amino acid sequence having at least 80%, at least 85%, at least 90%, at least 95%, or more, sequence identity to residues 7-153 of any one of SEQ ID NOs: 143-146, and/or the second subunit can comprise an amino acid sequence having at least 80%, at least 85%, at least 90%, at least 95%, or more, sequence identity to residues 198-344 of any one of SEQ ID NOs: 143-146. In some particular embodiments, the HVR1 region can comprise residues 24-79 of any one of SEQ ID NOs: 143-146. In some particular embodiments, the HVR2 region can comprise residues 215-270 of any one of SEQ ID NOs: 143-146. In some particular embodiments, the first subunit comprises residues 7-153 of any one of SEQ ID NOs: 143-146. In some particular embodiments, the second subunit comprises residues 198-344 of any one of SEQ ID NOs: 143-146. In some embodiments, the engineered nuclease is a single-chain meganuclease comprising a linker, wherein the linker covalently joins the first subunit and the second subunit. In some particular embodiments, the engineered meganuclease comprises the amino acid sequence of any one of SEQ ID NOs: 143-146.

In some particular embodiments, the recognition sequence comprises SEQ ID NO: 29 (*i.e.*, the FXN 11-12 recognition sequence). In some such embodiments, the first subunit can comprise an amino acid sequence having at least 80%, at least 85%, at least 90%, at least 95%, or more, sequence identity to residues 198-344 of any one of SEQ ID NOs: 182-185, and/or the second subunit can comprise an amino acid sequence having at least 80%, at least 85%, at least 90%, at least 95%, or more, sequence identity to residues 7-153 of any one of SEQ ID NOs: 182-185. In some particular embodiments, the HVR1 region can comprise residues 215-270 of any one of SEQ ID NOs: 182-185. In some particular embodiments, the HVR2 region can comprise residues 24-79 of any one of SEQ ID NOs: 182-185. In some particular embodiments, the first subunit comprises residues 198-344 of any one of SEQ ID NOs: 182-185. In some particular embodiments, the second subunit comprises residues 7-153 of any one of SEQ ID NOs: 182-185. In some embodiments, the engineered nuclease is a single-chain meganuclease comprising a linker, wherein the linker covalently joins the first subunit and the second subunit. In some particular embodiments, the engineered meganuclease comprises the amino acid sequence of any one of SEQ ID NOs: 182-185.

In some embodiments of the methods, the invention provides an engineered meganuclease that recognizes and cleaves a recognition sequence within the human *C9ORF72* gene, wherein the engineered meganuclease comprises a first subunit and a second subunit, wherein the first subunit binds to a first recognition half-site of the recognition sequence and comprises a first hypervariable (HVR1) region, and wherein the second subunit binds to a second recognition half-site of the recognition sequence and comprises a second hypervariable (HVR2) region.

In some particular embodiments, the recognition sequence comprises SEQ ID NO: 194 (*i.e.*, the ORF 7-8 recognition sequence). In some such embodiments, the first subunit can comprise an amino acid sequence having at least 80%, at least 85%, at least 90%, at least 95%, or more, sequence identity to residues 7-153 of any one of SEQ ID NOs: 198-200 or residues 198-344 of SEQ ID NO: 201, and/or the second subunit can comprise an amino acid sequence having at least 80%, at least 85%, at least 90%, at least 95%, or more, sequence identity to residues 198-344 of any one of SEQ ID NOs: 198-200 or residues 7-153 of SEQ ID NO: 201. In some particular embodiments, the HVR1 region can comprise residues 24-79 of any one of SEQ ID NOs: 198-200 or residues 215-270 of SEQ ID NO: 201. In some particular embodiments, the HVR2 region can comprise residues 215-270 of any one of SEQ ID NOs: 198-200 or residues 24-79 of SEQ ID NO: 201. In some particular embodiments, the first subunit can comprise residues 7-153 of any one of SEQ ID NOs: 198-200 or residues 198-344 of SEQ ID NO: 201. In some particular embodiments, the second subunit can comprise residues 198-344 of any one of SEQ ID NOs: 198-200 or residues 7-153 of SEQ ID NO: 201. In some embodiments, the engineered nuclease is a single-chain meganuclease comprising a linker, wherein the linker covalently joins the first subunit and the second subunit. In particular embodiments, the engineered meganuclease can comprise the amino acid sequence of any one of SEQ ID NOs: 198-201.

In some particular embodiments, the recognition sequence comprises SEQ ID NO: 195 (*i.e.*, the ORF 9-10 recognition sequence). In some such embodiments, the first subunit can comprise an amino acid sequence having at least 80%, at least 85%, at least 90%, at least 95%, or more, sequence identity to residues 7-153 of any one of SEQ ID NOs: 210-213, and/or the second subunit can comprise an amino acid sequence having at least 80%, at least 85%, at least 90%, at least 95%, or more, sequence identity to residues 198-344 of any one of SEQ ID NOs: 210-213. In some particular embodiments, the HVR1 region can comprise residues 24-79 of any one of SEQ ID NOs: 210-213. In some particular embodiments, the HVR2 region can comprise residues 215-270 of any one of SEQ ID NOs: 210-213. In some particular embodiments, the first subunit comprises residues 7-153 of any one of SEQ ID NOs: 210-213. In some particular embodiments, the second subunit comprises residues 198-344 of any one of SEQ ID NOs: 210-213. In some embodiments, the engineered nuclease is a single-chain meganuclease comprising a linker, wherein the linker covalently joins the first subunit and the second subunit. In particular embodiments, the engineered meganuclease comprises the amino acid sequence of any one of SEQ ID NOs: 210-213.

In some particular embodiments, the recognition sequence comprises SEQ ID NO: 196 (*i.e.*, the ORF 11-12 recognition sequence). In some such embodiments, the first subunit can comprise an amino acid sequence having at least 80%, at least 85%, at least 90%, at least 95%, or more, sequence identity to residues 198-344 of any one of SEQ ID NOs: 222-225, and/or the second subunit can comprise an amino acid sequence having at least 80%, at least 85%, at least 90%, at least 95%, or more, sequence identity to residues 7-153 of any one of SEQ ID NOs: 222-225. In some particular embodiments, the HVR1 region can comprise residues 215-270 of any one of SEQ ID NOs: 222-225. In some particular embodiments, the HVR2 region can comprise residues 24-79 of any one of SEQ ID NOs: 222-225. In some particular embodiments, the first subunit comprises residues 198-344 of any one of SEQ ID NOs: 222-225. In some particular embodiments, the second subunit comprises residues 7-153 of any one of SEQ ID NOs: 222-225. In some embodiments, the engineered nuclease is a single-chain meganuclease comprising a linker, wherein the linker covalently joins the first subunit and the second subunit. In some particular embodiments, the engineered meganuclease comprises the amino acid sequence of any one of SEQ ID NOs: 222-225.

In some particular embodiments, the recognition sequence comprises SEQ ID NO: 197 (*i.e.*, the ORF 13-14 recognition sequence). In some such embodiments, the first subunit can comprise an amino acid sequence having at least 80%, at least 85%, at least 90%, at least 95%, or more, sequence identity to residues 7-153 of any one of SEQ ID NOs: 234-237, and/or the second subunit can comprise an amino acid sequence having at least 80%, at least 85%, at least 90%, at least 95%, or more, sequence identity to residues 198-344 of any one of SEQ ID NOs: 234-237. In some particular embodiments, the HVR1 region can comprise residues 24-79 of any one of SEQ ID NOs: 234-237. In some particular embodiments, the HVR2 region can comprise residues 215-270 of any one of SEQ ID NOs: 234-237. In some particular embodiments, the first subunit comprises residues 7-153 of any one of SEQ ID NOs: 234-237. In some particular embodiments, the second subunit comprises residues 198-344 of any one of SEQ ID NOs: 234-237. In some embodiments, the engineered nuclease is a single-chain meganuclease comprising a linker, wherein the linker covalently joins the first subunit and the second subunit. In some particular embodiments, the engineered meganuclease comprises the amino acid sequence of any one of SEQ ID NOs: 234-237.

In another aspect, the invention provides isolated polynucleotides comprising nucleic acid sequences encoding engineered nucleases of the invention. In some embodiments, the isolated polynucleotide is an mRNA.

In some embodiments, the invention provides a recombinant DNA construct comprising a nucleic acid sequence encoding an engineered nuclease of the invention. In some such embodiments, the recombinant DNA construct encodes a viral vector. In some embodiments, the viral vector is a retrovirus, a lentivirus, an adenovirus, or an adeno-associated virus (AAV) vector. In particular embodiments, the viral vector is a recombinant AAV vector.

In some embodiments, the invention provides a viral vector comprising a nucleic acid sequence encoding an engineered nuclease of the invention. In some embodiments, the viral vector is a retrovirus, a lentivirus, an adenovirus, or an adeno-associated virus (AAV) vector. In particular embodiments, the viral vector is a recombinant AAV vector.

In another aspect, the invention provides a pharmaceutical composition for treatment of a subject having a nucleotide repeat expansion disorder, wherein the nucleotide repeat expansion disorder is characterized by expansion of a nucleotide repeat in a gene of interest. The pharmaceutical composition of the invention comprises a pharmaceutically acceptable carrier and: (a) a first nucleic acid encoding a first engineered nuclease and a second nucleic acid encoding a second engineered nuclease, wherein the first engineered nuclease and the second engineered nuclease are expressed in a target cell *in vivo*; or (b) a first engineered nuclease protein and a second engineered nuclease protein; wherein the first engineered nuclease recognizes and cleaves a first recognition sequence positioned 5' upstream of the nucleotide repeat in the gene of interest and wherein the second engineered nuclease recognizes and cleaves a second recognition sequence positioned 3' downstream of the nucleotide repeat in the gene of interest.

In some embodiments, the first recognition sequence and/or the second recognition sequence are positioned within the same exon, the same intron, or the same untranslated region (UTR) as the nucleotide repeat.

In another embodiment, the first recognition sequence and/or the second recognition sequence are not positioned within the same exon, the same intron, or the same UTR as the nucleotide repeat. In some non-limiting examples, the first recognition sequence and/or the second recognition sequence are positioned in an exon, an intron, or a UTR adjacent to the exon, intron, or UTR which is occupied by the nucleotide repeat.

In some embodiments, the first nucleic acid and/or the second nucleic acid is an mRNA.

In some embodiments, the pharmaceutical composition comprises a first recombinant DNA construct comprising the first nucleic acid and/or a second recombinant DNA construct comprising the second nucleic acid.

In some embodiments, the pharmaceutical composition comprises a first viral vector comprising the first nucleic acid and/or a second viral vector comprising the second nucleic acid.

In some embodiments, the first viral vector and/or the second viral vector is a retrovirus, a lentivirus, an adenovirus, or an AAV vector. In particular embodiments, the first viral vector and/or the second viral vector is a recombinant AAV vector.

In some embodiments, the first engineered nuclease and/or the second engineered nuclease is an engineered meganuclease, a zinc finger nuclease, a TALEN, a compact TALEN, a CRISPR, or a megaTAL.

In some embodiments, the first engineered nuclease is a first engineered meganuclease and/or the second engineered nuclease is a second engineered meganuclease.

In some embodiments, the pharmaceutical composition is useful for the treatment of Friedreich's ataxia and the gene of interest is the human *FXN* gene. In such embodiments, the first recognition sequence can comprise any one of SEQ ID NOs: 12-73. In other such embodiments, the second recognition sequence can comprise any one of SEQ ID NOs: 75-95.

In some particular embodiments, the first recognition sequence can comprise SEQ ID NO: 34 (*i.e.*, the FXN 3-4 recognition sequence), and the first engineered meganuclease can be any engineered meganuclease of the invention which recognizes and cleaves the recognition sequence of SEQ ID NO: 34. In some particular embodiments, the first engineered meganuclease comprises the amino acid sequence of any one of SEQ ID NOs: 155-159.

In some particular embodiments, the first recognition sequence can comprise SEQ ID NO: 29 (*i.e.*, the FXN 1-2 recognition sequence), and the first engineered meganuclease can be any engineered meganuclease of the invention which recognizes and cleaves the recognition sequence of SEQ ID NO: 29. In some particular embodiments, the first engineered meganuclease comprises the amino acid sequence of any one of SEQ ID NOs: 143-146.

In some particular embodiments, the first recognition sequence can comprise SEQ ID NO: 63 (*i.e.*, the FXN 11-12 recognition sequence), and the first engineered meganuclease can be any engineered meganuclease of the invention which recognizes and cleaves the recognition sequence of SEQ ID NO: 63. In some particular embodiments, the first engineered meganuclease comprises the amino acid sequence of any one of SEQ ID NOs: 182-185.

In some particular embodiments, the second recognition sequence can comprise SEQ ID NO: 89 (*i.e.*, the FXN 5-6 recognition sequence), and the second engineered meganuclease can be any engineered meganuclease of the invention which recognizes and cleaves the recognition sequence of SEQ ID NO: 89. In some particular embodiments, the second engineered meganuclease comprises the amino acid sequence of any one of SEQ ID NOs: 170-173.

In some particular embodiments, the first recognition sequence comprises SEQ ID NO: 34 (*i.e.*, the FXN 3-4 recognition sequence) and the second recognition sequence comprises SEQ ID NO: 89 (*i.e.*, the FXN 5-6 recognition sequence).

In some embodiments, the pharmaceutical composition is useful for the treatment of amyotrophic lateral sclerosis (ALS) and/or frontotemporal dementia (FTD) and the gene of interest is the human *C9ORF72* gene. In some such embodiments, the first recognition sequence can comprise SEQ ID NO: 194 (*i.e.*, the ORF 7-8 recognition sequence), and the first engineered meganuclease can comprise any engineered meganuclease of the invention which recognizes and cleaves SEQ ID NO: 194. In some particular embodiments, the first engineered meganuclease comprises the amino acid sequence of any one of SEQ ID NOs: 198-201.

In some particular embodiments, the first recognition sequence can comprise SEQ ID NO: 197 (*i.e.*, the ORF 13-14 recognition sequence), and the first engineered meganuclease comprises any engineered meganuclease of the invention which recognizes and cleaves SEQ ID NO: 197. In some particular embodiments, the first engineered meganuclease comprises the amino acid sequence of any one of SEQ ID NOs: 234-237.

In some particular embodiments, the second recognition sequence can comprise SEQ ID NO: 195 (*i.e.*, the ORF 9-10 recognition sequence), and the second engineered meganuclease comprises any engineered meganuclease of the invention which recognizes and cleaves SEQ ID NO: 195. In some particular embodiments, the second engineered meganuclease comprises the amino acid sequence of any one of SEQ ID NOs: 210-213.

In some particular embodiments, the second recognition sequence comprises SEQ ID NO: 196 (*i.e.*, the ORF 11-12 recognition sequence), and the second engineered meganuclease comprises any engineered meganuclease of the invention which recognizes and cleaves SEQ ID NO: 196. In some particular embodiments, the second engineered meganuclease comprises the amino acid sequence of any one of SEQ ID NOs: 222-225.

In some particular embodiments, the first recognition sequence comprises SEQ ID NO: 194 (*i.e.*, the ORF 7-8 recognition sequence) and the second recognition sequence comprises SEQ ID NO: 195 (*i.e.*, the ORF 9-10 recognition sequence). In some particular embodiments, the first recognition sequence comprises SEQ ID NO: 197 (*i.e.*, the ORF 13-14 recognition sequence) and the second recognition sequence comprises SEQ ID NO: 196 (*i.e.*, the ORF 11-12 recognition sequence).

In some particular embodiments, the pharmaceutical composition is useful for the treatment of Fragile X syndrome and the gene of interest is the human *FMR1* gene. In some such embodiments, the first recognition sequence can comprise SEQ ID NO: 2. In some such embodiments, the second recognition sequence can comprise SEQ ID NO: 4.

In some particular embodiments, the pharmaceutical composition is useful for the treatment of Huntington's disease and the gene of interest is the human *HTT* gene. In some particular embodiments, the first recognition sequence can comprise any one of SEQ ID NOs: 5, 6, or 141. In some particular embodiments, the second recognition sequence can comprise SEQ ID NO: 4.

In some particular embodiments, the pharmaceutical composition is useful for the treatment of myotonic dystrophy type I (MD1) and the gene of interest is the human *DMPK* gene. In some particular embodiments, the first recognition sequence can comprise any one of SEQ ID NOs: 97-101. In some particular embodiments, the second recognition sequence can comprise any one of SEQ ID NOs: 103-131.

In another aspect, the invention provides methods for treating a subject having a nucleotide repeat expansion disorder, wherein the nucleotide repeat expansion disorder is characterized by expansion of a nucleotide repeat in a gene of interest. The methods comprise delivering to target cells in the subject: (a) at least a first engineered nuclease protein and a second engineered nuclease protein; or (b) at least a first nucleic acid encoding the first engineered nuclease and a second nucleic acid encoding the second engineered nuclease, wherein the first engineered nuclease and the second engineered nuclease are expressed in the target cells *in vivo*; wherein the first engineered nuclease recognizes and cleaves a first recognition sequence positioned 5' upstream of the nucleotide repeat in said gene of interest; and wherein the second engineered nuclease recognizes and cleaves a second recognition sequence positioned 3' downstream of the nucleotide repeat in said gene of interest; and wherein an intervening DNA fragment between the first recognition sequence and the second recognition sequence is excised and the number of the nucleotide repeat is reduced in the gene of interest.

In some embodiments of the methods, the engineered nuclease is an engineered meganuclease, zinc finger nuclease, TALEN, compact TALEN, CRISPR, or megaTAL nuclease.

In some embodiments of the methods, the first engineered nuclease and the second engineered nuclease generate complementary overhangs which promote direct re-ligation of the gene of interest following cleavage and excision of the intervening DNA fragment.

In some embodiments of the methods, the nucleotide repeat is a trinucleotide repeat. In some such embodiments of the methods, the trinucleotide repeat is selected from the group consisting of CAG, CGG, CCG, GAA, and CTG.

In some embodiments of the methods, the nucleotide repeat expansion disorder is Friedreich's Ataxia, the trinucleotide repeat is GAA, and the gene of interest is the human frataxin (*FXN*) gene, wherein the trinucleotide repeat is positioned within intron 1 of the *FXN* gene. In some embodiments of the methods, the first recognition sequence is positioned 5' upstream of the trinucleotide repeat in intron 1 (SEQ ID NO: 74) and the second recognition sequence is positioned 3' downstream of the trinucleotide repeat in intron 1 (SEQ ID NO: 96). In some embodiments of the methods, the first engineered nuclease is a first engineered meganuclease and the second engineered nuclease is a second engineered meganuclease. In some embodiments of the methods, the first recognition sequence comprises any one of SEQ ID NOs: 12-73. In particular embodiments, the first recognition sequence comprises SEQ ID NO: 34 (*i.e.*, the FXN 3-4 recognition sequence), and the first engineered meganuclease comprises any engineered meganuclease of the invention which recognizes and cleaves SEQ ID NO: 34. In other particular embodiments, the first recognition sequence comprises SEQ ID NO: 29 (*i.e.*, the FXN 1-2 recognition sequence), and the first engineered meganuclease comprises any engineered meganuclease of the invention which recognizes and cleaves SEQ ID NO: 29. In other particular embodiments, the first recognition sequence comprises SEQ ID NO: 63 (*i.e.*, the FXN 11-12 recognition sequence), and the first engineered meganuclease comprises any engineered meganuclease of the invention which recognizes and cleaves SEQ ID NO: 63. In some embodiments of the methods, the second recognition sequence comprises any one of SEQ ID NOs: 75-95. In some particular embodiments, the second recognition sequence comprises SEQ ID NO: 89 (*i.e.*, the FXN 5-6 recognition sequence), and the second engineered meganuclease comprises any engineered meganuclease of the invention which recognizes and cleaves SEQ ID NO: 89.

In some embodiments of the methods, the nucleotide repeat is a hexanucleotide repeat. In some embodiments of the methods, the hexanucleotide repeat is GGGGCC.

In some embodiments of the methods, the nucleotide repeat expansion disorder is amyotrophic lateral sclerosis (ALS) or frontotemporal dementia (FTD), the hexanucleotide repeat is GGGGCC, and the gene of interest is the human *C9ORF72* gene, wherein the hexanucleotide repeat is positioned within intron 1 of the *C9ORF72* gene. In some embodiments of the methods, the first recognition sequence is positioned 5' upstream of the hexanucleotide repeat, and the second recognition sequence is positioned 3' downstream of the hexanucleotide repeat. In some embodiments of the methods, the first engineered nuclease is a first engineered meganuclease and the second engineered nuclease is a second engineered meganuclease. In some embodiments of the methods, the first recognition sequence can comprise SEQ ID NO: 194 or 197. In some particular embodiments, the first recognition sequence comprises SEQ ID NO: 194 (*i.e.*, the ORF 7-8 recognition sequence), and the first engineered meganuclease comprises any engineered meganuclease of the invention which recognizes and cleaves SEQ ID NO: 194. In other particular embodiments, the first recognition sequence comprises SEQ ID NO: 197 (*i.e.*, the ORF 13-14 recognition sequence), and the first engineered meganuclease comprises any engineered meganuclease of the invention which recognizes and cleaves SEQ ID NO: 197. In some embodiments of the methods, the second recognition sequence can comprise SEQ ID NO: 195 or 196. In some particular embodiments, the second recognition sequence comprises SEQ ID NO: 195(*i.e.*, the ORF 9-10 recognition sequence), and the second engineered meganuclease comprises any engineered meganuclease of the invention which recognizes and cleaves SEQ ID NO: 195. In other particular embodiments, the second recognition sequence comprises SEQ ID NO: 196(*i.e.*, the ORF 11-12 recognition sequence), and the second engineered meganuclease comprises any engineered meganuclease of the invention which recognizes and cleaves SEQ ID NO: 196.

In some embodiments of the methods, the nucleotide repeat expansion disorder is Fragile X syndrome, the trinucleotide repeat is CGG, and the gene of interest is the human *FMR1* gene. In some particular embodiments, the first recognition sequence can comprise SEQ ID NO: 2. In some particular embodiments, the second recognition sequence can comprise SEQ ID NO: 4.

In some embodiments of the methods, the nucleotide repeat expansion disorder is Huntington's disease, the trinucleotide repeat is CAG, and the gene of interest is the human *HTT* gene. In some embodiments, the first recognition sequence can comprise any one of SEQ ID NOs: 5, 6, or 141. In some embodiments, the second recognition sequence can comprise SEQ ID NO: 4.

In some embodiments of the methods, the nucleotide repeat expansion disorder is myotonic dystrophy type I (MD1), the nucleotide repeat is CTG, and the gene of interest is the human *DMPK* gene. In some embodiments, the first recognition sequence can comprise any one of SEQ ID NOs: 97-101. In some embodiments, the second recognition sequence can comprise any one of SEQ ID NOs: 103-131.

In some embodiments, the methods of treatment provided by the invention further comprise administering to the subject a pharmaceutical composition of the invention.

In another aspect, the invention provides an engineered nuclease, and particularly an engineered meganuclease, described herein for use as a medicament. The invention further provides the use of an engineered nuclease, and particularly an engineered meganuclease, described herein in the manufacture of a medicament for treating a nucleotide repeat expansion disorder.

In another aspect, the invention provides isolated polynucleotides for use as medicaments, wherein the isolated polynucleotides comprise a nucleic acid sequence encoding an engineered nuclease, and particularly an engineered meganuclease, of the invention. The invention further provides the use of an isolated polynucleotide in the manufacture of a medicament for treating a nucleotide repeat expansion disorder, wherein the isolated polynucleotide comprises a nucleic acid sequence encoding an engineered nuclease, and particularly an engineered meganuclease, of the invention.

In another aspect, the invention provides a recombinant AAV vector for use as a medicament, wherein the recombinant AAV vector comprises an isolated polynucleotide, and wherein the isolated polynucleotide comprises a nucleic acid sequence encoding an engineered nuclease, and particularly an engineered meganuclease, of the invention. The invention further provides the use of a recombinant AAV vector in the manufacture of a medicament for treating a nucleotide repeat expansion disorder, wherein the recombinant AAV vector comprises an isolated polynucleotide, and wherein the isolated polynucleotide comprises a nucleic acid sequence encoding an engineered nuclease, and particularly an engineered meganuclease, of the invention.

### BRIEF DESCRIPTION OF THE FIGURES

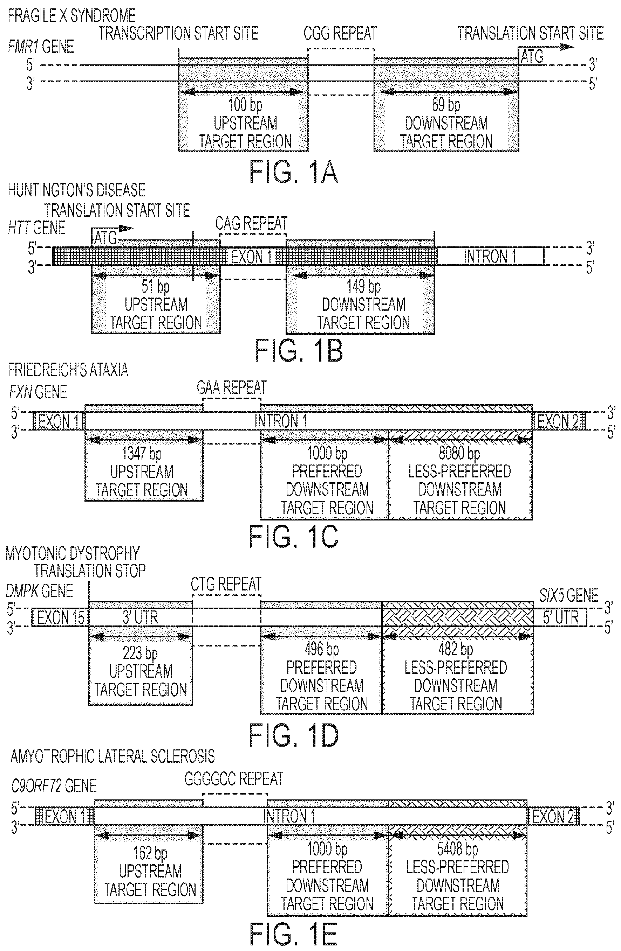

Figure 1. Structures of several human genes associated with nucleotide repeat expansion disorders. A) Structure of the 5' portion of the *FMR1* gene associated with Fragile X Syndrome. The CGG trinucleotide repeat is in the 5' untranslated region of the gene. The transcription- and translation-start sites are identified with relative distances (in basepairs) to the CGG repeat region. The upstream and downstream target regions to which a nuclease pair could be directed to excise the repeat are shaded. B) Structure of the 5' portion of the *HTT* gene associated with Huntington's Disease. The translation start site is identified, as is the rest of Exon 1 and the Exon1/Intron 1 boundary. The upstream and downstream target regions to which a nuclease pair could be directed to excise the repeat from the exon are shaded. C) The structure of Intron 1 of the human *FXN* gene associated with Friedreich's Ataxia. Exon/Intron borders are indicated and the preferred and less-preferred loci for targeting endonucleases upstream and downstream of the GAA repeat responsible for disease are shaded. D) Structure of the *DMPK* 3' untranslated region (UTR) showing the location of the CTG trinucleotide repeat associated with Myotonic Dystrophy. Preferred and less-preferred loci for targeting endonucleases upstream and downstream of the repeat are shaded. E) Structure of the 5' portion of the *C9ORF72* gene associated with ALS and FTD. Exon 1, intron 1, and exon 2 are represented, and the location of the hexanucleotide GGGGCC repeat in intron 1 is shown. Preferred and less-preferred loci for targeting endonucleases upstream and downstream of the repeat are shaded.

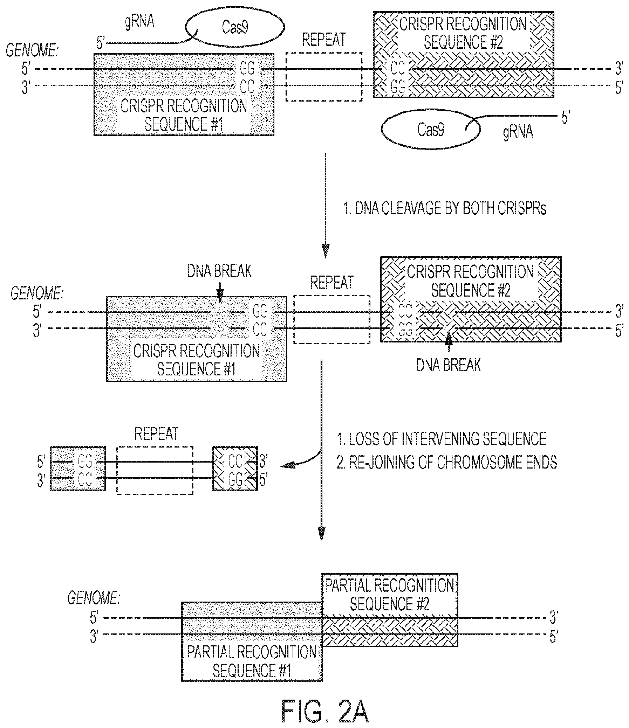

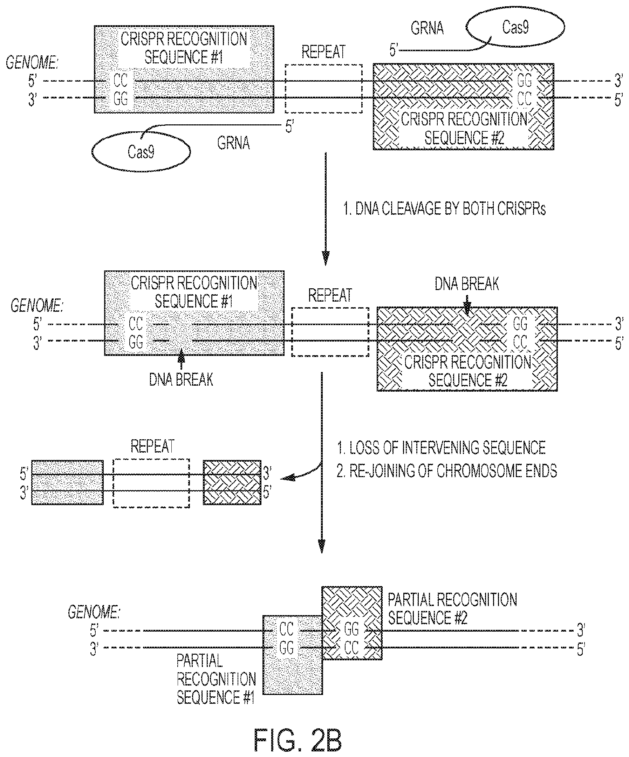

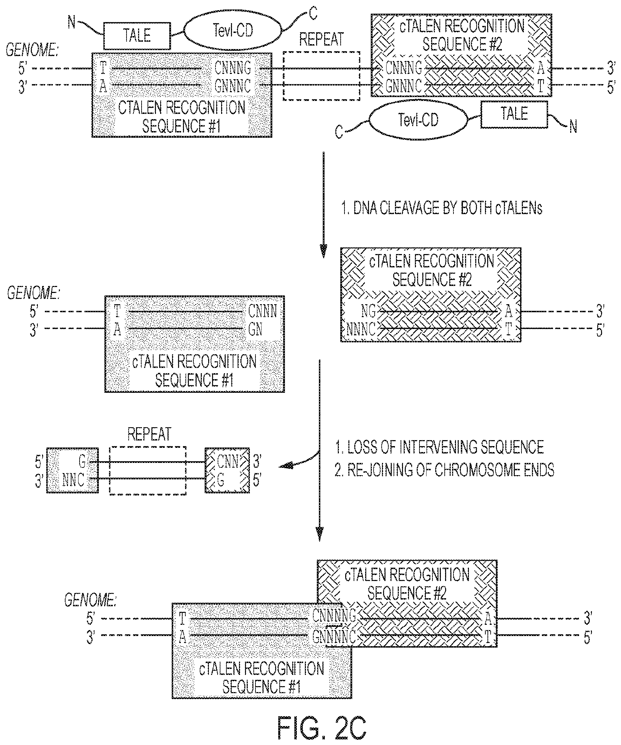

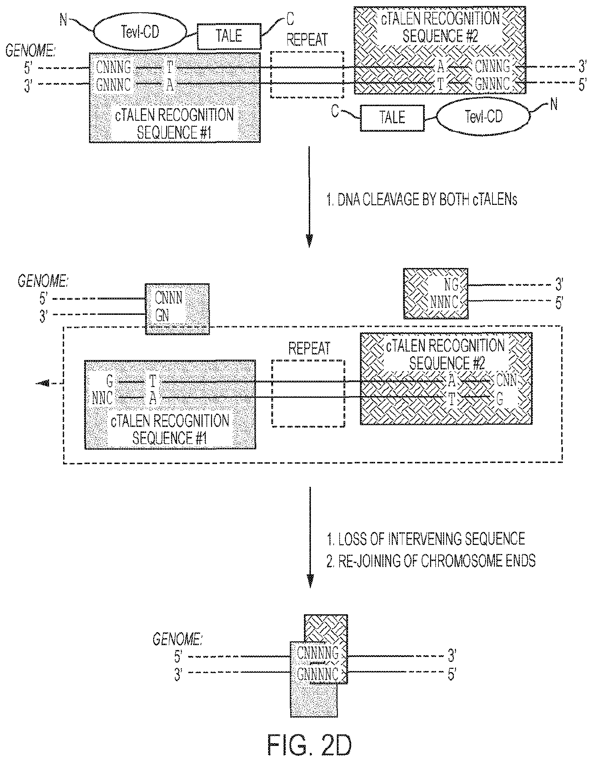

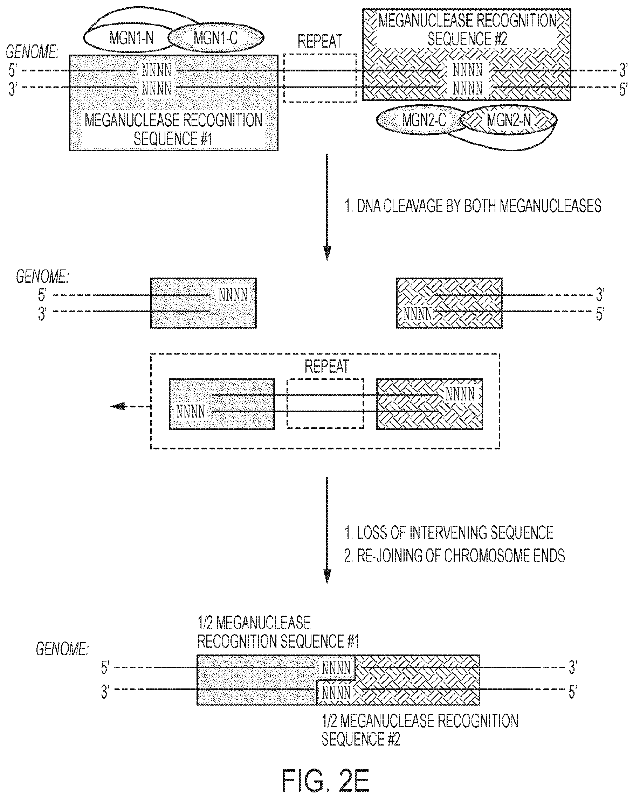

Figure 2. Strategies for deleting nucleotide repeats using different types of nucleases. 2A) Strategy for deleting a nucleotide repeat using a pair of CRISPRs. A pair of "guide RNAs" ("gRNAs") are used which are complementary to a pair of recognition sites flanking the nucleotide of interest. As drawn in this figure, the gRNAs can be complementary to recognition sequences that are distal to the conserved "GG" motif and the site of Cas9 DNA cleavage. In this orientation, the CRISPR recognition sequences are largely conserved following DNA cleavage, excision of the intervening fragment of genomic DNA, and re-joining of the chromosome ends. 2B) An alternative scheme for deleting a nucleotide repeat using a pair of CRISPRs in which the gRNAs are complementary to recognition sequences that are proximal to the nucleotide repeat. In this orientation, the CRISPR recognition sequences are largely deleted following DNA cleavage, excision of the intervening fragment of genomic DNA, and re-joining of the chromosome ends. It is contemplated in the invention could also comprise a hybrid of the schemes shown in 2A and 2B. 2C) Strategy for deleting a nucleotide repeat using a pair of compact TALENs (cTALENs). A pair of TAL effector DNA-binding domains ("TALEs") are used which bind to a pair of recognition sites flanking the nucleotide repeat of interest. As drawn in this figure, the TALEs can bind to recognition sequences that are distal to the conserved "CNNNG" motif that is recognized and cut by the I-TevI cleavage domain ("TevI-CD"). In this orientation, the cTALEN recognition sequences are largely conserved following DNA cleavage, excision of the intervening fragment of genomic DNA, and re-joining of the chromosome ends. Also, the cTALENs in this figure are shown with the TALE and TevI-CD domains in an N- to C- orientation. It is also possible to generate cTALENs with these two domains in a C- to N- orientation. 2D) An alternative scheme for deleting a nucleotide repeat using a pair of cTALENS in which the TALE domains bind to recognition sequences that are proximal to the repeat. In this orientation, the cTALEN recognition sequences are largely deleted following DNA cleavage, excision of the intervening fragment of genomic DNA, and re-joining of the chromosome ends. Also, the cTALENs in this figure are drawn with the TALE and TevI-CD domains in a C- to N- orientation. It is contemplated in the invention could also comprise a hybrid of the schemes shown in 2C and 2D. 2E) Strategy for deleting a nucleotide repeat gene using a pair of single-chain meganucleases. The meganucleases are drawn as two-domain proteins (MGN-N: the N-terminal domain; and MGN-C: the C-terminal domain) joined by a linker. In the figure, the C-terminal domain is drawn as binding to the half of the recognition sequence that is closest to the repeat. In some embodiments, however, the N-terminal domain can bind to this half of the recognition sequence. The central four basepairs of the recognition sequence are shown as "NNNN". These four basepairs become single-strand 3' "overhangs" following cleavage by the meganuclease. The subset of preferred four basepair sequences that comprise this region of the sequence are identified in WO/2010/009147. DNA cleavage by the pair of meganucleases generates a pair of four basepair 3' overhangs at the chromosome ends. If these overhangs are complementary, they can anneal to one another and be directly re-ligated, resulting in the four basepair sequence being retained in the chromosome following nucleotide repeat excision. Because meganucleases cleave near the middle of the recognition sequence, half of each recognition sequence will frequently be retained in the chromosome following excision of the repeat. The other half of each recognition sequence will removed from the genome with the repeat.

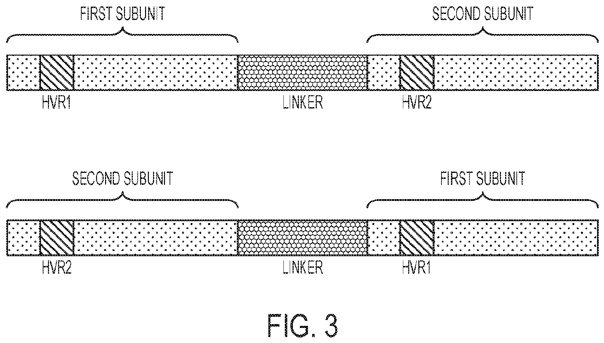

Figure 3. Engineered meganucleases of the invention comprise two subunits, wherein the first subunit comprising the HVR1 region binds to a first recognition half-site (*e.g.*, FXN1) and the second subunit comprising the HVR2 region binds to a second recognition half-site (*e.g.*, FXN2). In embodiments where the recombinant meganuclease is a single-chain meganuclease, the first subunit comprising the HVR1 region can be positioned as either the N-terminal or C-terminal subunit. Likewise, the second subunit comprising the HVR2 region can be positioned as either the N-terminal or C-terminal subunit.

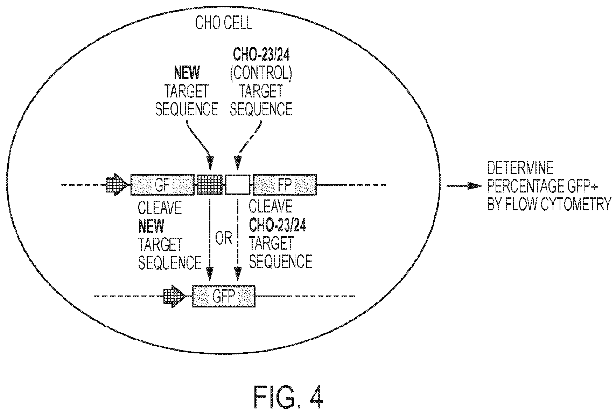

Figure 4. Schematic of reporter assay in CHO cells for evaluating recombinant meganucleases. For the recombinant meganucleases described herein, a CHO cell line was produced in which a reporter cassette was integrated stably into the genome of the cell. The reporter cassette comprised, in 5' to 3' order: an SV40 Early Promoter; the 5' 2/3 of the GFP gene; the recognition sequence for an engineered meganuclease of the invention; the recognition sequence for the CHO-23/24 meganuclease (WO/2012/167192); and the 3' 2/3 of the GFP gene. Cells stably transfected with this cassette did not express GFP in the absence of a DNA break-inducing agent. Meganucleases were introduced by transduction of plasmid DNA or mRNA encoding each meganuclease. When a DNA break was induced at either of the meganuclease recognition sequences, the duplicated regions of the GFP gene recombined with one another to produce a functional GFP gene. The percentage of GFP-expressing cells could then be determined by flow cytometry as an indirect measure of the frequency of genome cleavage by the meganucleases.

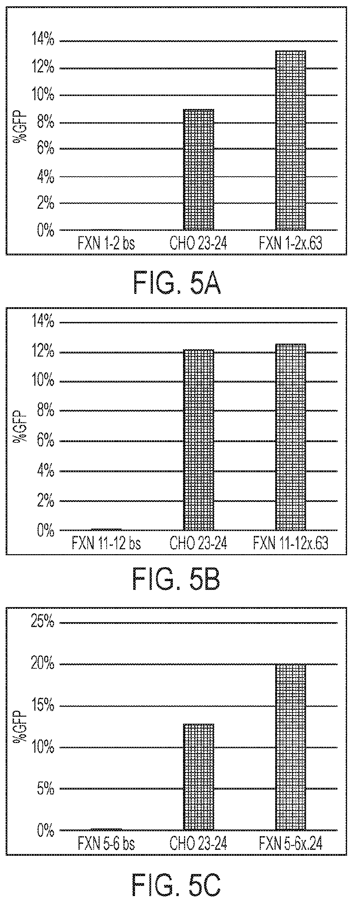

Figure 5. Efficiency of recombinant meganucleases for recognizing and cleaving recognition sequences upstream and downstream of the trinucleotide repeat region in the human *FXN* gene in a CHO cell reporter assay. Engineered meganucleases were designed to target the FXN 1-2 recognition sequence (SEQ ID NO: 29), the FXN 11-12 recognition sequence (SEQ ID NO: 63), and the FXN 5-6 recognition sequence (SEQ ID NO: 89), and were screened for efficacy in the CHO cell reporter assay. The results shown provide the percentage of GFP-expressing cells observed in each assay, which indicates the efficacy of each meganuclease for cleaving its respective target recognition sequence or the CHO-23/24 recognition sequence. A negative control (bs) was further included in each assay. A) Meganuclease targeting the FXN 1-2 recognition sequence. B) Meganuclease targeting the FXN 11-12 recognition sequence. C) Meganuclease targeting the FXN 5-6 recognition sequence.

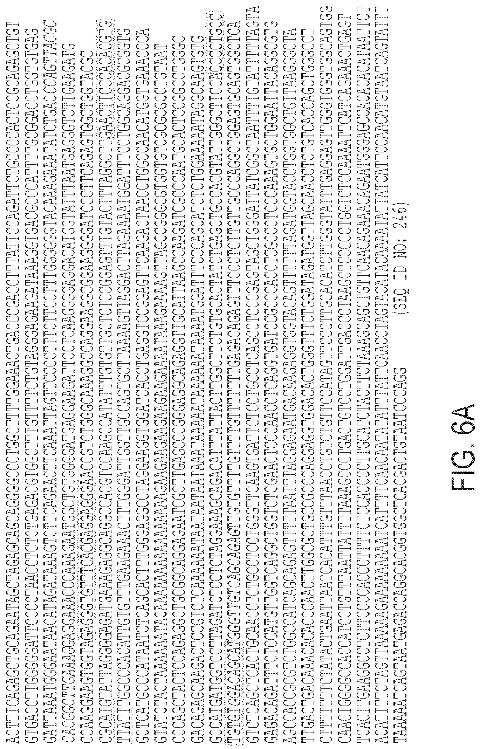

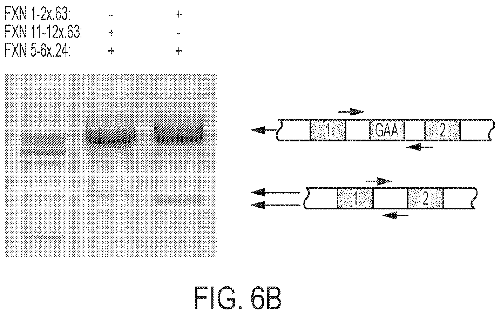

Figure 6. Excision of the *FXN* GAA repeat using the FXN 1-2x.63, FXN 11-12x.63, and FXN 5-6x.24 meganucleases. 6A) Sequence of *FXN* Intron 1 flanking the GAA repeat associated with Friedreich's Ataxia. The repeat sequence is underlined. Recognition sites for the FXN 1-2x.63, FXN 11-12x.63, and FXN 5-6x.24 meganucleases are shaded in gray. Annealing sites for a pair of PCR primers used for analysis are italicized. 6B) Agarose gel electrophoresis analysis of HEK-293 cells co-transfected with mRNA encoding the FXN 1-2x.63 + FXN 5-6x.24 or FXN 11-12x.63 + FXN 5-6x.24 meganucleases. Genomic DNA was isolated from the cells and evaluated by PCR using the primers indicated in (6A). PCR products were resolved on an agarose gel and it was found that HEK-293 cells co-expressing each of the two meganucleases pairs yielded a smaller PCR band consistent with deletion of the sequence intervening the meganuclease recognition sites.

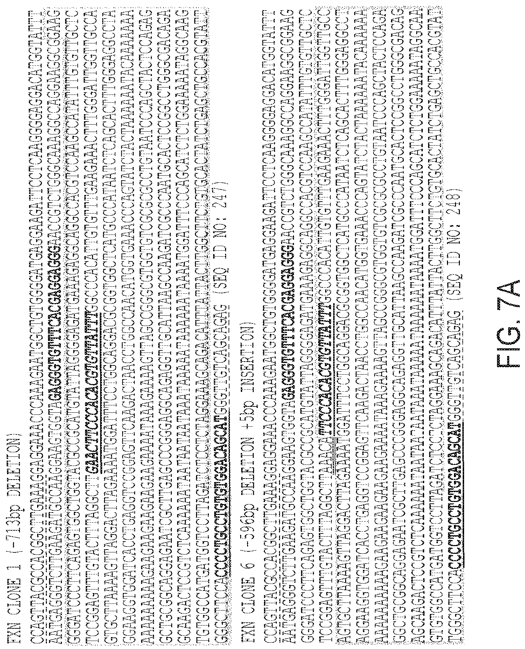

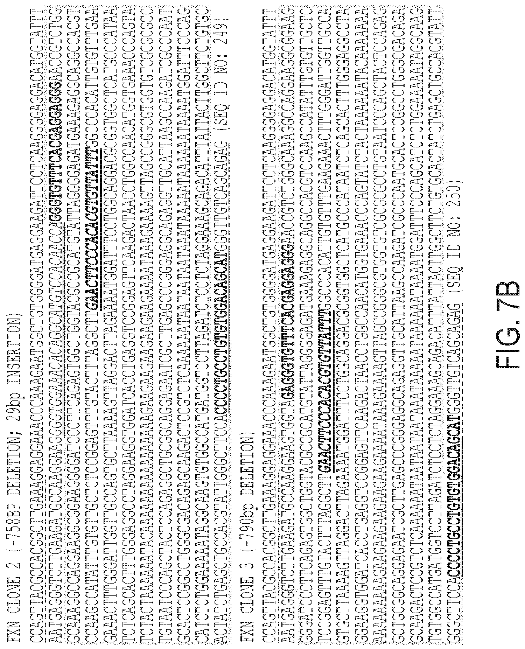

Figure 7. Example sequences from HEK-293 cells co-transfected with: A) FXN 11-12x.63 + FXN 5-6x.24; or B) FXN 1-2x.63 + FXN 5-6x.24. The smaller PCR bands shown in Figure 6B were isolated, cloned and sequenced, and two representative sequences are shown from each transfection. The FXN 1-2x.63 recognition site is shown in bolded font. The FXN 11-12x.63 recognition site is shown in bolded/italicized/underlined font. The FXN 5-6x.24 recognition site is shown in underlined/bolded font. Bases that are deleted from the sequence (*i.e.,* absent from the sequence) are shaded in gray. Bases that were added to the sequence (*i.e.,* via untemplated insertion) are double underlined. Primer sites are indicated by a dashed underline.

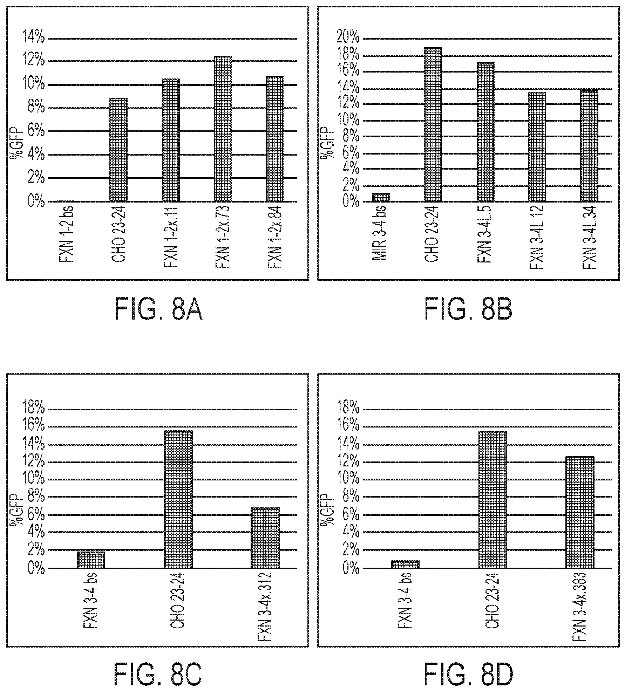

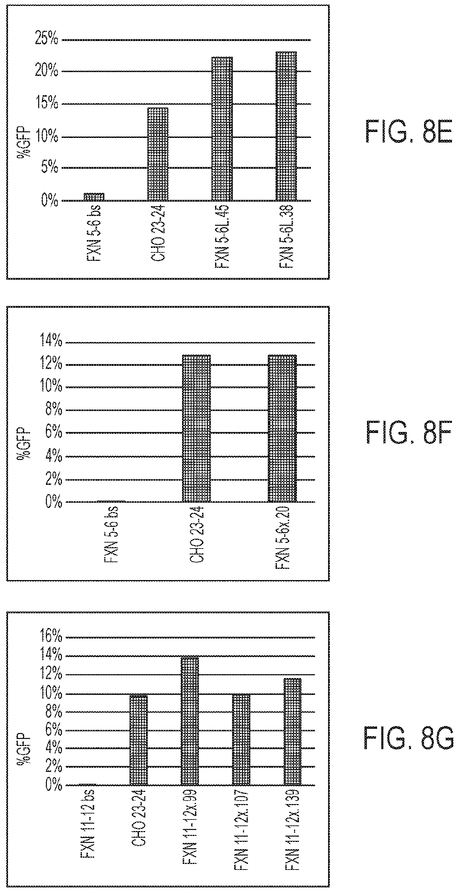

Figure 8. Efficiency of recombinant meganucleases for recognizing and cleaving recognition sequences upstream and downstream of the trinucleotide repeat region in the human *FXN* gene in a CHO cell reporter assay. Engineered meganucleases were designed to target the FXN 1-2 recognition sequence (SEQ ID NO: 29), the FXN 3-4 recognition sequence (SEQ ID NO: 34), the FXN 5-6 recognition sequence (SEQ ID NO: 89), and the FXN 11-12 recognition sequence (SEQ ID NO: 63), and were screened for efficacy in the CHO cell reporter assay. The results shown provide the percentage of GFP-expressing cells observed in each assay, which indicates the efficacy of each meganuclease for cleaving its respective target recognition sequence or the CHO-23/24 recognition sequence. A negative control (bs) was further included in each assay. A) Meganucleases targeting the FXN 1-2 recognition sequence. B)-D) Meganucleases targeting the FXN 3-4 recognition sequence. E)-F) Meganucleases targeting the FXN 5-6 recognition sequence. G) Meganucleases targeting the FXN 11-12 recognition sequence.

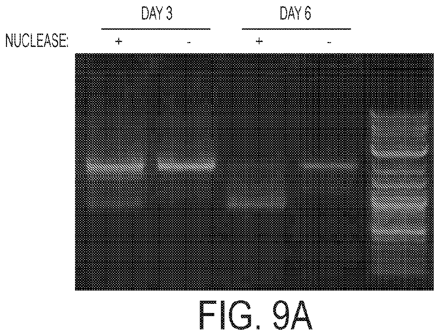

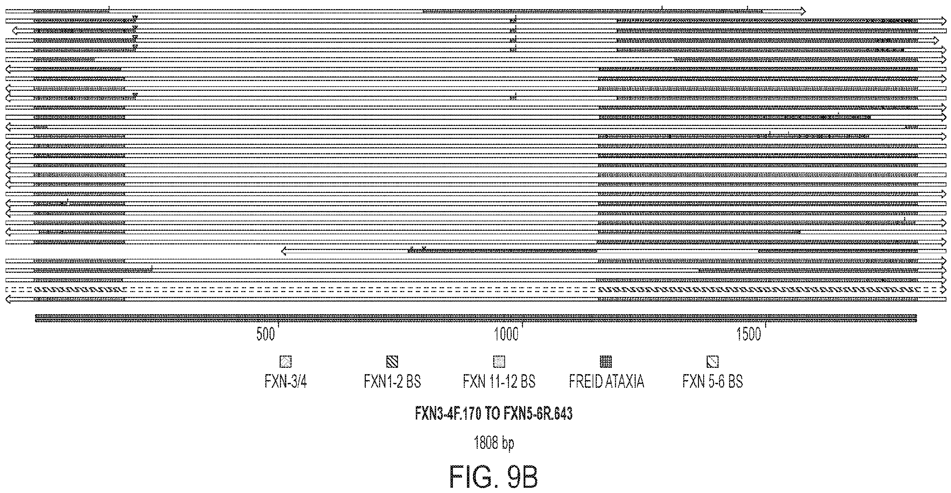

Figure 9. PCR analysis of trinucleotide repeat deletion in mammalian cells. A) HEK 293 cells were transformed with a pair of FXN 3-4/FXN 5-6 meganucleases and evaluated by PCT analysis to determine whether a deletion of the GAA repeat region occurred after 3 days or 6 days. B) Smaller bands from the PCR analysis were sequenced to determine whether re-ligation occurred between the FXN 3-4 and FXN 5-6 cleavage sites.

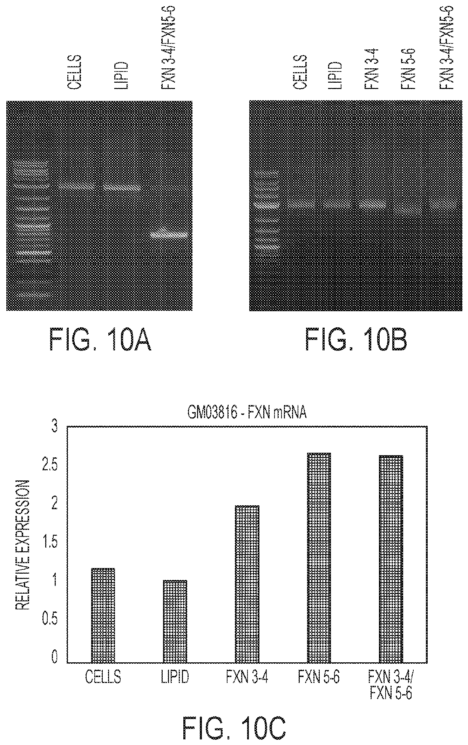

Figure 10. PCR analysis of trinucleotide repeat deletion in mammalian cells. GM03816 human fibroblast cells, which comprise an expanded GAA repeat in the FXN gene, were transformed with a FXN 3-4 meganuclease, a FXN 5-6 meganuclease, or a FXN 3-4/FXN 5-6 meganuclease pair to excise the GAA repeat region. A) PCR analysis of RNA from transformed cells after 4 days. B) PCR analysis of RNA from transformed cells after 18 days. C) At 10 days post-transfection, RNA was obtained from cells transfected with the FXN 3-4 meganuclease, the FXN 5-6 meganuclease, or the FXN 3-4/FXN 5-6 meganuclease pair, and was then analyzed by quantitative PCR to evaluate changes in FXN mRNA expression.

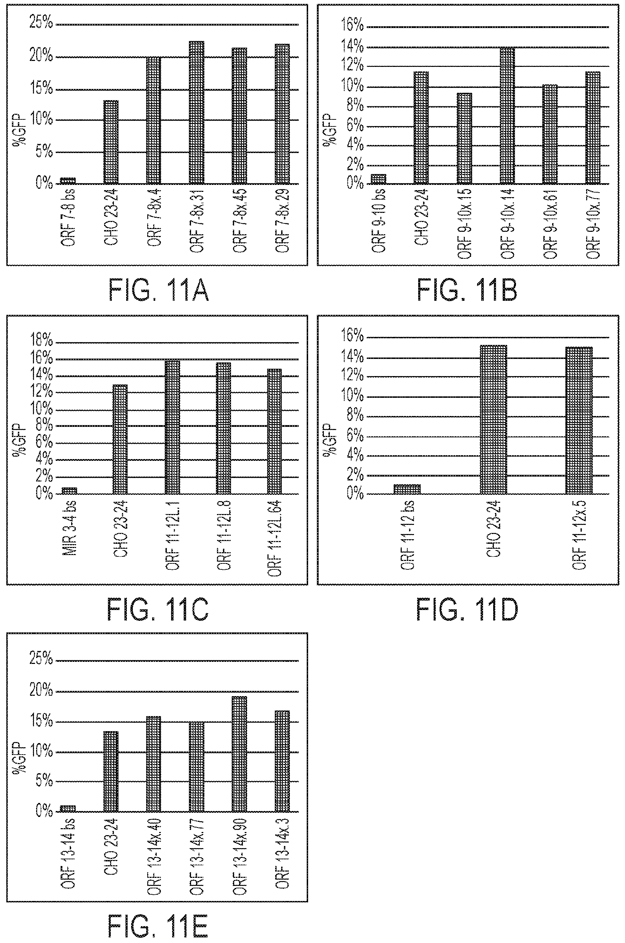

Figure 11. Efficiency of recombinant meganucleases for recognizing and cleaving recognition sequences upstream and downstream of the hexanucleotide repeat region in the human *C9ORF72* gene in a CHO cell reporter assay. Engineered meganucleases were designed to target the ORF 7-8 recognition sequence (SEQ ID NO: 194), the ORF 9-10 recognition sequence (SEQ ID NO: 195), the ORF 11-12 recognition sequence (SEQ ID NO: 196), and the ORF 13-14 recognition sequence (SEQ ID NO: 197), and were screened for efficacy in the CHO cell reporter assay. The results shown provide the percentage of GFP-expressing cells observed in each assay, which indicates the efficacy of each meganuclease for cleaving its respective target recognition sequence or the CHO-23/24 recognition sequence. A negative control (bs) was further included in each assay. A) Meganucleases targeting the ORF 7-8 recognition sequence. B) Meganucleases targeting the ORF 9-10 recognition sequence. C)-D) Meganucleases targeting the ORF 11-12 recognition sequence. E) Meganucleases targeting the ORF 13-14 recognition sequence.

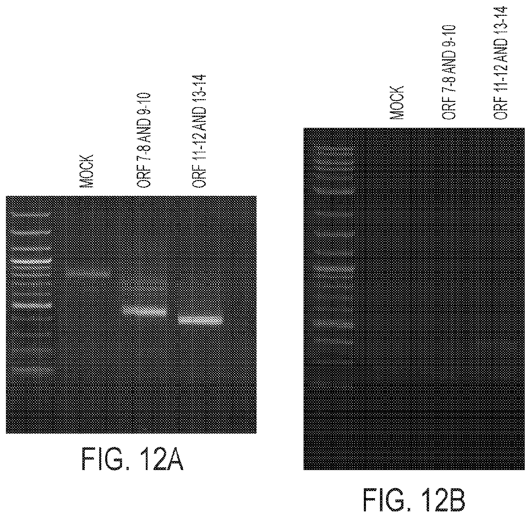

Figure 12. PCR analysis of hexanucleotide repeat deletion in mammalian cells. A) Combinations of engineered ORF meganucleases were introduced by RNA into HEK293 cells to determine if each pair would delete the *C9ORF72* hexanucleotide repeat in a consistent manner due to direct re-ligation of compatible overhangs. DNA was isolated from cells 48 hours post-transfection and analyzed by PCR using primers that flank the hexanucleotide repeat region. B) Combinations of engineered ORF meganucleases were introduced by RNA into ND42496 cells (fibroblasts derived from a patient homozygous for a hexanucleotide repeat expansion in the *C9ORF72* gene) to determine if each pair would delete the *C9ORF72* hexanucleotide repeat in a consistent manner due to direct re-ligation of compatible overhangs. DNA was isolated from cells 48 hours post-transfection and analyzed by PCR using primers that flank the hexanucleotide repeat region.

### BRIEF DESCRIPTION OF THE SEQUENCES

SEQ ID NO: 1 sets forth the amino acid sequence of the wild-type I-CreI meganuclease.
SEQ ID NO: 2 sets forth the nucleic acid sequence of a meganuclease recognition sequence upstream of the *FMR1* CGG repeat.
SEQ ID NO: 3 sets forth the nucleic acid sequence of a 5' UTR of the *FMR1* gene upstream of CGG repeat.
SEQ ID NO: 4 sets forth the nucleic acid sequence of a meganuclease recognition sequence downstream of the *FMR1* CGG repeat.
SEQ ID NO: 5 sets forth the nucleic acid sequence of a meganuclease recognition sequence upstream of the *HTT* CAG Repeat.
SEQ ID NO: 6 sets forth the nucleic acid sequence of a meganuclease recognition sequence upstream of the *HTT* CAG Repeat.
SEQ ID NO: 7 sets forth the nucleic acid sequence of a 5' region of *HTT* Exon 1 upstream of CAG repeat.
SEQ ID NO: 8 sets forth the nucleic acid sequence of a meganuclease recognition sequence downstream of the *HTT* CAG repeat.
SEQ ID NO: 9 sets forth the nucleic acid sequence of a meganuclease recognition sequence downstream of the *HTT* CAG repeat.
SEQ ID NO: 10 sets forth the nucleic acid sequence of a meganuclease recognition sequence downstream of the *HTT* CAG repeat.
SEQ ID NO: 11 sets forth the nucleic acid sequence of a 3' region of *HTT* Exon 1 downstream of CAG repeat.
SEQ ID NO: 12 sets forth the nucleic acid sequence of a meganuclease recognition sequence upstream of *FXN* GAA repeat.
SEQ ID NO: 13 sets forth the nucleic acid sequence of a meganuclease recognition sequence upstream of *FXN* GAA repeat.
SEQ ID NO: 14 sets forth the nucleic acid sequence of a meganuclease recognition sequence upstream of *FXN* GAA repeat.
SEQ ID NO: 15 sets forth the nucleic acid sequence of a meganuclease recognition sequence upstream of *FXN* GAA repeat.
SEQ ID NO: 16 sets forth the nucleic acid sequence of a meganuclease recognition sequence upstream of *FXN* GAA repeat.
SEQ ID NO: 17 sets forth the nucleic acid sequence of a meganuclease recognition sequence upstream of *FXN* GAA repeat.
SEQ ID NO: 18 sets forth the nucleic acid sequence of a meganuclease recognition sequence upstream of *FXN* GAA repeat.
SEQ ID NO: 19 sets forth the nucleic acid sequence of a meganuclease recognition sequence upstream of *FXN* GAA repeat.
SEQ ID NO: 20 sets forth the nucleic acid sequence of a meganuclease recognition sequence upstream of *FXN* GAA repeat.
SEQ ID NO: 21 sets forth the nucleic acid sequence of a meganuclease recognition sequence upstream of *FXN* GAA repeat.
SEQ ID NO: 22 sets forth the nucleic acid sequence of a meganuclease recognition sequence upstream of *FXN* GAA repeat.
SEQ ID NO: 23 sets forth the nucleic acid sequence of a meganuclease recognition sequence upstream of *FXN* GAA repeat.
SEQ ID NO: 24 sets forth the nucleic acid sequence of a meganuclease recognition sequence upstream of *FXN* GAA repeat.
SEQ ID NO: 25 sets forth the nucleic acid sequence of a meganuclease recognition sequence upstream of *FXN* GAA repeat.
SEQ ID NO: 26 sets forth the nucleic acid sequence of a meganuclease recognition sequence upstream of *FXN* GAA repeat.
SEQ ID NO: 27 sets forth the nucleic acid sequence of a meganuclease recognition sequence upstream of *FXN* GAA repeat.
SEQ ID NO: 28 sets forth the nucleic acid sequence of a meganuclease recognition sequence upstream of *FXN* GAA repeat.
SEQ ID NO: 29 sets forth the nucleic acid sequence of a meganuclease recognition sequence upstream of *FXN* GAA repeat.
SEQ ID NO: 30 sets forth the nucleic acid sequence of a meganuclease recognition sequence upstream of *FXN* GAA repeat.
SEQ ID NO: 31 sets forth the nucleic acid sequence of a meganuclease recognition sequence upstream of *FXN* GAA repeat.
SEQ ID NO: 32 sets forth the nucleic acid sequence of a meganuclease recognition sequence upstream of *FXN* GAA repeat.
SEQ ID NO: 33 sets forth the nucleic acid sequence of a meganuclease recognition sequence upstream of *FXN* GAA repeat.
SEQ ID NO: 34 sets forth the nucleic acid sequence of a meganuclease recognition sequence upstream of *FXN* GAA repeat.
SEQ ID NO: 35 sets forth the nucleic acid sequence of a meganuclease recognition sequence upstream of *FXN* GAA repeat.
SEQ ID NO: 36 sets forth the nucleic acid sequence of a meganuclease recognition sequence upstream of *FXN* GAA repeat.
SEQ ID NO: 37 sets forth the nucleic acid sequence of a meganuclease recognition sequence upstream of *FXN* GAA repeat.
SEQ ID NO: 38 sets forth the nucleic acid sequence of a meganuclease recognition sequence upstream of *FXN* GAA repeat.
SEQ ID NO: 39 sets forth the nucleic acid sequence of a meganuclease recognition sequence upstream of *FXN* GAA repeat.
SEQ ID NO: 40 sets forth the nucleic acid sequence of a meganuclease recognition sequence upstream of *FXN* GAA repeat.
SEQ ID NO: 41 sets forth the nucleic acid sequence of a meganuclease recognition sequence upstream of *FXN* GAA repeat.
SEQ ID NO: 42 sets forth the nucleic acid sequence of a meganuclease recognition sequence upstream of *FXN* GAA repeat.
SEQ ID NO: 43 sets forth the nucleic acid sequence of a meganuclease recognition sequence upstream of *FXN* GAA repeat.
SEQ ID NO: 44 sets forth the nucleic acid sequence of a meganuclease recognition sequence upstream of *FXN* GAA repeat.
SEQ ID NO: 45 sets forth the nucleic acid sequence of a meganuclease recognition sequence upstream of *FXN* GAA repeat.
SEQ ID NO: 46 sets forth the nucleic acid sequence of a meganuclease recognition sequence upstream of *FXN* GAA repeat.
SEQ ID NO: 47 sets forth the nucleic acid sequence of a meganuclease recognition sequence upstream of *FXN* GAA repeat.
SEQ ID NO: 48 sets forth the nucleic acid sequence of a meganuclease recognition sequence upstream of *FXN* GAA repeat.
SEQ ID NO: 49 sets forth the nucleic acid sequence of a meganuclease recognition sequence upstream of *FXN* GAA repeat.
SEQ ID NO: 50 sets forth the nucleic acid sequence of a meganuclease recognition sequence upstream of *FXN* GAA repeat.
SEQ ID NO: 51 sets forth the nucleic acid sequence of a meganuclease recognition sequence upstream of *FXN* GAA repeat.
SEQ ID NO: 52 sets forth the nucleic acid sequence of a meganuclease recognition sequence upstream of *FXN* GAA repeat.
SEQ ID NO: 53 sets forth the nucleic acid sequence of a meganuclease recognition sequence upstream of *FXN* GAA repeat.
SEQ ID NO: 54 sets forth the nucleic acid sequence of a meganuclease recognition sequence upstream of *FXN* GAA repeat.
SEQ ID NO: 55 sets forth the nucleic acid sequence of a meganuclease recognition sequence upstream of *FXN* GAA repeat.
SEQ ID NO: 56 sets forth the nucleic acid sequence of a meganuclease recognition sequence upstream of *FXN* GAA repeat.
SEQ ID NO: 57 sets forth the nucleic acid sequence of a meganuclease recognition sequence upstream of *FXN* GAA repeat.
SEQ ID NO: 58 sets forth the nucleic acid sequence of a meganuclease recognition sequence upstream of *FXN* GAA repeat.
SEQ ID NO: 59 sets forth the nucleic acid sequence of a meganuclease recognition sequence upstream of *FXN* GAA repeat.
SEQ ID NO: 60 sets forth the nucleic acid sequence of a meganuclease recognition sequence upstream of *FXN* GAA repeat.
SEQ ID NO: 61 sets forth the nucleic acid sequence of a meganuclease recognition sequence upstream of *FXN* GAA repeat.
SEQ ID NO: 62 sets forth the nucleic acid sequence of a meganuclease recognition sequence upstream of *FXN* GAA repeat.
SEQ ID NO: 63 sets forth the nucleic acid sequence of a meganuclease recognition sequence upstream of *FXN* GAA repeat.
SEQ ID NO: 64 sets forth the nucleic acid sequence of a meganuclease recognition sequence upstream of *FXN* GAA repeat.
SEQ ID NO: 65 sets forth the nucleic acid sequence of a meganuclease recognition sequence upstream of *FXN* GAA repeat.
SEQ ID NO: 66 sets forth the nucleic acid sequence of a meganuclease recognition sequence upstream of *FXN* GAA repeat.
SEQ ID NO: 67 sets forth the nucleic acid sequence of a meganuclease recognition sequence upstream of *FXN* GAA repeat.
SEQ ID NO: 68 sets forth the nucleic acid sequence of a meganuclease recognition sequence upstream of *FXN* GAA repeat.
SEQ ID NO: 69 sets forth the nucleic acid sequence of a meganuclease recognition sequence upstream of *FXN* GAA repeat.
SEQ ID NO: 70 sets forth the nucleic acid sequence of a meganuclease recognition sequence upstream of *FXN* GAA repeat.
SEQ ID NO: 71 sets forth the nucleic acid sequence of a meganuclease recognition sequence upstream of *FXN* GAA repeat.
SEQ ID NO: 72 sets forth the nucleic acid sequence of a meganuclease recognition sequence upstream of *FXN* GAA repeat.
SEQ ID NO: 73 sets forth the nucleic acid sequence of a meganuclease recognition sequence upstream of *FXN* GAA repeat.
SEQ ID NO: 74 sets forth the nucleic acid sequence of a *FXN* Intron 1 upstream of GAA repeat.
SEQ ID NO: 75 sets forth the nucleic acid sequence of a meganuclease recognition sequence downstream of *FXN* GAA repeat.
SEQ ID NO: 76 sets forth the nucleic acid sequence of a meganuclease recognition sequence downstream of *FXN* GAA repeat.
SEQ ID NO: 77 sets forth the nucleic acid sequence of a meganuclease recognition sequence downstream of *FXN* GAA repeat.
SEQ ID NO: 78 sets forth the nucleic acid sequence of a meganuclease recognition sequence downstream of *FXN* GAA repeat.
SEQ ID NO: 79 sets forth the nucleic acid sequence of a meganuclease recognition sequence downstream of *FXN* GAA repeat.
SEQ ID NO: 80 sets forth the nucleic acid sequence of a meganuclease recognition sequence downstream of *FXN* GAA repeat.
SEQ ID NO: 81 sets forth the nucleic acid sequence of a meganuclease recognition sequence downstream of *FXN* GAA repeat.
SEQ ID NO: 82 sets forth the nucleic acid sequence of a meganuclease recognition sequence downstream of *FXN* GAA repeat.
SEQ ID NO: 83 sets forth the nucleic acid sequence of a meganuclease recognition sequence downstream of *FXN* GAA repeat.
SEQ ID NO: 84 sets forth the nucleic acid sequence of a meganuclease recognition sequence downstream of *FXN* GAA repeat.
SEQ ID NO: 85 sets forth the nucleic acid sequence of a meganuclease recognition sequence downstream of *FXN* GAA repeat.
SEQ ID NO: 86 sets forth the nucleic acid sequence of a meganuclease recognition sequence downstream of *FXN* GAA repeat.
SEQ ID NO: 87 sets forth the nucleic acid sequence of a meganuclease recognition sequence downstream of *FXN* GAA repeat.
SEQ ID NO: 88 sets forth the nucleic acid sequence of a meganuclease recognition sequence downstream of *FXN* GAA repeat.
SEQ ID NO: 89 sets forth the nucleic acid sequence of a meganuclease recognition sequence downstream of *FXN* GAA repeat.
SEQ ID NO: 90 sets forth the nucleic acid sequence of a meganuclease recognition sequence downstream of *FXN* GAA repeat.
SEQ ID NO: 91 sets forth the nucleic acid sequence of a meganuclease recognition sequence downstream of *FXN* GAA repeat.
SEQ ID NO: 92 sets forth the nucleic acid sequence of a meganuclease recognition sequence downstream of *FXN* GAA repeat.
SEQ ID NO: 93 sets forth the nucleic acid sequence of a meganuclease recognition sequence downstream of *FXN* GAA repeat.
SEQ ID NO: 94 sets forth the nucleic acid sequence of a meganuclease recognition sequence downstream of *FXN* GAA repeat.
SEQ ID NO: 95 sets forth the nucleic acid sequence of a meganuclease recognition sequence downstream of *FXN* GAA repeat.
SEQ ID NO: 96 sets forth the nucleic acid sequence of a *FXN* intron 1 downstream of GAA repeat.
SEQ ID NO: 97 sets forth the nucleic acid sequence of a meganuclease recognition sequence upstream of the *DMPK* CTG repeat.
SEQ ID NO: 98 sets forth the nucleic acid sequence of a meganuclease recognition sequence upstream of the *DMPK* CTG repeat.
SEQ ID NO: 99 sets forth the nucleic acid sequence of a meganuclease recognition sequence upstream of the *DMPK* CTG repeat.
SEQ ID NO: 100 sets forth the nucleic acid sequence of a meganuclease recognition sequence upstream of the *DMPK* CTG repeat.
SEQ ID NO: 101 sets forth the nucleic acid sequence of a meganuclease recognition sequence upstream of the *DMPK* CTG repeat.
SEQ ID NO: 102 sets forth the nucleic acid sequence of a *DMPK* 3' UTR upstream of CTG repeat.
SEQ ID NO: 103 sets forth the nucleic acid sequence of a meganuclease recognition sequence downstream of the *DMPK* CTG repeat.
SEQ ID NO: 104 sets forth the nucleic acid sequence of a meganuclease recognition sequence downstream of the *DMPK* CTG repeat.
SEQ ID NO: 105 sets forth the nucleic acid sequence of a meganuclease recognition sequence downstream of the *DMPK* CTG repeat.
SEQ ID NO: 106 sets forth the nucleic acid sequence of a meganuclease recognition sequence downstream of the *DMPK* CTG repeat.
SEQ ID NO: 107 sets forth the nucleic acid sequence of a meganuclease recognition sequence downstream of the *DMPK* CTG repeat.
SEQ ID NO: 108 sets forth the nucleic acid sequence of a meganuclease recognition sequence downstream of the *DMPK* CTG repeat.
SEQ ID NO: 109 sets forth the nucleic acid sequence of a meganuclease recognition sequence downstream of the *DMPK* CTG repeat.
SEQ ID NO: 110 sets forth the nucleic acid sequence of a meganuclease recognition sequence downstream of the *DMPK* CTG repeat.
SEQ ID NO: 111 sets forth the nucleic acid sequence of a meganuclease recognition sequence downstream of the *DMPK* CTG repeat.
SEQ ID NO: 112 sets forth the nucleic acid sequence of a meganuclease recognition sequence downstream of the *DMPK* CTG repeat.
SEQ ID NO: 113 sets forth the nucleic acid sequence of a meganuclease recognition sequence downstream of the *DMPK* CTG repeat.
SEQ ID NO: 114 sets forth the nucleic acid sequence of a meganuclease recognition sequence downstream of the *DMPK* CTG repeat.
SEQ ID NO: 115 sets forth the nucleic acid sequence of a meganuclease recognition sequence downstream of the *DMPK* CTG repeat.
SEQ ID NO: 116 sets forth the nucleic acid sequence of a meganuclease recognition sequence downstream of the *DMPK* CTG repeat.
SEQ ID NO: 117 sets forth the nucleic acid sequence of a meganuclease recognition sequence downstream of the *DMPK* CTG repeat.
SEQ ID NO: 118 sets forth the nucleic acid sequence of a meganuclease recognition sequence downstream of the *DMPK* CTG repeat.
SEQ ID NO: 119 sets forth the nucleic acid sequence of a meganuclease recognition sequence downstream of the *DMPK* CTG repeat.
SEQ ID NO: 120 sets forth the nucleic acid sequence of a meganuclease recognition sequence downstream of the *DMPK* CTG repeat.
SEQ ID NO: 121 sets forth the nucleic acid sequence of a meganuclease recognition sequence downstream of the *DMPK* CTG repeat.
SEQ ID NO: 122 sets forth the nucleic acid sequence of a meganuclease recognition sequence downstream of the *DMPK* CTG repeat.
SEQ ID NO: 123 sets forth the nucleic acid sequence of a meganuclease recognition sequence downstream of the *DMPK* CTG repeat.
SEQ ID NO: 124 sets forth the nucleic acid sequence of a meganuclease recognition sequence downstream of the *DMPK* CTG repeat.
SEQ ID NO: 125 sets forth the nucleic acid sequence of a meganuclease recognition sequence downstream of the *DMPK* CTG repeat.
SEQ ID NO: 126 sets forth the nucleic acid sequence of a meganuclease recognition sequence downstream of the *DMPK* CTG repeat.
SEQ ID NO: 127 sets forth the nucleic acid sequence of a meganuclease recognition sequence downstream of the *DMPK* CTG repeat.
SEQ ID NO: 128 sets forth the nucleic acid sequence of a meganuclease recognition sequence downstream of the *DMPK* CTG repeat.
SEQ ID NO: 129 sets forth the nucleic acid sequence of a meganuclease recognition sequence downstream of the *DMPK* CTG repeat.
SEQ ID NO: 130 sets forth the nucleic acid sequence of a meganuclease recognition sequence downstream of the *DMPK* CTG repeat.
SEQ ID NO: 131 sets forth the nucleic acid sequence of a meganuclease recognition sequence downstream of the *DMPK* CTG repeat.
SEQ ID NO: 132 sets forth the nucleic acid sequence of a *DMPK* 3' UTR downstream of CTG repeat and *DMPK*-SIX5 intergenic region.
SEQ ID NO: 133 sets forth the amino acid sequence of a FXN 1-2x.63 meganuclease comprising an N-terminal nuclease-localization signal derived from SV40.
SEQ ID NO: 134 sets forth the amino acid sequence of a FXN 11-12x.63 meganuclease comprising an N-terminal nuclease-localization signal derived from SV40.
SEQ ID NO: 135 sets forth the amino acid sequence of a FXN 5-6x.24 meganuclease comprising an N-terminal nuclease-localization signal derived from SV40.
SEQ ID NO: 136 sets forth the nucleic acid sequence of a FXN 1-2x.63 PCR template for mRNA.
SEQ ID NO: 137 sets forth the nucleic acid sequence of a FXN 11-12x.63 PCR template for mRNA.
SEQ ID NO: 138 sets forth the nucleic acid sequence of a FXN 5-6x.24 PCR template for mRNA.
SEQ ID NO: 139 sets forth the nucleic acid sequence of a *FXN* upstream forward PCR primer.
SEQ ID NO: 140 sets forth the nucleic acid sequence of a *FXN* downstream reverse PCR primer.
SEQ ID NO: 141 sets forth the nucleic acid sequence of a meganuclease recognition sequence upstream of the *HTT* CAG repeat.
SEQ ID NO: 142 sets forth the nucleic acid sequence of a 5' UTR and translation start site of the *FMR1* gene downstream of CGG repeat.
SEQ ID NO: 143 sets forth the amino acid sequence of a FXN 1-2x.63 meganuclease.
SEQ ID NO: 144 sets forth the amino acid sequence of a FXN 1-2x.11 meganuclease.
SEQ ID NO: 145 sets forth the amino acid sequence of a FXN 1-2x.73 meganuclease.
SEQ ID NO: 146 sets forth the amino acid sequence of a FXN 1-2x.84 meganuclease.
SEQ ID NO: 147 sets forth the amino acid sequence of a FXN 1-2x.63 meganuclease FXN1-binding subunit.
SEQ ID NO: 148 sets forth the amino acid sequence of a FXN 1-2x.11 meganuclease FXN1-binding subunit.
SEQ ID NO: 149 sets forth the amino acid sequence of a FXN 1-2x.73 meganuclease FXN1-binding subunit.
SEQ ID NO: 150 sets forth the amino acid sequence of a FXN 1-2x.84 meganuclease FXN1-binding subunit.
SEQ ID NO: 151 sets forth the amino acid sequence of a FXN 1-2x.63 meganuclease FXN2-binding subunit.
SEQ ID NO: 152 sets forth the amino acid sequence of a FXN 1-2x.11 meganuclease FXN2-binding subunit.
SEQ ID NO: 153 sets forth the amino acid sequence of a FXN 1-2x.73 meganuclease FXN2-binding subunit.
SEQ ID NO: 154 sets forth the amino acid sequence of a FXN 1-2x.84 meganuclease FXN2-binding subunit.
SEQ ID NO: 155 sets forth the amino acid sequence of a FXN 3-4L.34 meganuclease.
SEQ ID NO: 156 sets forth the amino acid sequence of a FXN 3-4L.5 meganuclease.
SEQ ID NO: 157 sets forth the amino acid sequence of a FXN 3-4L.12 meganuclease.
SEQ ID NO: 158 sets forth the amino acid sequence of a FXN 3-4x.312 meganuclease.
SEQ ID NO: 159 sets forth the amino acid sequence of a FXN 3-4x.383 meganuclease.
SEQ ID NO: 160 sets forth the amino acid sequence of a FXN 3-4L.34 meganuclease FXN3-binding subunit.
SEQ ID NO: 161 sets forth the amino acid sequence of a FXN 3-4L.5 meganuclease FXN3-binding subunit.
SEQ ID NO: 162 sets forth the amino acid sequence of a FXN 3-4L.12 meganuclease FXN3-binding subunit.
SEQ ID NO: 163 sets forth the amino acid sequence of a FXN 3-4x.312 meganuclease FXN3-binding subunit.
SEQ ID NO: 164 sets forth the amino acid sequence of a FXN 3-4x.383 meganuclease FXN3-binding subunit.
SEQ ID NO: 165 sets forth the amino acid sequence of a FXN 3-4L.34 meganuclease FXN4-binding subunit.
SEQ ID NO: 166 sets forth the amino acid sequence of a FXN 3-4L.5 meganuclease FXN4-binding subunit.
SEQ ID NO: 167 sets forth the amino acid sequence of a FXN 3-4L.12 meganuclease FXN4-binding subunit.
SEQ ID NO: 168 sets forth the amino acid sequence of a FXN 3-4x.312 meganuclease FXN4-binding subunit.
SEQ ID NO: 169 sets forth the amino acid sequence of a FXN 3-4x.383 meganuclease FXN4-binding subunit.
SEQ ID NO: 170 sets forth the amino acid sequence of a FXN 5-6L.45 meganuclease.
SEQ ID NO: 171 sets forth the amino acid sequence of a FXN 5-6L.38 meganuclease.
SEQ ID NO: 172 sets forth the amino acid sequence of a FXN 5-6x.24 meganuclease.
SEQ ID NO: 173 sets forth the amino acid sequence of a FXN 5-6x.20 meganuclease.
SEQ ID NO: 174 sets forth the amino acid sequence of a FXN 5-6L.45 meganuclease FXN5-binding subunit.
SEQ ID NO: 175 sets forth the amino acid sequence of a FXN 5-6L.38 meganuclease FXN5-binding subunit.
SEQ ID NO: 176 sets forth the amino acid sequence of a FXN 5-6x.24 meganuclease FXN5-binding subunit.
SEQ ID NO: 177 sets forth the amino acid sequence of a FXN 5-6x.20 meganuclease FXN5-binding subunit.
SEQ ID NO: 178 sets forth the amino acid sequence of a FXN 5-6L.45 meganuclease FXN6-binding subunit.
SEQ ID NO: 179 sets forth the amino acid sequence of a FXN 5-6L.38 meganuclease FXN6-binding subunit.
SEQ ID NO: 180 sets forth the amino acid sequence of a FXN 5-6x.24 meganuclease FXN6-binding subunit.
SEQ ID NO: 181 sets forth the amino acid sequence of a FXN 5-6x.20 meganuclease FXN6-binding subunit.
SEQ ID NO: 182 sets forth the amino acid sequence of a FXN 11-12x.63 meganuclease.
SEQ ID NO: 183 sets forth the amino acid sequence of a FXN 11-12x.99 meganuclease.
SEQ ID NO: 184 sets forth the amino acid sequence of a FXN 11-12x.107 meganuclease.
SEQ ID NO: 185 sets forth the amino acid sequence of a FXN 11-12x.139 meganuclease.
SEQ ID NO: 186 sets forth the amino acid sequence of a FXN 11-12x.63 meganuclease FXN11-binding subunit.
SEQ ID NO: 187 sets forth the amino acid sequence of a FXN 11-12x.99 meganuclease FXN11-binding subunit.
SEQ ID NO: 188 sets forth the amino acid sequence of a FXN 11-12x.107 meganuclease FXN11-binding subunit.
SEQ ID NO: 189 sets forth the amino acid sequence of a FXN 11-12x.139 meganuclease FXN11-binding subunit.
SEQ ID NO: 190 sets forth the amino acid sequence of a FXN 11-12x.63 meganuclease FXN12-binding subunit.
SEQ ID NO: 191 sets forth the amino acid sequence of a FXN 11-12x.99 meganuclease FXN12-binding subunit.
SEQ ID NO: 192 sets forth the amino acid sequence of a FXN 11-12x.107 meganuclease FXN12-binding subunit.
SEQ ID NO: 193 sets forth the amino acid sequence of a FXN 11-12x.139 meganuclease FXN12-binding subunit.
SEQ ID NO: 194 sets forth the nucleic acid sequence of an ORF 7-8 recognition sequence.
SEQ ID NO: 195 sets forth the nucleic acid sequence of an ORF 9-10 recognition sequence.
SEQ ID NO: 196 sets forth the nucleic acid sequence of an ORF 11-12 recognition sequence.
SEQ ID NO: 197 sets forth the nucleic acid sequence of an ORF 13-14 recognition sequence.
SEQ ID NO: 198 sets forth the amino acid sequence of an ORF 7-8x.4 meganuclease.
SEQ ID NO: 199 sets forth the amino acid sequence of an ORF 7-8x.31 meganuclease.
SEQ ID NO: 200 sets forth the amino acid sequence of an ORF 7-8x.45 meganuclease.
SEQ ID NO: 201 sets forth the amino acid sequence of an ORF 7-8x.29 meganuclease.
SEQ ID NO: 202 sets forth the amino acid sequence of an ORF 7-8x.4 meganuclease ORF7-binding subunit.
SEQ ID NO: 203 sets forth the amino acid sequence of an ORF 7-8x.31 meganuclease ORF7-binding subunit.
SEQ ID NO: 204 sets forth the amino acid sequence of an ORF 7-8x.45 meganuclease ORF7-binding subunit.
SEQ ID NO: 205 sets forth the amino acid sequence of an ORF 7-8x.29 meganuclease ORF7-binding subunit.
SEQ ID NO: 206 sets forth the amino acid sequence of an ORF 7-8x.4 meganuclease ORF8-binding subunit.
SEQ ID NO: 207 sets forth the amino acid sequence of an ORF 7-8x.31 meganuclease ORF8-binding subunit.
SEQ ID NO: 208 sets forth the amino acid sequence of an ORF 7-8x.45 meganuclease ORF8-binding subunit.
SEQ ID NO: 209 sets forth the amino acid sequence of an ORF 7-8x.29 meganuclease ORF8-binding subunit.
SEQ ID NO: 210 sets forth the amino acid sequence of an ORF 9-10x.15 meganuclease.
SEQ ID NO: 211 sets forth the amino acid sequence of an ORF 9-10x.14 meganuclease.
SEQ ID NO: 212 sets forth the amino acid sequence of an ORF 9-10x.61 meganuclease.
SEQ ID NO: 213 sets forth the amino acid sequence of an ORF 9-10x.77 meganuclease.
SEQ ID NO: 214 sets forth the amino acid sequence of an ORF 9-10x.15 meganuclease ORF9-binding subunit.
SEQ ID NO: 215 sets forth the amino acid sequence of an ORF 9-10x.14 meganuclease ORF9-binding subunit.
SEQ ID NO: 216 sets forth the amino acid sequence of an ORF 9-10x.61 meganuclease ORF9-binding subunit.
SEQ ID NO: 217 sets forth the amino acid sequence of an ORF 9-10x.77 meganuclease ORF9-binding subunit.
SEQ ID NO: 218 sets forth the amino acid sequence of an ORF 9-10x.15 meganuclease ORF10-binding subunit.
SEQ ID NO: 219 sets forth the amino acid sequence of an ORF 9-10x.14 meganuclease ORF10-binding subunit.
SEQ ID NO: 220 sets forth the amino acid sequence of an ORF 9-10x.61 meganuclease ORF10-binding subunit.
SEQ ID NO: 221 sets forth the amino acid sequence of an ORF 9-10x.77 meganuclease ORF10-binding subunit.
SEQ ID NO: 222 sets forth the amino acid sequence of an ORF 11-12x.5 meganuclease.
SEQ ID NO: 223 sets forth the amino acid sequence of an ORF 11-12L.1 meganuclease.
SEQ ID NO: 224 sets forth the amino acid sequence of an ORF 11-12L.8 meganuclease.
SEQ ID NO: 225 sets forth the amino acid sequence of an ORF 11-12L.64 meganuclease.
SEQ ID NO: 226 sets forth the amino acid sequence of an ORF 11-12x.5 meganuclease ORF11-binding subunit.
SEQ ID NO: 227 sets forth the amino acid sequence of an ORF 11-12L.1 meganuclease ORF11-binding subunit.
SEQ ID NO: 228 sets forth the amino acid sequence of an ORF 11-12L.8 meganuclease ORF11-binding subunit.
SEQ ID NO: 229 sets forth the amino acid sequence of an ORF 11-12L.64 meganuclease ORF11-binding subunit.
SEQ ID NO: 230 sets forth the amino acid sequence of an ORF 11-12x.5 meganuclease ORF12-binding subunit.
SEQ ID NO: 231 sets forth the amino acid sequence of an ORF 11-12L.1 meganuclease ORF12-binding subunit.
SEQ ID NO: 232 sets forth the amino acid sequence of an ORF 11-12L.8 meganuclease ORF12-binding subunit.
SEQ ID NO: 233 sets forth the amino acid sequence of an ORF 11-12L.64 meganuclease ORF12-binding subunit.
SEQ ID NO: 234 sets forth the amino acid sequence of an ORF 13-14x.40 meganuclease.
SEQ ID NO: 235 sets forth the amino acid sequence of an ORF 13-14x.77 meganuclease.
SEQ ID NO: 236 sets forth the amino acid sequence of an ORF 13-14x.90 meganuclease.
SEQ ID NO: 237 sets forth the amino acid sequence of an ORF 13-14x.3 meganuclease.
SEQ ID NO: 238 sets forth the amino acid sequence of an ORF 13-14x.40 meganuclease ORF13-binding subunit.
SEQ ID NO: 239 sets forth the amino acid sequence of an ORF 13-14x.77 meganuclease ORF13-binding subunit.
SEQ ID NO: 240 sets forth the amino acid sequence of an ORF 13-14x.90 meganuclease ORF13-binding subunit.
SEQ ID NO: 241 sets forth the amino acid sequence of an ORF 13-14x.3 meganuclease ORF13-binding subunit.
SEQ ID NO: 242 sets forth the amino acid sequence of an ORF 13-14x.40 meganuclease ORF14-binding subunit.
SEQ ID NO: 243 sets forth the amino acid sequence of an ORF 13-14x.77 meganuclease ORF14-binding subunit.
SEQ ID NO: 244 sets forth the amino acid sequence of an ORF 13-14x.90 meganuclease ORF14-binding subunit.
SEQ ID NO: 245 sets forth the amino acid sequence of an ORF 13-14x.3 meganuclease ORF14-binding subunit.
SEQ ID NO: 246 sets forth the nucleic acid sequence of *FXN* intron 1 flanking the GAA repeat associated with Friedreich's Ataxia.
SEQ ID NO: 247 sets forth the nucleic acid sequence of a FXN clone #1 having a 713 bp deletion.
SEQ ID NO: 248 sets forth the nucleic acid sequence of a FXN clone #6 having a 596 bp deletion and a 5 bp insertion.
SEQ ID NO: 249 sets forth the nucleic acid sequence of a FXN clone #2 having a 758 bp deletion and a 29 bp insertion.
SEQ ID NO: 250 sets forth the nucleic acid sequence of a FXN clone #3 having a 790 bp deletion.

### DETAILED DESCRIPTION OF THE INVENTION

### 1.1 References and Definitions

The patent and scientific literature referred to herein establishes knowledge that is available to those of skill in the art. The issued U.S. patents, allowed applications, published foreign applications, and references, including GenBank database sequences, that are cited herein are hereby incorporated by reference to the same extent as if each was specifically and individually indicated to be incorporated by reference.

The present invention can be embodied in different forms and should not be construed as limited to the embodiments set forth herein. Rather, these embodiments are provided so that this disclosure will be thorough and complete, and will fully convey the scope of the invention to those skilled in the art. For example, features illustrated with respect to one embodiment can be incorporated into other embodiments, and features illustrated with respect to a particular embodiment can be deleted from that embodiment. In addition, numerous variations and additions to the embodiments suggested herein will be apparent to those skilled in the art in light of the instant disclosure, which do not depart from the instant invention.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

All publications, patent applications, patents, and other references mentioned herein are incorporated by reference herein in their entirety.

As used herein, "a," "an," or "the" can mean one or more than one. For example, "a" cell can mean a single cell or a multiplicity of cells.

As used herein, unless specifically indicated otherwise, the word "or" is used in the inclusive sense of "and/or" and not the exclusive sense of "either/or."

As used herein, the terms "nuclease" and "endonuclease" are used interchangeably to refer to naturally-occurring or engineered enzymes which cleave a phosphodiester bond within a polynucleotide chain.

As used herein, the term "meganuclease" refers to an endonuclease that is derived from I-CreI. The term meganuclease, as used herein, refers to an engineered variant of I-CreI that has been modified relative to natural I-CreI with respect to, for example, DNA-binding specificity, DNA cleavage activity, DNA-binding affinity, or dimerization properties. Methods for producing such modified variants of I-CreI are known in the art (*e.g.* WO 2007/047859). A meganuclease may bind to double-stranded DNA as a homodimer, as is the case for wild-type I-CreI, or it may bind to DNA as a heterodimer. A meganuclease may also be a "single-chain meganuclease" in which a pair of DNA-binding domains derived from I-CreI are joined into a single polypeptide using a peptide linker. The term "homing endonuclease" is synonymous with the term "meganuclease." Meganucleases of the invention are substantially non-toxic when expressed in cells without observing deleterious effects on cell viability or significant reductions in meganuclease cleavage activity when measured using the methods described herein.

As used herein, the term "single-chain meganuclease" refers to a polypeptide comprising a pair of meganuclease subunits joined by a linker. A single-chain meganuclease has the organization: N-terminal subunit - Linker - C-terminal subunit. The two meganuclease subunits, each of which is derived from I-CreI, will generally be non-identical in amino acid sequence and will recognize non-identical DNA sequences. Thus, single-chain meganucleases typically cleave pseudo-palindromic or non-palindromic recognition sequences. A single chain meganuclease may be referred to as a "single-chain heterodimer" or "single-chain heterodimeric meganuclease" although it is not, in fact, dimeric. For clarity, unless otherwise specified, the term "meganuclease" can refer to a dimeric or single-chain meganuclease.

As used herein, the term "linker" refers to an exogenous peptide sequence used to join two meganuclease subunits into a single polypeptide. A linker may have a sequence that is found in natural proteins, or may be an artificial sequence that is not found in any natural protein. A linker may be flexible and lacking in secondary structure or may have a propensity to form a specific three-dimensional structure under physiological conditions. A linker can include, without limitation, those encompassed by U.S. Patent No. 8,445,251. In some embodiments, a linker may have an amino acid sequence comprising residues 154-195 of any one of SEQ ID NOs: 143-146, 155-159, 170-173, 182-185, 198-201, 210-213, 222-225, or 234-237.

As used herein, the term "TALEN" refers to an endonuclease comprising a DNA-binding domain comprising 16-22 TAL domain repeats fused to any portion of the FokI nuclease domain.

As used herein, the term "Compact TALEN" refers to an endonuclease comprising a DNA-binding domain with 16-22 TAL domain repeats fused in any orientation to any portion of the I-TevI homing endonuclease.

As used herein, the term "zinc finger nuclease" or "ZFN" refers to a chimeric endonuclease comprising a zinc finger DNA-binding domain fused to the nuclease domain of the FokI restriction enzyme. The zinc finger domain can be redesigned through rational or experimental means to produce a protein which binds to a pre-determined DNA sequence ~18 basepairs in length, comprising a pair of nine basepair half-sites separated by 2-10 basepairs. Cleavage by a zinc finger nuclease can create a blunt end or a 5' overhand of variable length (frequently four basepairs).

As used herein, the term "CRISPR" refers to a caspase-based endonuclease comprising a caspase, such as Cas9, and a guide RNA that directs DNA cleavage of the caspase by hybridizing to a recognition site in the genomic DNA.

As used herein, the term "megaTAL" refers to a single-chain endonuclease comprising a transcription activator-like effector (TALE) DNA binding domain with an engineered, sequence-specific homing endonuclease.

As used herein, with respect to a protein, the term "recombinant" means having an altered amino acid sequence as a result of the application of genetic engineering techniques to nucleic acids which encode the protein, and cells or organisms which express the protein. With respect to a nucleic acid, the term "recombinant" means having an altered nucleic acid sequence as a result of the application of genetic engineering techniques. Genetic engineering techniques include, but are not limited to, PCR and DNA cloning technologies; transfection, transformation and other gene transfer technologies; homologous recombination; site-directed mutagenesis; and gene fusion. In accordance with this definition, a protein having an amino acid sequence identical to a naturally-occurring protein, but produced by cloning and expression in a heterologous host, is not considered recombinant.

As used herein, the term "wild-type" refers to any naturally-occurring form of a meganuclease. The term "wild-type" is not intended to mean the most common allelic variant of the enzyme in nature but, rather, any allelic variant found in nature. Wild-type homing endonucleases are distinguished from recombinant or non-naturally-occurring meganucleases.

The term "wild-type" can also refer to the most common naturally occurring allele (*i.e.*, polynucleotide sequence) in the allele population of the same type of gene, wherein a polypeptide encoded by the wild-type allele has its original functions. The term "wild-type" also refers a polypeptide encoded by a wild-type allele. Wild-type alleles (*i.e.*, polynucleotides) and polypeptides are distinguishable from mutant or variant alleles and polypeptides, which comprise one or more mutations and/or substitutions relative to the wild-type sequence(s). Whereas a wild-type allele or polypeptide can confer a normal phenotype in an organism, a mutant or variant allele or polypeptide can, in some instances, confer an altered phenotype.

As used herein, the term "genetically-modified" refers to a cell or organism in which, or in an ancestor of which, a genomic DNA sequence has been deliberately modified by recombinant technology. As used herein, the term "genetically-modified" encompasses the term "transgenic."

As used herein with respect to recombinant proteins, the term "modification" means any insertion, deletion, or substitution of an amino acid residue in the recombinant sequence relative to a reference sequence (*e.g.*, a wild-type or a native sequence).

As used herein, the term "recognition sequence" refers to a DNA sequence that is bound and cleaved by an endonuclease. In the case of a meganuclease, a recognition sequence comprises a pair of inverted, 9 basepair "half sites" which are separated by four basepairs. In the case of a single-chain meganuclease, the N-terminal domain of the protein contacts a first half-site and the C-terminal domain of the protein contacts a second half-site. Cleavage by a meganuclease produces four basepair 3' "overhangs". "Overhangs", or "sticky ends" are short, single-stranded DNA segments that can be produced by endonuclease cleavage of a double-stranded DNA sequence. In the case of meganucleases and single-chain meganucleases derived from I-CreI, the overhang comprises bases 10-13 of the 22 basepair recognition sequence. In the case of a Compact TALEN, the recognition sequence comprises a first CNNNGN sequence that is recognized by the I-TevI domain, followed by a non-specific spacer 4-16 basepairs in length, followed by a second sequence 16-22 bp in length that is recognized by the TAL-effector domain (this sequence typically has a 5' T base). Cleavage by a Compact TALEN produces two basepair 3' overhangs. In the case of a CRISPR, the recognition sequence is the sequence, typically 16-24 basepairs, to which the guide RNA binds to direct Cas9 cleavage. Cleavage by a CRISPR produced blunt ends. In the case of a zinc finger, the DNA binding domains typically recognize an 18-bp recognition sequence comprising a pair of nine basepair "half-sites" separated by 2-10 basepairs and cleavage by the nuclease creates a blunt end or a 5' overhang of variable length (frequently four basepairs).

As used herein, the term "target site" or "target sequence" refers to a region of the chromosomal DNA of a cell comprising a recognition sequence for a meganuclease.

As used herein, the term "DNA-binding affinity" or "binding affinity" means the tendency of a meganuclease to non-covalently associate with a reference DNA molecule (*e.g.*, a recognition sequence or an arbitrary sequence). Binding affinity is measured by a dissociation constant, K_{d} . As used herein, a nuclease has "altered" binding affinity if the K_{d} of the nuclease for a reference recognition sequence is increased or decreased by a statistically significant (p<0.05) amount relative to a reference nuclease.

As used herein, the term "specificity" means the ability of a meganuclease to recognize and cleave double-stranded DNA molecules only at a particular sequence of base pairs referred to as the recognition sequence, or only at a particular set of recognition sequences. The set of recognition sequences will share certain conserved positions or sequence motifs, but may be degenerate at one or more positions. A highly-specific meganuclease is capable of cleaving only one or a very few recognition sequences. Specificity can be determined by any method known in the art. As used herein, a meganuclease has "altered" specificity if it binds to and cleaves a recognition sequence which is not bound to and cleaved by a reference meganuclease (*e.g.*, a wild-type) under physiological conditions, or if the rate of cleavage of a recognition sequence is increased or decreased by a biologically significant amount (e.g., at least 2×, or 2×-10×) relative to a reference meganuclease.

As used herein, the term "homologous recombination" or "HR" refers to the natural, cellular process in which a double-stranded DNA-break is repaired using a homologous DNA sequence as the repair template (see, *e.g.,* Cahill et al. (2006), Front. Biosci. 11:1958-1976). The homologous DNA sequence may be an endogenous chromosomal sequence or an exogenous nucleic acid that was delivered to the cell.

As used herein, the term "non-homologous end-joining" or "NHEJ" refers to the natural, cellular process in which a double-stranded DNA-break is repaired by the direct joining of two non-homologous DNA segments (see, *e.g.,* Cahill et al. (2006), Front. Biosci. 11:1958-1976). DNA repair by non-homologous end-joining is error-prone and frequently results in the untemplated addition or deletion of DNA sequences at the site of repair.

As used herein, the term "re-ligation" refers to a process in which two DNA ends produced by a pair of double-strand DNA breaks are covalently attached to one another with the loss of the intervening DNA sequence but without the gain or loss of any additional DNA sequence. In the case of a pair of DNA breaks that are produced with single-strand overhangs, re-ligation can proceed via annealing of complementary overhangs followed by covalent attachment of 5' and 3' ends by a DNA ligase. Re-ligation is distinguished from NHEJ in that it does not result in the untemplated addition or removal of DNA from the site of repair.

As used herein, the term "precise deletion" refers to a deletion of a segment of chromosomal DNA in which, after the introduction of double stranded breaks flanking the segment to be deleted by the first engineered nuclease and the second engineered nuclease, the chromosome is re-ligated without loss of any additional basepairs (e.g., by exonuclease activity degrading 3' or 5' overhangs) or insertion of any additional basepairs (e.g., by homologous recombination). Precise deletion allows the final sequence of the re-ligated chromosome to be predicted based upon the original sequence of the chromosome and the cleavage sites of the nucleases.

As used herein with respect to both amino acid sequences and nucleic acid sequences, the terms "percent identity," "sequence identity," "percentage similarity," "sequence similarity" and the like refer to a measure of the degree of similarity of two sequences based upon an alignment of the sequences which maximizes similarity between aligned amino acid residues or nucleotides, and which is a function of the number of identical or similar residues or nucleotides, the number of total residues or nucleotides, and the presence and length of gaps in the sequence alignment. A variety of algorithms and computer programs are available for determining sequence similarity using standard parameters. As used herein, sequence similarity is measured using the BLASTp program for amino acid sequences and the BLASTn program for nucleic acid sequences, both of which are available through the National Center for Biotechnology Information (www.ncbi.nlm.nih.gov/), and are described in, for example, Altschul et al. (1990), J. Mol. Biol. 215:403-410; Gish and States (1993), Nature Genet. 3:266-272; Madden et al. (1996), Meth. Enzymol.266:131-141; Altschul et al. (1997), Nucleic Acids Res. 25:3389-3402); Zhang et al. (2000), J. Comput. Biol. 7(1-2):203-14. As used herein, percent similarity of two amino acid sequences is the score based upon the following parameters for the BLASTp algorithm: word size=3; gap opening penalty=-11; gap extension penalty=-1; and scoring matrix=BLOSUM62. As used herein, percent similarity of two nucleic acid sequences is the score based upon the following parameters for the BLASTn algorithm: word size=11; gap opening penalty=-5; gap extension penalty=-2; match reward=1; and mismatch penalty=-3.

As used herein with respect to modifications of two proteins or amino acid sequences, the term "corresponding to" is used to indicate that a specified modification in the first protein is a substitution of the same amino acid residue as in the modification in the second protein, and that the amino acid position of the modification in the first proteins corresponds to or aligns with the amino acid position of the modification in the second protein when the two proteins are subjected to standard sequence alignments (*e.g.*, using the BLASTp program). Thus, the modification of residue "X" to amino acid "A" in the first protein will correspond to the modification of residue "Y" to amino acid "A" in the second protein if residues X and Y correspond to each other in a sequence alignment, and despite the fact that X and Y may be different numbers.

As used herein, the term "recognition half-site," "recognition sequence half-site," or simply "half-site" means a nucleic acid sequence in a double-stranded DNA molecule which is recognized by a monomer of a homodimeric or heterodimeric meganuclease, or by one subunit of a single-chain meganuclease.

As used herein, the term "hypervariable region" refers to a localized sequence within a meganuclease monomer or subunit that comprises amino acids with relatively high variability. A hypervariable region can comprise about 50-60 contiguous residues, about 53-57 contiguous residues, or preferably about 56 residues. In some embodiments, the residues of a hypervariable region may correspond to positions 24-79 or positions 215-270 of any one of SEQ ID NOs: 143-146, 155-159, 170-173, 182-185, 198-201, 210-213, 222-225, or 234-237. A hypervariable region can comprise one or more residues that contact DNA bases in a recognition sequence and can be modified to alter base preference of the monomer or subunit. A hypervariable region can also comprise one or more residues that bind to the DNA backbone when the meganuclease associates with a double-stranded DNA recognition sequence. Such residues can be modified to alter the binding affinity of the meganuclease for the DNA backbone and the target recognition sequence. In different embodiments of the invention, a hypervariable region may comprise between 1-20 residues, or more, that exhibit variability and can be modified to influence base preference and/or DNA-binding affinity.

As used herein, the terms "*FXN*" or "frataxin gene" refers to a human gene located on chromosome 9. In humans, the *FXN* gene is identified by NCBI as Gene ID No. 2395 and is located from base pair 69,035,563 to base pair 69,100,178 on chromosome 9.

As used herein, the term "*HTT*" or "huntingtin gene" refers to a human gene located on chromosome 4. In humans, the *HTT* gene is identified by NCBI as Gene ID No. 3064 and is located from base pair 3,074,510 to base pair 3,243,960 on chromosome 4.

As used herein, the term "*FMR1*" or "fragile X mental retardation 1 gene" refers to a human gene located on the X chromosome. In humans, the *FMR1* gene is identified by NCBI as Gene ID No. 2332 and is located from base pair 147,911,951 to base pair 147,951,127 on the X chromosome.

As used herein, the term "*DMPK*" or "dystrophia myotonica protein kinase gene" refers to a human gene located on chromosome 19. In humans, the *DMPK* gene is identified by NCBI as Gene ID No. 1760 and is located from base pair 46,272,975 to base pair 45,782,557 on chromosome 19.

As used herein, the term "*ZNF9*" refers to a human gene located on chromosome 3 which is also referred to as *CNBP* or the CCHC-type zinc finger nucleic acid binding protein. In humans, the *ZNF9* gene is identified by NCBI as Gene ID No. 7555 and is located from base pair 129,167,815 to base pair 129,183,967 on chromosome 3.

As used herein, the term "*C9ORF72*" refers to a human gene located on chromosome 9. In humans, the *C9ORF72* gene is identified by NCBI as Gene ID No. 203228 and is located from base pair 27,546,546 to base pair 27,573,886 on chromosome 9.

The terms "recombinant DNA construct," "recombinant construct," "expression cassette," "expression construct," "chimeric construct," "construct," and "recombinant DNA fragment" are used interchangeably herein and are nucleic acid fragments. A recombinant construct comprises an artificial combination of nucleic acid fragments, including, without limitation, regulatory and coding sequences that are not found together in nature. For example, a recombinant DNA construct may comprise regulatory sequences and coding sequences that are derived from different sources, or regulatory sequences and coding sequences derived from the same source and arranged in a manner different than that found in nature. Such a construct may be used by itself or may be used in conjunction with a vector.

As used herein, a "vector" or "recombinant DNA vector" may be a construct that includes a replication system and sequences that are capable of transcription and translation of a polypeptide-encoding sequence in a given host cell. If a vector is used then the choice of vector is dependent upon the method that will be used to transform host cells as is well known to those skilled in the art. Vectors can include, without limitation, plasmid vectors and recombinant AAV vectors, or any other vector known in that art suitable for delivering a gene encoding a meganuclease of the invention to a target cell. The skilled artisan is well aware of the genetic elements that must be present on the vector in order to successfully transform, select and propagate host cells comprising any of the isolated nucleotides or nucleic acid sequences of the invention.

As used herein, a "vector" can also refer to a viral vector. Viral vectors can include, without limitation, retroviral vectors, lentiviral vectors, adenoviral vectors, and adeno-associated viral vectors (AAV).

As used herein with respect to modifications of two proteins or amino acid sequences, the term "corresponding to" is used to indicate that a specified modification in the first protein is a substitution of the same amino acid residue as in the modification in the second protein, and that the amino acid position of the modification in the first proteins corresponds to or aligns with the amino acid position of the modification in the second protein when the two proteins are subjected to standard sequence alignments (e.g., using the BLASTp program). Thus, the modification of residue "X" to amino acid "A" in the first protein will correspond to the modification of residue "Y" to amino acid "A" in the second protein if residues X and Y correspond to each other in a sequence alignment, and despite the fact that X and Y may be different numbers.

As used herein, the terms "treatment" or "treating a subject" refers to the administration of an engineered nuclease of the invention, or a nucleic acid encoding an engineered nuclease of the invention, to a subject having a nucleotide repeat expansion disorder. Such treatment results in a reduction in the number of pathogenic nucleotide repeats in a number of cells sufficient to provide partial or complete relief of one or more symptoms of a disease resulting from the nucleotide repeat expansion. In some aspects, an engineered nuclease of the invention, or a nucleic acid encoding the same, is administered during treatment as a pharmaceutical composition.

As used herein, the recitation of a numerical range for a variable is intended to convey that the invention may be practiced with the variable equal to any of the values within that range. Thus, for a variable which is inherently discrete, the variable can be equal to any integer value within the numerical range, including the end-points of the range. Similarly, for a variable which is inherently continuous, the variable can be equal to any real value within the numerical range, including the end-points of the range. As an example, and without limitation, a variable which is described as having values between 0 and 2 can take the values 0, 1 or 2 if the variable is inherently discrete, and can take the values 0.0, 0.1, 0.01, 0.001, or any other real values ≧0 and ≦2 if the variable is inherently continuous.

As used herein, unless the context clearly indicates otherwise, the word "or" is used in the inclusive sense of "and/or" and not the exclusive sense of "either/or."

### 2.1 Principle of Nucleotide Repeat Expansion Deletion

The present invention is based, in part, on the hypothesis that nucleotide repeat expansion disorders can be corrected by permanently deleting the repeat region from the genome. The repeat region can be deleted by delivering a pair of engineered, site-specific endonucleases (or the genes encoding them) to the cells of a patient such that the nucleases cut DNA sites upstream and downstream of the repeat and liberate the intervening fragment from the genome. Surprisingly, if a pair of nucleases are selected that generate identical overhangs, the resulting genome sequence following deletion of the intervening fragment will frequently have a well-defined sequence resulting from direct-religation of the broken chromosome ends mediated by the compatible overhangs.

### 2.2 Nucleases for Deleting Exons

It is known in the art that it is possible to use a site-specific nuclease to make a DNA break in the genome of a living cell and that such a DNA break can result in permanent modification of the genome via mutagenic NHEJ repair or via HR with a transgenic DNA sequence. The present invention, however, involves co-expression of a pair of nucleases in the same cell. Surprisingly, we found that a pair of nucleases targeted to DNA sites in close proximity to one another (less than 10,000 basepairs apart) can excise the intervening DNA fragment from the genome.

Also surprisingly, we found that DNA excision using a pair of nucleases frequently proceeds via a mechanism involving the single-stranded DNA overhangs generated by the nucleases. In experiments involving a pair of meganucleases that generate complementary DNA overhangs, it was found that the overhang sequence was frequently conserved following fragment excision and repair of the resulting chromosome ends. The mechanism of DNA repair, in this case, appears to direct re-ligation of the broken ends, which has not been observed in mammalian cells. Such precise deletion and re-ligation was not observed when using a pair of meganucleases that generated non-identical overhangs (see Example 1). Thus, in a preferred embodiment, the pair of nucleases used for nucleotide repeat excision are selected to generate complementary overhangs.

To excise an exon efficiently, the pair of nuclease cut sites need to be relatively close together. In general, the closer the two sites are to one another, the more efficient the process will be. Thus, the preferred embodiment of the invention uses a pair of nucleases that cut sequences that are less than 10,000 basepairs or, more preferably, 5,000 basepairs or, still more preferably, less than 2,500 basepairs, or, most preferably, less than 1,500 basepairs apart.

As shown in Figure 2, a variety of different types of nucleases are useful for practicing the invention. Figures 2A and 2B show examples of how the invention can be practiced using a pair of CRISPR nucleases. In this case, the invention can be practiced by delivering three genes to the cell: one gene encoding the Cas9 protein and one gene encoding each of the two guide RNAs. CRISPRs cleave DNA to leave blunt ends which are not generally re-ligated cleanly. As a result, the final product will generally have additional insertion and/or deletion ("indel") mutations in the sequence. In an alternative embodiment, a "CRISPR Nickase" may be used, as reported in Ran et al. (2013), Cell 154:1380-9. To practice this embodiment, it is necessary to express four guide RNAs in the cell, two of which are complementary to the sequence upstream of the exon and two of which are complementary to the sequence downstream of the exon. In this embodiment, the two pairs of guide RNAs hybridize with complementary strands in the target region and each member of the pair produces a single strand DNA nick on one of the strands, equivalent to a double-strand break. Thus, the two pairs of guides result in two pairs of nicks. Moreover, the nicks in each pair can be off-set from one another to yield a single-strand overhang that is advantageous for practicing the invention. Methods for making CRISPRs and CRISPR Nickases that recognize pre-determined DNA sites are known in the art, for example Ran et al. (2013), Nat. Protoc. 8:2281-308.

In alternative embodiments, as diagrammed in Figure 2C and 2D, the nuclease pair can be compact TALENs. A compact TALEN comprises a TAL-effector DNA-binding domain (TALE) fused at its N- or C-terminus to the cleavage domain from I-TevI, comprising at least residues 1-96 and preferably residues 1-182 of I-TevI. The I-TevI cleavage domain recognizes and cuts DNA sequences of the form 5'-CNbNNtG-3', where "b" represents the site of cleavage of the bottom strand and "t" represents the site of cleavage of the top strand, and where "N" is any of the four bases. A compact TALEN, thus, cleaves to produce two basepair 3' overhangs. In a preferred embodiment, for deletions of sequences within an exon, the compact TALEN pair , is selected to create complementary overhangs that can directly re-ligate. Methods for making TALE domains that bind to pre-determined DNA sites are known in the art, for example Reyon et al. (2012), Nat. Biotechnol. 30:460-5. In another alternative embodiment, a pair of TALENs can be used for practicing the invention. A TALEN can comprise a DNA-binding domain comprising 16-22 TAL domain repeats fused to any portion of the FokI nuclease domain.

In yet another alternative embodiment, a pair of zinc finger nucleases can be used for practicing the invention. Zinc finger nucleases bind to a pre-determined DNA sequence ~18 basepairs in length and comprise a pair of 9-basepair half-sites separated by 2-10 basepairs. Cleavage by a zinc finger nuclease can create a blunt end or a 5' overhand of variable length (frequently four basepairs).

In yet another alternative embodiment, a pair of megaTALs can be used for practicing the invention. MegaTALs are single-chain endonucleases comprising a transcription activator-like effector (TALE) DNA binding domain with an engineered, sequence-specific homing endonuclease.

In the preferred embodiment, as diagrammed in Figure 2E, the nucleases used to practice the invention are a pair of single-chain meganucleases. A single-chain meganuclease comprises an N-terminal domain and a C-terminal domain joined by a linker peptide. Each of the two domains recognizes half of the recognition sequence and the site of DNA cleavage is at the middle of the recognition sequence near the interface of the two subunits. DNA strand breaks are offset by four basepairs such that DNA cleavage by a meganuclease generates a pair of four basepair, 3' single-strand overhangs. In a preferred embodiment, single-chain meganucleases are selected which cut recognition sequences with complementary overhangs. Example recognition sequences for Fragile X Syndrome, Huntington's Disease, Friedreich's Ataxia, Myotonic Dystrophy, and ALS are listed in Tables 2-11.

Table 2 lists a single, non-limiting example recognition sequence (SEQ ID NO: 2) for an engineered nuclease, particularly a meganuclease, that can be found in the upstream target region of the *FMR1* gene between the transcription start site and the CGG repeat responsible for Fragile X Syndrome (SEQ ID NO: 3). Table 3 lists a single, non-limiting example recognition sequence (SEQ ID NO: 4) for an engineered nuclease, particularly a meganuclease, that can be found in the downstream target region of the *FMR1* gene between the CGG repeat responsible for Fragile X Syndrome and the start of translation (SEQ ID NO: 5). Thus, co-expressing a pair of nucleases that recognize and cut sequences in the upstream (SEQ ID NO: 3) and downstream (SEQ ID NO: 5) *FMR1* target regions in a cell is expected to delete the CGG repeat region without disrupting the transcription or translation start sites.

Table 4 lists non-limiting examples of recognition sequences for engineered nucleases, particularly meganucleases, that can be found in the region of the human *HTT* gene between the translation start site and the CAG trinucleotide repeat associated with Huntington's Disease (SEQ ID NO: 7). Table 5 lists non-limiting examples of recognition sequences for engineered nucleases, particularly meganucleases, that can be found in the region of the human *HTT* gene downstream of the CAG trinucleotide repeat but upstream of the 3' end of Exon 1 (SEQ ID NO: 11). Thus, expressing a pair of nucleases that cut an upstream site in SEQ ID NO: 7 and a downstream site in SEQ ID NO: 11 will be expected to delete the trinucleotide repeat from the gene. Because the repeat is in a translated portion of the *HTT* gene, it is preferable to delete the repeat such that the reading frame is maintained following excision of the region intervening the two endonuclease sites. Specifically, the sequence of coding or exon DNA which is deleted should be a multiple of 3 basepairs in length.

Table 6 lists non-limiting examples of recognition sequences for engineered nucleases, particularly meganucleases, that can be found in the region of the human *FXN* gene Intron 1 upstream of the GAA trinucleotide repeat responsible for Friedreich's Ataxia (SEQ ID NO: 74). Table 7 lists non-limiting examples of recognition sequences for engineered nucleases, particularly meganucleases, that can be found in the region of the human *FXN* gene Intron 1 in the first 1000 basepairs downstream of the GAA trinucleotide repeat (SEQ ID NO: 96). This is the preferred region to target a nuclease downstream of the GAA repeat in the *FXN* gene, although the invention also embodies nucleases that recognize DNA sequences that are in Intron 1 but more than 1000 basepairs downstream of the repeat. Thus, expressing a pair of nucleases that cut an upstream site in SEQ ID NO: 74 and a downstream site in SEQ ID NO: 96 will be expected to delete the trinucleotide repeat from the gene. In preferred embodiments, the upstream and downstream recognition sequences are selected such that cleavage by the cognate nuclease pair will generate identical single-strand overhangs. For example, an engineered nuclease, particularly a meganuclease, can be selected that recognizes and cuts SEQ ID NO: 34 upstream of the *FXN* trinucleotide repeat and this nuclease can be paired with a second engineered nuclease, particularly a meganuclease, that recognizes and cuts SEQ ID NO: 89 downstream of the repeat. Because both engineered nucleases can generate 3' overhangs with the sequence 5'-GTGT-3', simultaneous cleavage by both nucleases in the same cell will generate compatible overhangs that can re-ligate with one another following removal of the intervening sequence. Similarly, an engineered nuclease, particularly a meganuclease, that recognizes SEQ ID NO: 63 can be advantageously paired with an engineered nuclease, particularly a meganuclease, that recognizes SEQ ID NO: 80 because both nucleases can generate compatible overhangs with the sequence 5'-ACAC-3'.

Table 8 lists non-limiting examples of recognition sequences for engineered nucleases, particularly meganucleases, that can be found in the region of the human *DMPK* gene 3' untranslated region (UTR) upstream of the CTG trinucleotide repeat associated with Myotonic Dystrophy (SEQ ID NO: 102). Table 9 lists non-limiting examples of recognition sequences for engineered nucleases, particularly meganucleases, that can be found in the region of the human *DMPK* gene 3' untranslated region (UTR) downstream of the CTG trinucleotide repeat as well as in the less-preferred region between the *DMPK* 3'UTR and the 5' UTR of the adjacent *SIX5* gene (SEQ ID NO: 132). Thus, expressing a pair of nucleases that cut an upstream site in SEQ ID NO: 102 and a downstream site in SEQ ID NO: 132 will be expected to delete the trinucleotide repeat from the gene. In a preferred embodiment, the upstream and downstream recognition sequences are selected such that cleavage by the cognate nuclease pair will generate identical single-strand overhangs. For example, an engineered nuclease, particularly a meganuclease, can be selected that recognizes and cuts SEQ ID NO: 98 upstream of the *BMPK* trinucleotide repeat and this nuclease can be paired with a second engineered nuclease, particularly a meganuclease, that recognizes and cuts SEQ ID NO: 123 downstream of the repeat. Because both engineered nucleases can generate 3' overhangs with the sequence 5'-GTGC-3', simultaneous cleavage by both nucleases in the same cell will generate compatible overhangs that can re-ligate with one another following removal of the intervening sequence.

Table 10 lists non-limiting examples of recognition sequences for engineered nucleases, particularly meganucleases, that can be found in the non-coding region of intron 1 in the human *C9ORF72* gene upstream of the GGGGCC hexanucleotide repeat associated with ALS and FTD. Table 11 lists non-limiting examples of recognition sequences for engineered nucleases, particularly meganucleases, that can be found in the non-coding region of intron 1 in the human *C9ORF72* gene downstream of the GGGGCC hexanucleotide repeat associated with ALS and FTD. Thus, expressing a pair of nucleases that cut an upstream site and a downstream site in intron 1 of the *C9ORF72* gene will be expected to delete the hexanucleotide repeat from the gene. In a preferred embodiment, the upstream and downstream recognition sequences are selected such that cleavage by the cognate nuclease pair will generate identical single-strand overhangs. For example, an engineered nuclease, particularly a meganuclease, can be selected that recognizes and cuts SEQ ID NO: 194 (*i.e.*, the ORF 7-8 recognition sequence) upstream of the GGGGCC hexanucleotide repeat, and this nuclease can be paired with a second engineered nuclease, particularly a meganuclease, that recognizes and cuts SEQ ID NO: 195 (*i.e.*, the ORF 9-10 recognition sequence) downstream of the repeat. Both engineered nucleases can generate 3' overhangs with the sequence 5'-GTGC-3', so simultaneous cleavage by both nucleases in the same cell will generate compatible overhangs that can re-ligate with one another following removal of the intervening sequence. In another example, an engineered nuclease, particularly a meganuclease, can be selected that recognizes and cuts SEQ ID NO: 197 (*i.e.*, the ORF 13-14 recognition sequence) upstream of the GGGGCC hexanucleotide repeat and this nuclease can be paired with a second engineered nuclease, particularly a meganuclease, that recognizes and cuts SEQ ID NO: 196 (*i.e.*, the ORF 11-12 recognition sequence) downstream of the repeat. Both engineered nucleases can generate 3' overhangs with the sequence 5'-GTGT-3' and, therefore, simultaneous cleavage by both nucleases in the same cell will generate compatible overhangs that can re-ligate with one another following removal of the intervening sequence.

**Table 2. Example Nuclease Recognition Sequence Upstream of the FMR1 CGG Repeat**

| Recognition Sequence | SEQ ID NO: | Overhang |
|---|---|---|
| GGGCCGACGGCGAGCGCGGGCG | 2 | GCGA |

**Table 3. Example Nuclease Recognition Sequence Downstream of the FMR1 CGG Repeat**

| Recognition Sequence | SEQ ID NO: | Overhang |
|---|---|---|
| GGCTGGGCCTCGAGCGCCCGCA | 4 | TCGA |

**Table 4. Example Nuclease Recognition Sequences Upstream of the HTT CAG Repeat**

| Recognition Sequence | SEQ ID NO: | Overhang |
|---|---|---|
| TGAAGGCCTTCGAGTCCCTCAA | 141 | TCGA |
| TCGAGTCCCTCAAGTCCTTCCA | 5 | TCAA |
| ACCGCCATGGCGACCCTGGAAA | 6 | GCGA |

**Table 5. Example Nuclease Recognition Sequences Downstream of the HTT CAG Repeat**

| Recognition Sequence | SEQ ID NO: | Overhang |
|---|---|---|
| GGAGCCGCTGCACCGACCGTGA | 8 | GCAC |
| GCACCGACCGTGAGTTTGGGCC | 9 | GTGA |
| CCGCCGCAGGCACAGCCGCTGC | 10 | GCAC |

**Table 6. Example Nuclease Recognition Sequences Upstream of FXN GAA Repeat**

| Recognition Sequence | SEQ ID NO: | Overhang |
|---|---|---|
| TGGGATTGGTTGCCAGTGCTTA | 12 | TTGC |
| TCGCTCCGGGTACGCGCGCTGG | 13 | GTAC |
| CGCAGAGCTGTGTGACCTTGGG | 14 | GTGT |
| TGGAACGAGGTGAAACTTTCAG | 15 | GTGA |
| CCGCGGGCCGCACGCCGCGGGC | 16 | GCAC |
| TGTCCTGCGGTGCGACTGCGGG | 17 | GTGC |
| TTAGGGGAGATGAAAGAGGCAG | 18 | ATGA |
| CTGACCCAGTTACGCCACGGCT | 19 | TTAC |
| GACCCAGTTACGCCACGGCTTG | 20 | ACGC |
| CCGCGGGCCGCACGCCGCACGC | 21 | GCAC |
| TGTTTGCGCGCACGGGCGCGCG | 22 | GCAC |
| GAAGCGGCCTTGCAACTCCCTT | 23 | TTGC |
| AGGCGCCGCGCACGCCGGGGTC | 24 | GCAC |
| GAAGGTGGATCACCTGAGGTCC | 25 | TCAC |
| TAATAGATGGTATCTGCTAGTA | 26 | GTAT |
| AGCGGCCTTGCAACTCCCTTCT | 27 | GCAA |
| TTCCGAGGGGTGTGCGGCTGTC | 28 | GTGT |
| GAGGGTGTTTCACGAGGAGGGA | 29 | TCAC |
| ATAATGTGTGTGTCTGTGTGTA | 30 | GTGT |
| TGTGTGTCTGTGTGTATCTGTA | 31 | GTGT |
| GCACGCCGCACGCCTGCGCAGG | 32 | ACGC |
| GTGTGTTGTGTGTGTGTGTTTG | 33 | GTGT |
| GCGGACCTGGTGTGAGGATTAA | 34 | GTGT |
| CACTTCTCTGCGATAACTTGTT | 35 | GCGA |
| GTGGCTGGTACGCCGCATGTAT | 36 | ACGC |
| TGCTAGTATATACATACACATA | 37 | ATAC |
| GCGGGCCGCACGCCGCACGCCT | 38 | ACGC |
| GCGCCGCGCACGCCGGGGTCGC | 39 | ACGC |
| GGCGCGCGCACACCTAATATTT | 40 | ACAC |
| TGTGTGTGTTTGCGCGCACGGG | 41 | TTGC |
| ACACATAATGTGTGTGTCTGTG | 42 | GTGT |
| CCACACGTGTTATTTGGCCCAC | 43 | TTAT |
| ATAAAGGTGACGCCCATTTTGC | 44 | ACGC |
| ATGAGGGTCTTGAAGATGCCAA | 45 | TTGA |
| TGCCAGTGCTTAAAAGTTAGGA | 46 | TTAA |
| GCGTGTGTGTTGTGTGTGTGTG | 47 | TTGT |
| GTGTGTGTTGTGTGTGTGTGTT | 48 | GTGT |
| CGCCACGGCTTGAAAGGAGGAA | 49 | TTGA |
| TAAATGGGAATAACATAGATAA | 50 | ATAA |
| AGGCAGGCCACGTCCAAGCCAT | 51 | ACGT |
| CGCGGTGGCTCATGCCCATAAT | 52 | TCAT |
| TGCGATAACTTGTTTCAGTAAT | 53 | TTGT |
| TATCTGCTAGTATATACATACA | 54 | GTAT |
| TATATACATACACATAATGTGT | 55 | ACAC |
| AAGGCACGGGCGAAGGCAGGGC | 56 | GCGA |
| CTGTATATAGCGTGTGTGTTGT | 57 | GCGT |
| GTGTTGTGTGTGTGTGTTTGCG | 58 | GTGT |
| ATAACATAGATAAAGTCTTCAG | 59 | ATAA |
| GAAGATTCCTCAAGGGGAGGAC | 60 | TCAA |
| GTACGCCGCATGTATTAGGGGA | 61 | ATGT |
| TGTCTGTGTGTATCTGTATATA | 62 | GTAT |
| GAACTTCCCACACGTGTTATTT | 63 | ACAC |
| TGTATCTGTATATAGCGTGTGT | 64 | ATAT |
| TGGATTTTTTTGAACGAAATGC | 65 | TTGA |
| ACTTCCCACACGTGTTATTTGG | 66 | ACGT |
| ACATGGTATTTAATGAGGGTCT | 67 | TTAA |
| CGGTGGCTCATGCCCATAATCT | 68 | ATGC |
| TCTCCATGCTTGTCACTTCTCT | 69 | TTGT |
| GCTGTCTCCATGCTTGTCACTT | 70 | ATGC |
| GGACCTGGTGTGAGGATTAAAT | 71 | GTGA |
| GGGTGTTTCACGAGGAGGGAAC | 72 | ACGA |
| CTAGTATATACATACACATAAT | 73 | ACAT |

**Table 7. Example Nuclease Recognition Sequences Downstream of FXN GAA Repeat**

| Recognition Sequence | SEQ ID NO: | Overhang |
|---|---|---|
| CGCGCGCCTGTAATCCCAGCTA | 75 | GTAA |
| CAACCTCAGGTGATCCGCCCAC | 76 | GTGA |
| GGCGTGGTGTCGCGCGCCTGTA | 77 | TCGC |
| TATACTGAATTAATCACATTTG | 78 | TTAA |
| GAGAATCGCTTGAGCCCGGGAG | 79 | TTGA |
| AGGAGGTGGACACTGGGTTTCT | 80 | ACAC |
| ATTACAGGCGTGAGCCACCGCG | 81 | GTGA |
| AGACATTTATTACTTGGCTTCT | 82 | TTAC |
| GAAAAATAGGCAAGTGTGGCCA | 83 | GCAA |
| TAAGGGCTATTGACTGACAAAC | 84 | TTGA |
| AGCTGCCACGTATTGGGCTTCC | 85 | GTAT |
| TTTTAGATGGTACCTGGTGGCT | 86 | GTAC |
| TTTGTTTTTTTGAGACAGAGTT | 87 | TTGA |
| TAGCTGGGATTATCGGCTAATT | 88 | TTAT |
| CCCCTGCCTGTGTGGACAGCAT | 89 | GTGT |
| GACTCCGTCTCAAAAAATAATA | 90 | TCAA |
| GAATTACAGGCGTGAGCCACCG | 91 | GCGT |
| TATTGACTGACAAACACACCCA | 92 | ACAA |
| GGAAAGCAGACATTTATTACTT | 93 | ACAT |
| GAGGTTGCATTAAGCCAAGATC | 94 | TTAA |
| AGCAGACATTTATTACTTGGCT | 95 | TTAT |

**Table 8. Example Nuclease Recognition Sequences Upstream of the DMPK CTG Repeat**

| Recognition Sequence | SEQ ID NO: | Overhang |
|---|---|---|
| TGCCAGTTCACAACCGCTCCGA | 97 | ACAA |
| GAAGACTGAGTGCCCGGGGCAC | 98 | GTGC |
| TCCAGTCCTGTGATCCGGGCCC | 99 | GTGA |
| CCGCTCCGAGCGTGGGTCTCCG | 100 | GCGT |
| GAACTGTCTTCGACTCCGGGGC | 101 | TCGA |

**Table 9. Example Nuclease Recognition Sequences Downstream of the DMPK CTG Repeat**

| Recognition Sequence | SEQ ID NO: | Overhang |
|---|---|---|
| TAGCGGGATGCGAAGCGGCCGA | 103 | GCGA |
| TGCGCCTGCGCACGCCACGCGC | 104 | GCAC |
| CTTTCTTGTGCATGACGCCCTG | 105 | GCAT |
| CTGGGGGGATCACAGACCATTT | 106 | TCAC |
| CTCTGGGGAGCGTCTGGCGCGA | 107 | GCGT |
| GGGAAGGCAGCAAGCCGGGCCG | 108 | GCAA |
| ACGCCACGCGCATCCGCTCCTG | 109 | GCAT |
| GGGCCGTCCGTGTTCCATCCTC | 110 | GTGT |
| GCTCCTGGGACGCAAGCTCGAG | 111 | ACGC |
| TCCGGAGGCGTGTGGAGGCGGC | 112 | GTGT |
| GGCCCAGCTGTGCCACCGAGCG | 113 | GTGC |
| CCCCACCTATCGTTGGTTCGCA | 114 | TCGT |
| ATCGTTGGTTCGCAAAGTGCAA | 115 | TCGC |
| GGGGCTTTGGCGTCCGGCCAAT | 116 | GCGT |
| CGGTATTTATTGTCTGTCCCCA | 117 | TTGT |
| CGCCTGCGCACGCCACGCGCAT | 118 | ACGC |
| CGGTTTGCGTTGTGGGCCGGAG | 119 | TTGT |
| AATCCGGAGGCGTGTGGAGGCG | 120 | GCGT |
| GAGGCCCTGACGTGGATGGGCA | 121 | ACGT |
| CCGACCCTCGCGAATAAAAGGC | 122 | GCGA |
| AGCTTTCTTGTGCATGACGCCC | 123 | GTGC |
| TCTGCCTGCTTACTCGGGAAAT | 124 | TTAC |
| TTTGGATATTTATTGACCTCGT | 125 | TTAT |
| TCCTCCGACTCGCTGACAGGCT | 126 | TCGC |
| AATTTGCTTTTGCCAAACCCGC | 127 | TTGC |
| AAAGCTTTCTTGTGCATGACGC | 128 | TTGT |
| GTTTTGGATGCACTGAGACCCC | 129 | GCAC |
| CCCCGACCCTCGCGAATAAAAG | 130 | TCGC |
| GCGGTTTGGATATTTATTGACC | 131 | ATAT |

**Table 10. Example Nuclease Recognition Sequences Upstream of the C9ORF72 GGGGCC Repeat**

| Recognition Sequence | SEQ ID NO: | Overhang |
|---|---|---|
| GAAAGAGAGGTGCGTCAAACAG | 194 | GTGC |
| GTTTAGGAGGTGTGTGTTTTTG | 197 | GTGT |

**Table 11. Example Nuclease Recognition Sequences Downstream of the C9ORF72 GGGGCC Repeat**

| Recognition Sequence | SEQ ID NO: | Overhang |
|---|---|---|
| GCGGTTGCGGTGCCTGCGCCCG | 195 | GTGC |
| CGAAGCTGGGTGTCGGGCTTTC | 196 | GTGT |

Recombinant meganucleases of the invention comprise a first subunit, comprising a first hypervariable (HVR1) region, and a second subunit, comprising a second hypervariable (HVR2) region. Further, the first subunit binds to a first recognition half-site in the recognition sequence (*e.g.*, a FXN1 half-site), and the second subunit binds to a second recognition half-site in the recognition sequence (*e.g.*, a FXN2 half-site). In embodiments where the recombinant meganuclease is a single-chain meganuclease, the first and second subunits can be oriented such that the first subunit, which comprises the HVR1 region and binds the first half-site, is positioned as the N-terminal subunit, and the second subunit, which comprises the HVR2 region and binds the second half-site, is positioned as the C-terminal subunit. In alternative embodiments, the first and second subunits can be oriented such that the first subunit, which comprises the HVR1 region and binds the first half-site, is positioned as the C-terminal subunit, and the second subunit, which comprises the HVR2 region and binds the second half-site, is positioned as the N-terminal subunit.

In particular embodiments, engineered meganucleases of the invention comprise an N-terminal nuclease-localization signal derived from SV40, a first meganuclease subunit, a linker sequence, and a second meganuclease subunit. Each meganuclease subunit binds a half-site of a recognition sequence and comprises a 56 base pair hypervariable (HVR) region, referred to as HVR1 and HVR2 for the first and second subunits, respectively. Each subunit is highly conserved outside of the HVR region with the exception of the amino acid corresponding to position 80 and/or position 271 of any one of SEQ ID NOs: 143-146, 155-159, 170-173, 182-185, 198-201, 210-213, 222-225, or 234-237, which can be occupied by Q or E to modulate the DNA-binding affinity of the engineered meganuclease.

In some particular examples, engineered nucleases, and particularly meganucleases, of the invention have been engineered to recognize and cleave the FXN 1-2 recognition sequence (SEQ ID NO: 29). Such recombinant nucleases are collectively referred to herein as "FXN 1-2 nucleases" or "FXN 1-2 meganucleases," as appropriate. Exemplary FXN 1-2 meganucleases are provided in SEQ ID NOs: 143-146.

In additional examples, engineered nucleases, and particularly meganucleases, of the invention have been engineered to recognize and cleave the FXN 3-4 recognition sequence (SEQ ID NO: 34). Such recombinant nucleases are collectively referred to herein as "FXN 3-4 nucleases" or "FXN 3-4 meganucleases," as appropriate. Exemplary FXN 3-4 meganucleases are provided in SEQ ID NOs: 155-159.

In additional examples, engineered nucleases, and particularly meganucleases, of the invention have been engineered to recognize and cleave the FXN 5-6 recognition sequence (SEQ ID NO: 89). Such recombinant nucleases are collectively referred to herein as "FXN 5-6 nucleases" or "FXN 5-6 meganucleases," as appropriate. Exemplary FXN 5-6 meganucleases are provided in SEQ ID NOs: 170-173.

In additional examples, engineered nucleases, and particularly meganucleases, of the invention have been engineered to recognize and cleave the FXN 11-12 recognition sequence (SEQ ID NO: 63). Such recombinant nucleases are collectively referred to herein as "FXN 11-12 nucleases" or "FXN 11-12 meganucleases," as appropriate. Exemplary FXN 11-12 meganucleases are provided in SEQ ID NOs: 182-185.

In additional examples, engineered nucleases, and particularly meganucleases, of the invention have been engineered to recognize and cleave the ORF 7-8 recognition sequence (SEQ ID NO: 194). Such recombinant nucleases are collectively referred to herein as "ORF 7-8 nucleases" or "ORF 7-8 meganucleases," as appropriate. Exemplary ORF 7-8 meganucleases are provided in SEQ ID NOs: 198-201.

In additional examples, engineered nucleases, and particularly meganucleases, of the invention have been engineered to recognize and cleave the ORF 9-10 recognition sequence (SEQ ID NO: 195). Such recombinant nucleases are collectively referred to herein as "ORF 9-10 nucleases" or "ORF 9-10 meganucleases," as appropriate Exemplary ORF 9-10 meganucleases are provided in SEQ ID NOs: 210-213.

In additional examples, engineered nucleases, and particularly meganucleases, of the invention have been engineered to recognize and cleave the ORF 11-12 recognition sequence (SEQ ID NO: 196). Such recombinant nucleases are collectively referred to herein as "ORF 11-12 nucleases" or "ORF 11-12 meganucleases," as appropriate. Exemplary ORF 11-12 meganucleases are provided in SEQ ID NOs: 222-225.

In additional examples, engineered nucleases, and particularly meganucleases, of the invention have been engineered to recognize and cleave the ORF 13-14 recognition sequence (SEQ ID NO: 197). Such recombinant nucleases are collectively referred to herein as "ORF 13-14 nucleases" or "ORF 13-14 meganucleases," as appropriate. Exemplary ORF 13-14 meganucleases are provided in SEQ ID NOs: 234-237.

Exemplary FXN 1-2, FXN 3-4, FXN 5-6, and FXN 11-12 meganucleases of the invention are provided in Tables 12-15, respectively. Exemplary ORF 7-8, ORF 9-10, ORF 11-12, and ORF 13-14 meganucleases of the invention are provided in Tables 16-19, respectively.

**Table 12. Exemplary recombinant meganucleases engineered to recognize and cleave the FXN 1-2 recognition sequence (SEQ ID NO: 29)**

| **Meganuclease** | **AA SEQ ID** | **FXN1 Subunit Residues** | **FXN1 Subunit SEQ ID** | ***FXN1 Subunit %** | **FXN2 Subunit Residues** | **FXN2 Subunit SEQ ID** | ***FXN2 Subunit %** |
|---|---|---|---|---|---|---|---|
| FXN 1-2x.63 | 143 | 7-153 | 147 | 100 | 198-344 | 151 | 100 |
| FXN 1-2x.11 | 144 | 7-153 | 148 | 93.2 | 198-344 | 152 | 93.88 |
| FXN 1-2x.73 | 145 | 7-153 | 149 | 90.48 | 198-344 | 153 | 93.2 |
| FXN 1-2x.84 | 146 | 7-153 | 150 | 93.88 | 198-344 | 154 | 93.88 |

| | | | | | | | |
|---|---|---|---|---|---|---|---|
| *"FXN1 Subunit %" and "FXN2 Subunit %" represent the amino acid sequence identity (>90%) between the FXN1-binding and FXN2-binding subunit regions of each meganuclease and the FXN1-binding and FXN2-binding subunit regions, respectively, of the FXN 1-2x.63 meganuclease. | | | | | | | |

**Table 13. Exemplary recombinant meganucleases engineered to recognize and cleave the FXN 3-4 recognition sequence (SEQ ID NO: 34)**

| **Meganuclease** | **AA SEQ ID** | **FXN3 Subunit Residues** | **FXN3 Subunit SEQ ID** | ***FXN3 Subunit %** | **FXN4 Subunit Residues** | **FXN4 Subunit SEQ ID** | ***FXN4 Subunit %** |
|---|---|---|---|---|---|---|---|
| FXN 3-4L.34 | 155 | 7-153 | 160 | 100 | 198-344 | 165 | 100 |
| FXN 3-4L.5 | 156 | 7-153 | 161 | 98.64 | 198-344 | 166 | 100 |
| FXN 3-4L.12 | 157 | 7-153 | 162 | 97.96 | 198-344 | 167 | 100 |
| FXN 3-4x.312 | 158 | 7-153 | 163 | 97.96 | 198-344 | 168 | 95.24 |
| FXN 3-4x.383 | 159 | 7-153 | 164 | 90.48 | 198-344 | 169 | 93.2 |

| | | | | | | | |
|---|---|---|---|---|---|---|---|
| *"FXN3 Subunit %" and "FXN4 Subunit %" represent the amino acid sequence identity (>90%) between the FXN3-binding and FXN4-binding subunit regions of each meganuclease and the FXN3-binding and FXN4-binding subunit regions, respectively, of the FXN 3-4L.34 meganuclease. | | | | | | | |

**Table 14. Exemplary recombinant meganucleases engineered to recognize and cleave the FXN 5-6 recognition sequence (SEQ ID NO: 89)**

| **Meganuclease** | **AA SEQ ID** | **FXN5 Subunit Residues** | **FXN5 Subunit SEQ ID** | ***FXN5 Subunit %** | **FXN6 Subunit Residues** | **FXN6 Subunit SEQ ID** | ***FXN6 Subunit %** |
|---|---|---|---|---|---|---|---|
| FXN 5-6L.45 | 170 | 198-344 | 174 | 100 | 7-153 | 178 | 100 |
| FXN 5-6L.38 | 171 | 198-344 | 175 | 100 | 7-153 | 179 | 97.28 |
| FXN 5-6x.24 | 172 | 198-344 | 176 | 100 | 7-153 | 180 | 97.28 |
| FXN 5-6x.20 | 173 | 7-153 | 177 | 94.56 | 198-344 | 181 | 92.52 |

| | | | | | | | |
|---|---|---|---|---|---|---|---|
| *"FXN5 Subunit %" and "FXN6 Subunit %" represent the amino acid sequence identity (>90%) between the FXN5-binding and FXN6-binding subunit regions of each meganuclease and the FXN5-binding and FXN6-binding subunit regions, respectively, of the FXN 5-6L.45 meganuclease. | | | | | | | |

**Table 15. Exemplary recombinant meganucleases engineered to recognize and cleave the FXN 11-12 recognition sequence (SEQ ID NO: 63)**

| **Meganuclease** | **AA SEQ ID** | **FXN11 Subunit Residues** | **FXN11 Subunit SEQ ID** | ***FXN11 Subunit %** | **FXN12 Subunit Residues** | **FXN12 Subunit SEQ ID** | ***FXN12 Subunit %** |
|---|---|---|---|---|---|---|---|
| FXN 11-12x.63 | 182 | 198-344 | 186 | 100 | 7-153 | 190 | 100 |
| FXN 11-12x.99 | 183 | 198-344 | 187 | 91.16 | 7-153 | 191 | 92.52 |
| FXN 11-12x.107 | 184 | 198-344 | 188 | 91.16 | 7-153 | 192 | 92.52 |
| FXN 11-12x.139 | 185 | 198-344 | 189 | 90.48 | 7-153 | 193 | 91.84 |

| | | | | | | | |
|---|---|---|---|---|---|---|---|
| *"FXN11 Subunit %" and "FXN12 Subunit %" represent the amino acid sequence identity (>90%) between the FXN11-binding and FXN12-binding subunit regions of each meganuclease and the FXN11-binding and FXN12-binding subunit regions, respectively, of the FXN 11-12x.63 meganuclease. | | | | | | | |

**Table 16. Exemplary recombinant meganucleases engineered to recognize and cleave the ORF 7-8 recognition sequence (SEQ ID NO: 194)**

| **Meganuclease** | **AA SEQ ID** | **ORF7 Subunit Residues** | **ORF7 Subunit SEQ ID** | ***ORF7 Subunit %** | **ORF8 Subunit Residues** | **ORF8 Subunit SEQ ID** | ***ORF8 Subunit %** |
|---|---|---|---|---|---|---|---|
| ORF 7-8x.4 | 198 | 7-153 | 202 | 100 | 198-344 | 206 | 100 |
| ORF 7-8x.31 | 199 | 7-153 | 203 | 98.64 | 198-344 | 207 | 99.32 |
| ORF 7-8x.45 | 200 | 7-153 | 204 | 99.32 | 198-344 | 208 | 98.64 |
| ORF 7-8x.29 | 201 | 198-344 | 205 | 91.84 | 7-153 | 209 | 92.52 |

| | | | | | | | |
|---|---|---|---|---|---|---|---|
| *"ORF7 Subunit %" and "ORF8 Subunit %" represent the amino acid sequence identity (>90%) between the ORF7-binding and ORF8-binding subunit regions of each meganuclease and the ORF7-binding and ORF8-binding subunit regions, respectively, of the ORF 7-8x.4 meganuclease. | | | | | | | |

**Table 17. Exemplary recombinant meganucleases engineered to recognize and cleave the ORF 9-10 recognition sequence (SEQ ID NO: 195)**

| **Meganuclease** | **AA SEQ ID** | **ORF9 Subunit Residues** | **ORF9 Subunit SEQ ID** | ***ORF9 Subunit %** | **ORF10 Subunit Residues** | **ORF10 Subunit SEQ ID** | ***ORF10 Subunit %** |
|---|---|---|---|---|---|---|---|
| ORF 9-10x.15 | 210 | 7-153 | 214 | 100 | 198-344 | 218 | 100 |
| ORF 9-10x.14 | 211 | 7-153 | 215 | 90.48 | 198-344 | 219 | 93.88 |
| ORF 9-10x.61 | 212 | 7-153 | 216 | 93.88 | 198-344 | 220 | 100 |
| ORF 9-10x.77 | 213 | 7-153 | 217 | 90.48 | 198-344 | 221 | 92.52 |

| | | | | | | | |
|---|---|---|---|---|---|---|---|
| *"ORF9 Subunit %" and "ORF10 Subunit %" represent the amino acid sequence identity (>90%) between the ORF9-binding and ORF10-binding subunit regions of each meganuclease and the ORF9-binding and ORF10-binding subunit regions, respectively, of the ORF 9-10x.15 meganuclease. | | | | | | | |

**Table 18. Exemplary recombinant meganucleases engineered to recognize and cleave the ORF 11-12 recognition sequence (SEQ ID NO: 196)**

| **Meganuclease** | **AA SEQ ID** | **ORF11 Subunit Residues** | **ORF11 Subunit SEQ ID** | ***ORF11 Subunit %** | **ORF12 Subunit Residues** | **ORF12 Subunit SEQ ID** | ***ORF12 Subunit %** |
|---|---|---|---|---|---|---|---|
| ORF 11-12x.5 | 222 | 198-344 | 226 | 100 | 7-153 | 230 | 100 |
| ORF 11-12L.1 | 223 | 198-344 | 227 | 97.28 | 7-153 | 231 | 100 |
| ORF 11-12L.8 | 224 | 198-344 | 228 | 97.28 | 7-153 | 232 | 100 |
| ORF 11-12L.64 | 225 | 198-344 | 229 | 97.28 | 7-153 | 233 | 100 |

| | | | | | | | |
|---|---|---|---|---|---|---|---|
| *"ORF11 Subunit %" and "ORF12 Subunit %" represent the amino acid sequence identity (>90%) between the ORF11-binding and ORF12-binding subunit regions of each meganuclease and the ORF11-binding and ORF12-binding subunit regions, respectively, of the ORF 11-12x.5 meganuclease. | | | | | | | |

**Table 19. Exemplary recombinant meganucleases engineered to recognize and cleave the ORF 13-14 recognition sequence (SEQ ID NO: 197)**

| **Meganuclease** | **AA SEQ ID** | **ORF13 Subunit Residues** | **ORF13 Subunit SEQ ID** | ***ORF13 Subunit %** | **ORF14 Subunit Residues** | **ORF14 Subunit SEQ ID** | ***ORF14 Subunit %** |
|---|---|---|---|---|---|---|---|
| ORF 13-14x.40 | 234 | 7-153 | 238 | 100 | 198-344 | 242 | 100 |
| ORF 13-14x.77 | 235 | 7-153 | 239 | 99.32 | 198-344 | 243 | 94.56 |
| ORF 13-14x.90 | 236 | 7-153 | 240 | 99.32 | 198-344 | 244 | 91.84 |
| ORF 13-14x.3 | 237 | 7-153 | 241 | 100 | 198-344 | 245 | 94.56 |

| | | | | | | | |
|---|---|---|---|---|---|---|---|
| *"ORF13 Subunit %" and "ORF14 Subunit %" represent the amino acid sequence identity (>90%) between the ORF13-binding and ORF14-binding subunit regions of each meganuclease and the ORF13-binding and ORF14-binding subunit regions, respectively, of the ORF 13-14x.40 meganuclease. | | | | | | | |

### 2.3 Methods for Delivering and Expressing Nucleases

Treating nucleotide repeat expansion disorders in accordance with the invention requires that a pair of nucleases be expressed simultaneously in cells in the appropriate tissues. The target tissue(s) for delivery of nucleases of the invention will vary depending on the disorder being treated. For example, treating Huntington's disease, Friedreich's Ataxia, or ALS requires delivery to CNS whereas treating Myotonic Dystrophy requires delivery to muscle. Table 20 lists the target tissues for each of the common nucleotide repeat expansion disorders.

The nucleases can be delivered to target cells in a subject during treatment as purified protein or as RNA or DNA encoding the nucleases. Preferably, engineered nucleases of the invention are delivered as a nucleic acid encoding the engineered nuclease. Such nucleic acids can be DNA (*e.g.,* circular or linearized plasmid DNA or PCR products) or RNA (*e.g.*, mRNA). In some embodiments, mRNA encoding an engineered nuclease is delivered to a cell because this reduces the likelihood that the gene encoding the engineered nuclease will integrate into the genome of the cell. Such mRNA encoding an engineered nuclease can be produced using methods known in the art such as *in vitro* transcription. In some embodiments, the mRNA is capped using 7-methyl-guanosine. In some embodiments, the mRNA may be polyadenylated.

In some particular embodiments, a nucleic acid encoding an endonuclease of the invention can be introduced into a cell using a single-stranded DNA template. In some such embodiments, the single-stranded DNA can further comprise a 5' and/or a 3' AAV inverted terminal repeat (ITR) sequence flanking the DNA encoding the nuclease sequence. In other embodiments, the single-stranded DNA can further comprise a 5' homology arm and/or a 3' homology arm flaking the DNA encoding the nuclease sequence.

Purified nuclease proteins can be delivered into cells to cleave genomic DNA by a variety of different mechanisms known in the art, including those further detailed herein below.

In one embodiment, the nuclease proteins or mRNA or vector encoding the nucleases are supplied to target cells via injection directly to the target tissue. Muscle disorders, for example, can be treated by intramuscular injection (Maltzahn et al. (2012), Proc. Natl. Acad. Sci. U S A. 109:20614-9) or hydrodynamic injection (Taniyama et al. (2012), Curr. Top. Med. Chem. 12:1630-7; Hegge et al. (2010), Hum. Gene Ther. 21:829-42). CNS disorders can be treated, for example, by intracranial injection of the proteins or nucleic acids. Alternatively, nuclease protein, mRNA, or DNA can be delivered systemically via the circulatory system.

In some embodiments, engineered nuclease proteins, or DNA/mRNA encoding engineered nucleases, are formulated for systemic administration, or administration to target tissues, in a pharmaceutically acceptable carrier in accordance with known techniques. See, *e.g.*, Remington, The Science and Practice of Pharmacy 21st Edition (Philadelphia: Lippincott Williams & Wilkins, 2005). In the manufacture of a pharmaceutical formulation according to the invention, proteins/RNA/mRNA are typically admixed with a pharmaceutically acceptable carrier. The carrier must, of course, be acceptable in the sense of being compatible with any other ingredients in the formulation and must not be deleterious to the patient. The carrier can be a solid or a liquid, or both, and can be formulated with the compound as a unit-dose formulation.

To facilitate cellular uptake, the proteins or nucleic acid(s) can be coupled to a cell penetrating peptide to facilitate uptake by cells. Examples of cell penetrating peptides known in the art include poly-arginine (Jearawiriyapaisarn et al. (2008), Mol. Ther. 16:1624-9), TAT peptide from the HIV virus (Hudecz et al. (2005), Med. Res. Rev. 25: 679-736), MPG (Simeoni et al. (2003), Nucleic Acids Res. 31:2717-2724), Pep-1 (Deshayes et al. (2004), Biochemistry 43: 7698-7706, and HSV-1 VP-22 (Deshayes et al. (2005) Cell Mol Life Sci. 62:1839-49. Alternatively, cell penetration can be facilitated by liposome encapsulation (see, *e.g.*, Lipofectamine^{™}, Life Technologies Corp., Carlsbad, CA). The liposome formulation can be used to facilitate lipid bilayer fusion with a target cell, thereby allowing the contents of the liposome or proteins associated with its surface to be brought into the cell. In an alternative embodiment, the nucleases or DNA/mRNA encoding the nucleases are coupled covalently or non-covalently to an antibody that recognizes a specific cell-surface receptor expressed on target cells such that the nuclease protein/DNA/mRNA binds to and is internalized by the target cells. Alternatively, the nuclease protein/DNA/mRNA can be coupled covalently or non-covalently to the natural ligand (or a portion of the natural ligand) for such a cell-surface receptor. (McCall et al. (2014), Tissue Barriers 2(4):e944449; Dinda et al. (2013), Curr. Pharm. Biotechnol. 14:1264-74; Kang et al. (2014), Curr. Pharm. Biotechnol. 15(3):220-30; Qian et al. (2014), Expert Opin. Drug Metab. Toxicol. 10(11):1491-508).

**Table 20. Target Tissues for Common Nucleotide Repeat Expansion Disorders**

| Disorder | Target Tissue |
|---|---|
| DRPLA (Dentatorubropallidoluysian atrophy) | CNS |
| HD (Huntington's disease) | CNS |
| SBMA (Spinal and bulbar muscular atrophy) | CNS |
| SCA1 (Spinocerebellar ataxia Type 1) | CNS |
| SCA2 (Spinocerebellar ataxia Type 2) | CNS |
| SCA3 (Spinocerebellar ataxia Type 3 or Machado-Joseph disease) | CNS |
| SCA6 (Spinocerebellar ataxia Type 6) | CNS |
| SCA7 (Spinocerebellar ataxia Type 7) | CNS |
| SCA17 (Spinocerebellar ataxia Type 17) | CNS |
| FRAXA (Fragile X syndrome) | CNS |
| FXTAS (Fragile X-associated tremor/ataxia syndrome) | CNS |
| FRAXE (Fragile XE mental retardation) | CNS |
| FRDA (Friedreich's ataxia) | CNS |
| DM (Myotonic dystrophy) | Muscle |
| SCA8 (Spinocerebellar ataxia Type 8) | CNS |
| SCA12 (Spinocerebellar ataxia Type 12) | CNS |
| ALS (Amyotrophic lateral sclerosis) or FTD (Frontotemporal dementia) | CNS |

In some embodiments, endonuclease proteins, or DNA/mRNA encoding endonucleases, are encapsulated within biodegradable hydrogels for injection or implantation within a desired organ (*e.g.*, the region of the liver in proximity to hepatic sinusoidal endothelial cells, or progenitor cells which differentiate into hepatic sinusoidal endothelial cells). Hydrogels also can provide sustained and tunable release of the therapeutic payload to the desired region of an organ without the need for frequent injections, and stimuli-responsive materials (*e.g.*, temperature- and pH-responsive hydrogels) can be designed to release the payload in response to environmental or externally applied cues (see, *e.g.*, Kang Derwent et al. (2008), Trans. Am. Ophthalmol. Soc. 106:206-214).

In some embodiments, the nuclease proteins or DNA/mRNA encoding the nucleases are coupled covalently or, preferably, non-covalently to a nanoparticle. A nanoparticle is any solid support, such as metal, polymer, or biological macromolecule, to which multiple copies of the nuclease proteins, mRNA, or DNA can be attached. This increases the copy number of the protein/mRNA/DNA that is delivered to each cell and, thereby, increases the intracellular expression of each nuclease to maximize the likelihood that the recognition sequences will be cut. Nanoparticles may additionally be advantageously coupled to targeting molecules to direct the nanoparticles to the appropriate cell type and/or increase the likelihood of cellular uptake. Examples of such targeting molecules include antibodies specific for cell-surface receptors and the natural ligands (or portions of the natural ligands) for cell surface receptors.

In some embodiments, nuclease proteins or DNA/mRNA encoding the nucleases, are encapsulated within liposomes or complexed using cationic lipids (see, *e.g.*, Lipofectamine^{™}, Life Technologies Corp., Carlsbad, CA; Zuris et al. (2015), Nat. Biotechnol. 33: 73-80; Mishra et al. (2011), J. Drug Deliv. 2011:863734). The liposome and lipoplex formulations can protect the payload from degradation, enhance accumulation and retention at the target site, and facilitate cellular uptake and delivery efficiency through fusion with and/or disruption of the cellular membranes of the target cells.

In some embodiments, nuclease proteins, or DNA/mRNA encoding nucleases, are encapsulated within polymeric scaffolds (*e.g.*, PLGA) or complexed using cationic polymers (*e.g.*, PEI, PLL) (Tamboli et al. (2011), Ther. Deliv. 2(4): 523-536). Polymeric carriers can be designed to provide tunable drug release rates through control of polymer erosion and drug diffusion, and high drug encapsulation efficiencies can offer protection of the therapeutic payload until intracellular delivery to the desired target cell population.

In some embodiments, nuclease proteins, or DNA/mRNA encoding nucleases, are combined with amphiphilic molecules that self-assemble into micelles (Tong et al. (2007), J. Gene Med. 9(11): 956-66). Polymeric micelles may include a micellar shell formed with a hydrophilic polymer (*e.g.*, polyethylene glycol) that can prevent aggregation, mask charge interactions, and reduce nonspecific interactions within the vitreous fluid.

In some embodiments, nuclease proteins, or DNA/mRNA encoding nucleases, are formulated into an emulsion or a nanoemulsion (*i.e.*, having an average particle diameter of < 1nm) for administration and/or delivery to the target cell. The term "emulsion" refers to, without limitation, any oil-in-water, water-in-oil, water-in-oil-in-water, or oil-in-water-in-oil dispersions or droplets, including lipid structures that can form as a result of hydrophobic forces that drive apolar residues (*e.g.*, long hydrocarbon chains) away from water and polar head groups toward water, when a water immiscible phase is mixed with an aqueous phase. These other lipid structures include, but are not limited to, unilamellar, paucilamellar, and multilamellar lipid vesicles, micelles, and lamellar phases. Emulsions are composed of an aqueous phase and a lipophilic phase (typically containing an oil and an organic solvent). Emulsions also frequently contain one or more surfactants. Nanoemulsion formulations are well known, *e.g.*, as described in US Patent Application Nos. 2002/0045667 and 2004/0043041, and US Pat. Nos. 6,015,832, 6,506,803, 6,635,676, and 6,559,189, each of which is incorporated herein by reference in its entirety.

In some embodiments, nuclease proteins, or DNA/mRNA encoding nucleases, are covalently attached to, or non-covalently associated with, multifunctional polymer conjugates, DNA dendrimers, and polymeric dendrimers (Mastorakos et al. (2015), Nanoscale. 7(9): 3845-56; Cheng et al. (2008), J. Pharm. Sci. 97(1): 123-43). The dendrimer generation can control the payload capacity and size, and can provide a high drug payload capacity. Moreover, display of multiple surface groups can be leveraged to improve stability, reduce nonspecific interactions, and enhance cell-specific targeting and drug release.

In some embodiments, the genes encoding a pair of nucleases are delivered using a viral vector, or a pair of viral vectors. Such vectors are known in the art and include retroviral vectors, lentiviral vectors, adenoviral vectors, and adeno-associated virus (AAV) vectors (reviewed in Vannucci et al. (2013), New Microbiol. 36:1-22). In some embodiments, the viral vectors are injected directly into target tissues (Bosch et al. (2000), Mol. Ther. 1:63-70; Greig et al. (2014), PLoS One. 9(11):e112268). In alternative embodiments, the viral vectors are delivered systemically via the circulatory system. It is known in the art that different AAV vectors tend to localize to different tissues. Muscle-tropic AAV serotypes include AAV1, AAV9, and AAV2.5 (Bowles et al. (2012), Mol. Ther. 20:443-55). Thus, these serotypes are preferred for the delivery of nucleases to muscle tissue. The AAV serotypes most commonly used for CNS applications include AAV1, AAV2, AAV4, AAV5, AAV6, AAV8, and AAV9 (McCown (2005), Curr. Gene Ther. 5:333-8; Lentz et al. (2012), Neurobiol. Dis. 48:179-88).

In one embodiment, a vector used for endonuclease gene delivery is a self-limiting vector. A self-limiting vector can have limited persistence time in a cell or organism due to the presence of a recognition sequence for a nuclease within the vector that leads to destruction of the vector. Thus, a self-limiting vector can be engineered to provide coding sequences for a promoter, a nuclease as described herein, and a nuclease recognition site within the self-limiting vector itself. The self-limiting vector delivers the nuclease gene to a cell, tissue, or organism, such that the nuclease is expressed and able to cut the genome of the cell at an endogenous recognition sequence within the genome. The delivered nuclease will also find its target site within the self-limiting vector itself, and cut the vector at this target site. Once cut, the 5' and 3' ends of the vector will be exposed and degraded by exonucleases, thus destroying the vector and ceasing production of the endonuclease.

If the nuclease genes are delivered in DNA form (*e.g.,* plasmid) and/or via a viral vector (*e.g.* AAV) they must be operably linked to a promoter. In some embodiments, this can be a viral promoter such as endogenous promoters from the viral vector (*e.g.,* the LTR of a lentiviral vector) or the well-known cytomegalovirus- or SV40 virus-early promoters. In a preferred embodiment, the nuclease genes are operably linked to a promoter that drives gene expression preferentially in the target cells. Examples of muscle-specific promoters include C5-12 (Liu et al. (2004), Hum. Gene Ther. 15:783-92), the muscle-specific creatine kinase (MCK) promoter (Yuasa et al. (2002), Gene Ther. 9:1576-88), or the smooth muscle 22 (SM22) promoter (Haase et al. (2013), BMC Biotechnol. 13:49-54). Examples of CNS (neuron)-specific promoters include the NSE, Synapsin, and MeCP2 promoters (Lentz et al. (2012), Neurobiol. Dis. 48:179-88). In some embodiments, the nuclease genes are under the control of two separate promoters. In alternative embodiments, the genes are under the control of a single promoter and are separated by an internal-ribosome entry site (IRES) or a 2A peptide sequence (Szymczak and Vignali (2005), Expert Opin. Biol. Ther. 5:627-38).

It is envisioned that a single treatment will permanently delete nucleotide repeats from a percentage of patient cells. If the frequency of nucleotide repeat deletion is low, however, or if a large percentage of target cells need to be corrected, it may be necessary to perform multiple treatments on each patient.

### 2.4 Pharmaceutical Compositions

In some embodiments, the invention provides a pharmaceutical composition comprising a pharmaceutically acceptable carrier and engineered nuclease of the invention, or a pharmaceutically acceptable carrier and a nucleic acid encoding an engineered nuclease of the invention. Pharmaceutical compositions of the invention can be useful for treating a subject having a nucleotide repeat expansion disorder.

Such pharmaceutical compositions can be prepared in accordance with known techniques. See, *e.g.*, Remington, The Science and Practice of Pharmacy 21st Edition (Philadelphia: Lippincott Williams & Wilkins, 2005). In the manufacture of a pharmaceutical formulation according to the invention, engineered nuclease polypeptides (or DNA/RNA encoding the same) are typically admixed with a pharmaceutically acceptable carrier and the resulting composition is administered to a subject. The carrier must, of course, be acceptable in the sense of being compatible with any other ingredients in the formulation and must not be deleterious to the subject. In some embodiments, pharmaceutical compositions of the invention can further comprise one or more additional agents or biological molecules useful in the treatment of a disease in the subject. Likewise, the additional agent(s) and/or biological molecule(s) can be co-administered as a separate composition.

### 2.5 Methods for Producing Recombinant AAV Vectors

In some embodiments, the invention provides recombinant AAV vectors for use in the methods of the invention. Recombinant AAV vectors are typically produced in mammalian cell lines such as HEK-293. Because the viral *cap* and *rep* genes are removed from the vector to prevent its self-replication and to make room for the therapeutic gene(s) to be delivered (*e.g.,* the endonuclease gene), it is necessary to provide these *in trans* in the packaging cell line. In addition, it is necessary to provide the "helper" (*e.g.,* adenoviral) components necessary to support replication (Cots et al. (2013), Curr. Gene Ther. 13(5): 370-81). Frequently, recombinant AAV vectors are produced using a triple-transfection in which a cell line is transfected with a first plasmid encoding the "helper" components, a second plasmid comprising the *cap* and *rep* genes, and a third plasmid comprising the viral ITRs containing the intervening DNA sequence to be packaged into the virus. Viral particles comprising a genome (ITRs and intervening gene(s) of interest) encased in a capsid are then isolated from cells by freeze-thaw cycles, sonication, detergent, or other means known in the art. Particles are then purified using cesium-chloride density gradient centrifugation or affinity chromatography and subsequently delivered to the gene(s) of interest to cells, tissues, or an organism such as a human patient.

Because recombinant AAV particles are typically produced (manufactured) in cells, precautions must be taken in practicing the current invention to ensure that the site-specific nuclease is not expressed in the packaging cells. Because the viral genomes of the invention comprise a recognition sequence for the nuclease, any endonuclease expressed in the packaging cell line will be capable of cleaving the viral genome before it can be packaged into viral particles. This will result in reduced packaging efficiency and/or the packaging of fragmented genomes. Several approaches can be used to prevent nuclease expression in the packaging cells, including:
1. The nuclease can be placed under the control of a tissue-specific promoter that is not active in the packaging cells. For example, if a viral vector is developed for delivery of (a) nuclease gene(s) to muscle tissue, a muscle-specific promoter can be used. Examples of muscle-specific promoters include C5-12 (Liu et al. (2004), Hum. Gene Ther. 15:783-92), the muscle-specific creatine kinase (MCK) promoter (Yuasa et al. (2002), Gene Ther. 9:1576-88), or the smooth muscle 22 (SM22) promoter (Haase et al. (2013), BMC Biotechnol. 13:49-54). Examples of CNS (neuron)-specific promoters include the NSE, Synapsin, and MeCP2 promoters (Lentz et al. (2012), Neurobiol. Dis. 48:179-88). Examples of liver-specific promoters include albumin promoters (such as Palb), human α1-antitrypsin (such as Pa1AT), and hemopexin (such as Phpx) (Kramer et al. (2003), Mol. Therapy 7:375-85). Examples of eye-specific promoters include opsin, and corneal epithelium-specific K12 promoters (Martin et al. (2002), Methods (28): 267-75), (Tong et al. (2007), J. Gene Med, 9:956-66). These promoters, or other tissue-specific promoters known in the art, are not highly-active in HEK-293 cells and, thus, will not expected to yield significant levels of nuclease gene expression in packaging cells when incorporated into viral vectors of the present invention. Similarly, the viral vectors of the present invention contemplate the use of other cell lines with the use of incompatible tissue specific promoters (*i.e.*, the well-known HeLa cell line (human epithelial cell) and using the liver-specific hemopexin promoter). Other examples of tissue specific promoters include: synovial sarcomas PDZD4 (cerebellum), C6 (liver), ASB5 (muscle), PPP1R12B (heart), SLC5A12 (kidney), cholesterol regulation APOM (liver), ADPRHL1 (heart), and monogenic malformation syndromes TP73L (muscle). (Jacox et al. (2010), PLoS One v.5(8):e12274).
2. Alternatively, the vector can be packaged in cells from a different species in which the nuclease is not likely to be expressed. For example, viral particles can be produced in microbial, insect, or plant cells using mammalian promoters, such as the well-known cytomegalovirus- or SV40 virus-early promoters, which are not active in the non-mammalian packaging cells. In a preferred embodiment, viral particles are produced in insect cells using the baculovirus system as described by Gao *et al.* (Gao et al. (2007), J. Biotechnol. 131(2):138-43). A nuclease under the control of a mammalian promoter is unlikely to be expressed in these cells (Airenne et al. (2013), Mol. Ther. 21(4):739-49). Moreover, insect cells utilize different mRNA splicing motifs than mammalian cells. Thus, it is possible to incorporate a mammalian intron, such as the human growth hormone (HGH) intron or the SV40 large T antigen intron, into the coding sequence of a nuclease. Because these introns are not spliced efficiently from pre-mRNA transcripts in insect cells, insect cells will not express a functional nuclease and will package the full-length genome. In contrast, mammalian cells to which the resulting recombinant AAV particles are delivered will properly splice the pre-mRNA and will express functional nuclease protein. The use of the HGH and SV40 large T antigen introns to attenuate expression of the toxic proteins barnase and diphtheria toxin fragment A in insect packaging cells has been reported, enabling the production of recombinant AAV vectors carrying these toxin genes (Chen (2012), Mol. Ther. Nucleic Acids 1(11): e57).
3. The nuclease gene can be operably linked to an inducible promoter such that a small-molecule inducer is required for nuclease expression. Examples of inducible promoters include the Tet-On system (Clontech, Mountain View, CA); Chen et al. (2015), BMC Biotechnol. 15(1):4)) and the RheoSwitch system (Intrexon, Research Triangle Park, NC; Sowa et al. (2011), Spine 36(10): E623-8). Both systems, as well as similar systems known in the art, rely on ligand-inducible transcription factors (variants of the Tet Repressor and Ecdysone receptor, respectively) that activate transcription in response to a small-molecule activator (Doxycycline or Ecdysone, respectively). Practicing the current invention using such ligand-inducible transcription activators includes: 1) placing the nuclease gene under the control of a promoter that responds to the corresponding transcription factor, the nuclease gene having (a) binding site(s) for the transcription factor; and 2) including the gene encoding the transcription factor in the packaged viral genome, The latter step is necessary because the nuclease will not be expressed in the target cells or tissues following recombinant AAV delivery if the transcription activator is not also provided to the same cells. The transcription activator then induces nuclease gene expression only in cells or tissues that are treated with the cognate small-molecule activator. This approach is advantageous because it enables nuclease gene expression to be regulated in a spatiotemporal manner by selecting when and to which tissues the small-molecule inducer is delivered. However, the requirement to include the inducer in the viral genome, which has significantly limited carrying capacity, creates a drawback to this approach.
4. In another preferred embodiment, recombinant AAV particles are produced in a mammalian cell line that expresses a transcription repressor that prevents expression of the nuclease. Transcription repressors are known in the art and include the Tet-Repressor, the Lac-Repressor, the Cro repressor, and the Lambda-repressor. Many nuclear hormone receptors such as the ecdysone receptor also act as transcription repressors in the absence of their cognate hormone ligand. To practice the current invention, packaging cells are transfected/transduced with a vector encoding a transcription repressor and the nuclease gene in the viral genome (packaging vector) is operably linked to a promoter that is modified to comprise binding sites for the repressor such that the repressor silences the promoter. The gene encoding the transcription repressor can be placed in a variety of positions. It can be encoded on a separate vector; it can be incorporated into the packaging vector outside of the ITR sequences; it can be incorporated into the cap/rep vector or the adenoviral helper vector; or, most preferably, it can be stably integrated into the genome of the packaging cell such that it is expressed constitutively. Methods to modify common mammalian promoters to incorporate transcription repressor sites are known in the art. For example, the strong, constitutive CMV and RSV promoters have been modified to comprise operators for the Lac repressor and showed that gene expression from the modified promoters was greatly attenuated in cells expressing the repressor (Chang and Roninson (1996), Gene 183:137-42). The use of a non-human transcription repressor ensures that transcription of the nuclease gene will be repressed only in the packaging cells expressing the repressor and not in target cells or tissues transduced with the resulting recombinant AAV vector.

### 2.6 Engineered Nuclease Variants

Embodiments of the invention encompass the engineered nucleases described herein, and variants thereof. Further embodiments of the invention encompass isolated polynucleotides comprising a nucleic acid sequence encoding the nucleases described herein, and variants of such polynucleotides.

As used herein, the term "variants" is intended to mean substantially similar sequences. A "variant" polypeptide is intended to mean a polypeptide derived from the original or "native" polypeptide by deletion or addition of one or more amino acids at one or more internal sites in the native protein and/or substitution of one or more amino acids at one or more sites in the native polypeptide. As used herein, an original or "native" polynucleotide or polypeptide comprises a parental sequence from which variants are derived. Variant polypeptides encompassed by the embodiments are biologically active. That is, they continue to possess the desired biological activity of the native protein; *i*.*e*.*,* the ability to recognize and cleave recognition sequences found upstream and downstream of a chromosomal locus to be deleted (*e.g.*, a pathogenic nucleotide repeat expansion in a gene of interest). Such variants may result, for example, from human manipulation or natural mutagenesis. Biologically active variants of a native polypeptide of the embodiments, or biologically active variants of the recognition half-site binding subunits described herein, will have at least about 40%, about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, or about 99%, sequence identity to the amino acid sequence of the native polypeptide or native subunit, as determined by sequence alignment programs and parameters described elsewhere herein. A biologically active variant of a polypeptide or subunit of the embodiments may differ from that polypeptide or subunit by as few as about 1-40 amino acid residues, as few as about 1-20, as few as about 1-10, as few as about 5, as few as 4, 3, 2, or even 1 amino acid residue.

The polypeptides of the embodiments may be altered in various ways including amino acid substitutions, deletions, truncations, and insertions. Methods for such manipulations are generally known in the art. For example, amino acid sequence variants can be prepared by mutations in the DNA of the original or native form. Methods for mutagenesis and polynucleotide alterations are well known in the art. See, for example, Kunkel (1985), Proc. Natl. Acad. Sci. USA 82:488-492; Kunkel et al. (1987), Methods in Enzymol. 154:367-382; U.S. Pat. No. 4,873,192; Walker and Gaastra, eds. (1983), Techniques in Molecular Biology (MacMillan Publishing Company, New York) and the references cited therein. Guidance as to appropriate amino acid substitutions that do not affect biological activity of the protein of interest may be found in the model of Dayhoff et al. (1978), Atlas of Protein Sequence and Structure (Natl. Biomed. Res. Found., Washington, D.C.), herein incorporated by reference. Conservative substitutions, such as exchanging one amino acid with another having similar properties, may be optimal.

A substantial number of amino acid modifications to the DNA recognition domain of the wild-type I-Crel meganuclease have previously been identified (*e*.*g*., U.S. Pat. No. 8,021,867) which, singly or in combination, result in recombinant meganucleases with specificities altered at individual bases within the DNA recognition sequence half-site, such that the resulting rationally-designed meganucleases have half-site specificities different from the wild-type enzyme. Table 21 provides potential substitutions that can be made in a recombinant I-Crel-derived meganuclease monomer or subunit to enhance specificity based on the base present at each half-site position (-1 through -9) of a recognition half-site.

**Table 21.**

| | Favored Sense-Strand Base | | | | | | | | | | |
|---|---|---|---|---|---|---|---|---|---|---|---|
| Posn. | A | C | G | T | A/T | A/C | A/G | C/T | G/T | A/G/T | A/C/G/T |
| -1 | Y75 | **R70*** | K70 | Q70* | | | | **T46*** | | | G70 |
| | L75* | H75* | E70* | C70 | | | | | | | A70 |
| | C75* | R75* | E75* | L70 | | | | | | | S70 |
| | Y139* | H46* | E46* | Y75* | | | | | | | G46* |
| | C46* | K46* | D46* | Q75* | | | | | | | |
| | A46* | R46* | | H75* | | | | | | | |
| | | | | H139 | | | | | | | |
| | | | | Q46* | | | | | | | |
| | | | | H46* | | | | | | | |
| -2 | Q70 | E70 | H70 | **Q44*** | C44* | | | | | | |
| | T44* | D70 | D44* | | | | | | | | |
| | A44* | K44* | E44* | | | | | | | | |
| | V44* | R44* | | | | | | | | | |
| | I44* | | | | | | | | | | |
| | L44* | | | | | | | | | | |
| | N44* | | | | | | | | | | |
| -3 | Q68 | E68 | **R68** | M68 | | H68 | | Y68 | K68 | | |
| | C24* | F68 | | C68 | | | | | | | |
| | **I24*** | K24* | | L68 | | | | | | | |
| | | R24* | | F68 | | | | | | | |
| -4 | A26* | E77 | R77 | | | | | S77 | | | S26* |
| | Q77 | K26* | E26* | | | | | **Q26*** | | | |
| -5 | | E42 | R42 | | | **K28*** | C28* | | | | M66 |
| | | | | | | | Q42 | | | | K66 |
| -6 | Q40 | E40 | R40 | C40 | A40 | | | | | | **S40** |
| | C28* | R28* | | I40 | A79 | | | | | | S28* |
| | | | | V40 | A28* | | | | | | |
| | | | | C79 | H28* | | | | | | |
| | | | | I79 | | | | | | | |
| | | | | V79 | | | | | | | |
| | | | | Q28* | | | | | | | |
| -7 | **N30*** | E38 | K38 | I38 | | | C38 | | | | H38 |
| | **Q38** | K30* | R38 | L38 | | | | | | | N38 |
| | | R30* | E30* | | | | | | | | Q30* |
| -8 | F33 | E33 | F33 | L33 | | R32* | R33 | | | | |
| | **Y33** | D33 | H33 | V33 | | | | | | | |
| | | | | I33 | | | | | | | |
| | | | | F33 | | | | | | | |
| | | | | C33 | | | | | | | |
| -9 | | E32 | R32 | L32 | | | | D32 | | | **S32** |
| | | | K32 | V32 | | | | I32 | | | N32 |
| | | | | A32 | | | | | | | H32 |
| | | | | C32 | | | | | | | Q32 |
| | | | | | | | | | | | T32 |

For polynucleotides, a "variant" comprises a deletion and/or addition of one or more nucleotides at one or more sites within the native polynucleotide. One of skill in the art will recognize that variants of the nucleic acids of the embodiments will be constructed such that the open reading frame is maintained. For polynucleotides, conservative variants include those sequences that, because of the degeneracy of the genetic code, encode the amino acid sequence of one of the polypeptides of the embodiments. Variant polynucleotides include synthetically derived polynucleotides, such as those generated, for example, by using site-directed mutagenesis but which still encode a recombinant meganuclease of the embodiments. Generally, variants of a particular polynucleotide of the embodiments will have at least about 40%, about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, about 99% or more sequence identity to that particular polynucleotide as determined by sequence alignment programs and parameters described elsewhere herein. Variants of a particular polynucleotide of the embodiments (i.e., the reference polynucleotide) can also be evaluated by comparison of the percent sequence identity between the polypeptide encoded by a variant polynucleotide and the polypeptide encoded by the reference polynucleotide.

The deletions, insertions, and substitutions of the protein sequences encompassed herein are not expected to produce radical changes in the characteristics of the polypeptide. However, when it is difficult to predict the exact effect of the substitution, deletion, or insertion in advance of doing so, one skilled in the art will appreciate that the effect can be evaluated by routine screening of the polypeptide for its ability to preferentially recognize and cleave recognition sequences found upstream or downstream of a chromosomal locus to be deleted (*e*.*g*., a pathogenic nucleotide repeat expansion in a gene of interest).

### EXAMPLES

This invention is further illustrated by the following examples, which should not be construed as limiting. Those skilled in the art will recognize, or be able to ascertain, using no more than routine experimentation, numerous equivalents to the specific substances and procedures described herein. Such equivalents are intended to be encompassed in the scope of the claims that follow the examples below.

### EXAMPLE 1

### Deletion of the FXN Trinucleotide Repeat Using a Pair of Engineered, Single-Chain Meganucleases

### 1. Meganucleases that recognize SEQ ID NO: 29, SEQ ID NO: 63, and SEQ ID NO: 89

An engineered meganuclease called "FXN 1-2x.63" (SEQ ID NO: 143) was made which recognizes and cuts SEQ ID NO: 29 in *FXN* Intron 1 upstream of the GAA trinucleotide repeat. A second engineered meganuclease called "FXN 11-12x.63" (SEQ ID NO: 182) was produced which recognizes and cleaves SEQ ID NO: 63 in *FXN* Intron 1 upstream of the GAA trinucleotide repeat. A third engineered meganuclease called "FXN 5-6x.24" (SEQ ID NO: 172) was made which recognizes and cuts SEQ ID NO: 89 in *FXN* Intron 1 downstream of the GAA trinucleotide repeat (Figure 6A). Each meganuclease comprises an N-terminal nuclease-localization signal derived from SV40, a first meganuclease subunit, a linker sequence, and a second meganuclease subunit.

To determine whether each FXN meganuclease could recognize and cleave its respective recognition sequence, each recombinant meganuclease was evaluated using a CHO cell reporter assay previously described (see, WO2012/167192 and Figure 4). To perform the assays, CHO cell reporter lines were produced which carried a non-functional Green Fluorescent Protein (GFP) gene expression cassette integrated into the genome of the cells. The GFP gene in each cell line was interrupted by a pair of recognition sequences such that intracellular cleavage of either recognition sequence by a meganuclease would stimulate a homologous recombination event resulting in a functional GFP gene.

In CHO reporter cell lines developed for this study, one recognition sequence inserted into the GFP gene was the FXN 1-2 recognition sequence (SEQ ID NO: 29), the FXN 11-12 recognition sequence (SEQ ID NO: 63), or the FXN 5-6 recognition sequence (SEQ ID NO: 89). The second recognition sequence inserted into the GFP gene was a CHO-23/24 recognition sequence, which is recognized and cleaved by a control meganuclease called "CHO-23/24". CHO reporter cells comprising the FXN 1-2 recognition sequence and the CHO-23/24 recognition sequence are referred to herein as "FXN 1-2 cells." CHO reporter cells comprising the FXN 11-12 recognition sequence and the CHO-23/24 recognition sequence are referred to herein as "FXN 11-12 cells." CHO reporter cells comprising the FXN 5-6 recognition sequence and the CHO-23/24 recognition sequence are referred to herein as "FXN 5-6 cells."

CHO reporter cells were transfected with plasmid DNA encoding their corresponding recombinant meganucleases (*e*.*g.*, FXN 1-2 cells were transfected with plasmid DNA encoding the FXN 1-2 meganuclease) or encoding the CHO-23/24 meganuclease. In each assay, 4x10⁵ CHO reporter cells were transfected with 50 ng of plasmid DNA in a 96-well plate using Lipofectamine 2000 (ThermoFisher) according to the manufacturer's instructions. At 48 hours post-transfection, cells were evaluated by flow cytometry to determine the percentage of GFP-positive cells compared to an untransfected negative control (FXN 1-2bs, FXN 11-12bs, or FXN 5-6bs). As shown in Figure 5, the FXN 1-2x.63, FXN 11-12x.63, and FXN 5-6x.24 meganucleases were found to produce GFP-positive cells in cell lines comprising their corresponding recognition sequence at frequencies significantly exceeding the negative control.

### 2. Deletion of the FXN Trinucleotide Repeat in HEK-293 cells

Human embryonic kidney (HEK-293) cells were co-transfected with mRNA encoding FXN 1-2x.63 and FXN 5-6x.24 or FXN 11-12x.63 and FXN 5-6x.24. mRNA was prepared by first producing a PCR template for an *in vitro* transcription reaction (SEQ ID NO: 136, SEQ ID NO: 137, and SEQ ID NO: 138). Each PCR product included a T7 promoter and 609 bp of vector sequence downstream of the meganuclease gene. The PCR product was gel purified to ensure a single template. Capped (m7G) RNA was generated using the RiboMAX T7 kit (Promega, Madison, WI) according to the manufacturer's instructions. Ribo m7G cap analog (Promega, Madison, WI) was included in the reaction and 0.5 µg of the purified meganuclease PCR product served as the DNA template. Capped RNA was purified using the SV Total RNA Isolation System (Promega, Madison, WI) according to the manufacturer's instructions.

1.5x10⁶ HEK-293 cells were nucleofected with 1.5x10¹² copies of mRNA encoding the upstream meganuclease (FXN 1-2x.63 or FXN 11-12x.63) and 1.5x10¹² copies of mRNA encoding FXN 5-6x.24 (2x10⁶ copies/cell) using an Amaxa Nucleofector II device (Lonza, Basel, Switzerland) according to the manufacturer's instructions. 48 hours post-transfection, genomic DNA was isolated from the cells using a FlexiGene kit (Qiagen, Hilden, Germany) according to the manufacturer's instructions. The genomic DNA was then subjected to PCR using primers flanking the GAA trinucleotide repeat and adjacent meganuclease recognition sequences (SEQ ID NO: 139 and SEQ ID NO: 140). When PCR products were resolved by agarose gel electrophoresis, it was apparent that cells co-expressing the nuclease pairs yielded two PCR products (Figure 3B). The larger product is consistent in size with the expected PCR product from cells with an unmodified *FXN* gene. The smaller band in each case is consistent in size with what would be expected if the corresponding nuclease pair deleted a fragment from the *FXN* gene.

The smaller PCR products were isolated from the gel and cloned into a bacterial plasmid (pUC-19) for sequence analysis. Two plasmid clones were sequenced for each of the two transfections and it was found that all four plasmids contained a fragment of *FXN* Intron 1 in which a portion of the intron comprising the GAA trinucleotide repeat was deleted. In each case, the deletion spanned the two meganuclease cut sites and extended a variable distance 5' of the upstream cut site. As a consequence, all four sequences obtained were different. It is important to note that the meganucleases used in this experiment did not generate compatible overhangs and, therefore, a consistent sequence resulting from direct re-ligation events was not expected.

### 3. Conclusions

We have demonstrated that it is possible to use a pair of engineered single-chain meganucleases to excise a fragment from the human genome comprising a nucleotide repeat implicated in disease.

### EXAMPLE 2

### Deletion of the FXN Trinucleotide Repeat Using Additional Pairs of Engineered, Single-Chain Meganucleases

### 1. Meganucleases that recognize FXN 1-2 (SEQ ID NO: 29), FXN 3-4 (SEQ ID NO: 34), FXN 5-6 (SEQ ID NO: 63), and FXN 11-12 (SEQ ID NO: 89) recognition sequence

To identify engineered meganucleases with different cleavage capabilities than the FXN meganucleases described above, we generated additional engineered meganucleases targeting FXN 1-2 (SEQ ID NO: 29), FXN 11-12 (SEQ ID NO: 63), and FXN 5-6 (SEQ ID NO: 89) recognition sequences. In addition, new engineered meganucleases referred to as FXN 3-4 meganucleases were made to recognize and cut the FXN 3-4 recognition sequence (SEQ ID NO: 34), found 5' upstream of the GAA trinucleotide repeat. FXN 3-4 was added because cleavage at its recognition sequence would generate compatible 3' overhangs with overhangs produced by FXN 5-6 meganucleases. As described above, each engineered meganuclease comprises an N-terminal nuclease-localization signal derived from SV40, a first meganuclease subunit, a linker sequence, and a second meganuclease subunit.

These engineered meganucleases were tested in the CHO cell reporter assay as described above, and the results are shown in Figure 8. Figure 8A shows that several variant FXN 1-2 meganucleases, FXN 1-2x.11 (SEQ ID NO: 144), FXN1-2x.73 (SEQ ID NO: 145), and FXN 1-2x.84 (SEQ ID NO: 146) produce GFP-positive cells in cell lines comprising the FXN 1-2 recognition sequence at frequencies significantly exceeding the negative control. Figures 8B, 8C and 8D show the results for several variant FXN 3-4 meganucleases: FXN 3-4L.34 (SEQ ID NO: 155), FXN 3-4L.5 (SEQ ID NO: 156), FXN 3-4L.12 (SEQ ID NO: 157), FXN 3-4x.312 (SEQ ID NO: 158), and FXN 3-4x.383 (SEQ ID NO: 159) all produce GFP-positive cells with high frequencies, albeit slightly lower than the positive controls. Figures 8E and 8F show the results for several variant FXN 5-6 meganucleases: FXN 5-6L.45 (SEQ ID NO: 170), FXN 5-6.L38 (SEQ ID NO: 171), and FXN 5-6x.20 (SEQ ID NO: 173) all produce GFP-positive cells at frequencies equal to or greater than the positive control. Figure 8G shows results for several variant FXN 11-12 meganucleases: FXN 11-12x.99 (SEQ ID NO: 183), FXN 11-12x.107 (SEQ ID NO: 184), and FXN 11-12x.139 (SEQ ID NO: 185) all produced GFP-positive cells in cell lines comprising the FXN 11-12 recognition sequence at frequencies greater than or equal to the positive control

### 2. Deletion of trinucleotide repeat in mammalian cells and detection of re-ligation

Since FXN 3-4 and FXN 5-6 meganucleases produce compatible overhangs, we next looked to determine whether we could detect signs of direct re-ligation. HEK 293 cells were electroporated as described above with a FXN 3-4/FXN 5-6 pair; the new FXN 3-4x.312 and the previously used FXN 5-6x.24). PCR using primers flanking the trinucleotide repeat was performed, and PCR products were resolved by agarose gel electrophoresis. As described above, two PCR products were observed after 3 days and 6 days (Figure 9A). The smaller band was gel purified and cloned, and 38 clones were sequenced. 35 of the 38 clones showed that the trinucleotide repeat had been entirely deleted in cells. Of those 35 clones showing complete deletion of the repeat region, 20 clones showed perfect re-ligation of the overhangs generated by FXN 3-4 and FXN 5-6 meganucleases (Figure 9B).

### 3. Deletion of trinucleotide repeat in human fibroblasts

To further test whether the new meganucleases could delete the trinucleotide repeat, FXN 3-4x.312 and FXN 5-6x.24 were tested in fibroblasts derived from a patient homozygous for the expansion in the *FXN* gene, with alleles of approximately 330 and 390 repeats (GM03816, Coriell Institute for Medical Research). In this experiment, the FXN 3-4 and FXN 5-6 pair was used since they generate compatible 3' overhangs and could potentially delete the trinucleotide repeat in a consistent manner due to direct re-ligation of the overhangs. RNA for the FXN 3-4 and FXN 5-6 meganucleases was generated as described above and GM03816 cells were transfected using the Stemfect RNA transfection reagent (Stemgent, Cambridge, MA). Using PCR analysis at 4 days (Figure 10A) and 18 days (Figure 10B), two PCR products were again observed in samples treated with the FXN 3-4/FXN 5-6 meganuclease pair, indicating the deletion of the GAA repeat region.

Additionally, at 10 days post-transfection, RNA was obtained from cells transfected with the FXN 3-4 meganuclease, the FXN 5-6 meganuclease, or the FXN 3-4/FXN 5-6 meganuclease pair, which was then analyzed by quantitative PCR for change in FXN mRNA expression. As shown in Figure 10C, meganuclease transfection was accompanied by an increased in frataxin mRNA expression in GM03816 human fibroblasts.

To further test whether the newly designed meganucleases could delete the GAA trinucleotide repeat expansion, several combinations were tested in GM03816 fibroblasts. In this experiment only FXN 3-4 and FXN 5-6 pairs were used since they generate compatible 3' overhangs and could potentially delete the trinucleotide repeat in a consistent manner due to direct re-ligation of the overhangs. RNA for FXN 3-4 and FXN 5-6 meganucleases was generated as described above and GM03816 cells were transfected with the pairs shown in Table 22 using the Stemfect RNA transfection reagent (Stemgent, Cambridge, MA).

At both 3 and 6 days post-transfection, genomic DNA was harvested and the cells were analyzed for *FXN* repeat deletion by a digital PCR assay. In the digital PCR assay, two primer sets are used along with two fluorescently labeled probes. The first primer set amplifies a region downstream of the FXN 3-4 recognition sequence, but upstream of the trinucleotide repeats in the *FXN* gene. The second primer set amplifies an irrelevant gene sequence and serves as a reference sequence to control for template number. A carboxyfluorescein (FAM)-labeled probe anneals within the amplicon generated from the first primer set and a VIC-labeled probe anneals within the amplicon generated by the second set of primers. If the FXN 3-4/FXN 5-6 meganuclease pairs successfully delete the region between the two recognition sequences, the probe binding the first amplicon no longer anneals and the signal is lost. Thus, by comparing the ratio of FAM:VIC, it is possible to accurately quantitate what percent of the cells have had the region between the FXN 3-4 and FXN 5-6 recognition sequences deleted.

**Table 22.**

| FXN 3-4/FXN 5-6 pair | | % Deleted (Day 3) | % Deleted (Day 6) |
|---|---|---|---|
| FXN 3-4x.312 | FXN 5-6x.38 | 10.5 | 6.2 |
| FXN 3-4x.5 | FXN 5-6x.38 | 16.3 | 12.8 |
| FXN 3-4x.12 | FXN 5-6x.38 | 17.3 | 15.1 |
| FXN 3-4x.34 | FXN 5-6x.38 | 17.4 | 15.9 |
| FXN 3-4x.34 | FXN 5-6x.45 | 21.4 | 18.4 |
| FXN 3-4x.312 | FXN 5-6x.24 | 13.5 | 10 |
| Mock-transfected control | | 0 | 0 |
| Non-transfected control | | 0 | 0 |

As shown in Table 22, the FXN 3-4/FXN 5-6 pairs tested in GM03816 cells were all capable of deleting the repeat region, with efficiencies ranging from 10.5% to 21.4% at day 3 post-transfection. At day 6 post-transfection, deletion efficiencies were decreased slightly but still demonstrated that up to 18.4% of the cells had the trinucleotide repeat deleted.

Further, genomic DNA was obtained on day 3 samples from the above experiment, which was then analyzed by deep sequencing to assess the efficiency of precise end joining between the FXN 3-4 and FXN 5-6 cleavage sites after excision of the GAA repeat region. As shown in Table 23, the efficiency of precision end joining was exceptionally high, exceeding 83% in all samples.

**Table 23.**

| FXN 3-4/FXN 5-6 pair | | % Precise End Joining |
|---|---|---|
| FXN 3-4x.312 | FXN 5-6x.38 | 90.7% |
| FXN 3-4x.5 | FXN 5-6x.38 | 88.6% |
| FXN 3-4x.12 | FXN 5-6x.38 | 86.1% |
| FXN 3-4x.34 | FXN 5-6x.38 | 84.7% |
| FXN 3-4x.34 | FXN 5-6x.45 | 83.6% |
| FXN 3-4x.312 | FXN 5-6x.24 | 83.7% |

### 4. Conclusions

These data clearly demonstrate that it is possible to use two engineered meganucleases flanking the trinucleotide repeat region in the FXN gene to delete the repeat region. Data from HEK 293 cells and from fibroblasts derived from FRDA patients show that deletion of the repeat region is highly efficient, removing the repeat in over 20% of cells in one assay. Moreover, if the two engineered meganucleases generate compatible 3' overhangs, the overhangs can directly re-ligate, resulting in highly predictable, consistent repaired sequence in over 50% of clones.

### EXAMPLE 3

### Engineered, Single-Chain Meganucleases With Specificity For Recognition Sequences Within the C9ORF72 Gene

### 1. Meganucleases that recognize ORF 7-8 (SEQ ID NO: 194), ORF 9-10 (SEQ ID NO: 195), ORF 11-12 (SEQ ID NO: 196), and ORF 13-14 (SEQ ID NO: 197)

Several engineered meganucleases were made to recognize and cut 5' upstream or 3' downstream of the GGGGCC hexanucleotide repeat in the *C9ORF72* gene. Each meganuclease comprises an N-terminal nuclease-localization signal derived from SV40, a first meganuclease subunit, a linker sequence, and a second meganuclease subunit.

A first series of meganucleases recognize and cleave the upstream ORF 7-8 recognition sequence (SEQ ID NO: 194). These meganucleases include ORF 7-8x.4 (SEQ ID NO: 198), ORF 7-8x.31 (SEQ ID NO: 199), ORF 7-8x.45 (SEQ ID NO: 200), and ORF 7-8x.29 (SEQ ID NO: 201). A second series of meganucleases recognize and cleave the downstream ORF 9-10 recognition sequence (SEQ ID NO: 195). These meganucleases include ORF 9-10x.15 (SEQ ID NO: 210), ORF 9-10x.14 (SEQ ID NO: 211), ORF 9-10x.61 (SEQ ID NO: 212), and ORF 9-10x.77 (SEQ ID NO: 213). Following cleavage, ORF 7-8 and ORF 9-10 meganucleases will generate compatible overhangs to promote direct re-ligation.

A third series of engineered meganucleases recognize and cleave the downstream ORF 11-12 recognition sequence (SEQ ID NO: 196). These meganucleases include ORF 11-12x.5 (SEQ ID NO: 222), ORF 11-12L.1 (SEQ ID NO: 223), ORF 11-12L.8 (SEQ ID NO: 224), and ORF 11-12L.64 (SEQ ID NO: 225). A fourth series of engineered meganucleases recognize and cleave the upstream ORF 13-14 recognition sequence (SEQ ID NO: 197). These include ORF 13-14x.40 (SEQ ID NO: 234), ORF 13-14x.77 (SEQ ID NO: 235), ORF 13-14x.90 (SEQ ID NO: 236), and ORF 13-14x.3 (SEQ ID NO: 237). Following cleavage, ORF 11-12 and ORF 13-14 meganucleases will generate compatible overhangs to promote direct re-ligation.

To determine whether each ORF meganuclease could recognize and cleave its respective recognition sequence, each recombinant meganuclease was evaluated using a CHO cell reporter assay previously described (see, WO2012/167192 and Figure 4). To perform the assays, CHO cell reporter lines were produced which carried a non-functional Green Fluorescent Protein (GFP) gene expression cassette integrated into the genome of the cells. The GFP gene in each cell line was interrupted by a pair of recognition sequences such that intracellular cleavage of either recognition sequence by a meganuclease would stimulate a homologous recombination event resulting in a functional GFP gene.

In CHO reporter cell lines developed for this study, one recognition sequence inserted into the GFP gene was the ORF 7-8 recognition sequence (SEQ ID NO: 194), the ORF 9-10 recognition sequence (SEQ ID NO: 195), the ORF 11-12 recognition sequence (SEQ ID NO: 196), or the ORF 13-14 recognition sequence (SEQ ID NO: 197). The second recognition sequence inserted into the GFP gene was a CHO-23/24 recognition sequence, which is recognized and cleaved by a control meganuclease called "CHO-23/24". CHO reporter cells comprising the ORF 7-8 recognition sequence and the CHO-23/24 recognition sequence are referred to herein as "ORF 7-8 cells." CHO reporter cells comprising the ORF 9-10 recognition sequence and the CHO-23/24 recognition sequence are referred to herein as "ORF 9-10 cells." CHO reporter cells comprising the ORF 11-12 recognition sequence and the CHO-23/24 recognition sequence are referred to herein as "ORF 11-12 cells." CHO reporter cells comprising the ORF 13-14 recognition sequence and the CHO-23/24 recognition sequence are referred to herein as "ORF 13-14 cells."

CHO reporter cells were transfected with plasmid DNA encoding their corresponding recombinant meganucleases (*e*.*g.*, ORF 7-8 cells were transfected with plasmid DNA encoding the ORF 7-8 meganuclease) or encoding the CHO-23/24 meganuclease. In each assay, 4x10⁵ CHO reporter cells were transfected with 50 ng of plasmid DNA in a 96-well plate using Lipofectamine 2000 (ThermoFisher, Waltham, MA) according to the manufacturer's instructions. At 48 hours post-transfection, cells were evaluated by flow cytometry to determine the percentage of GFP-positive cells compared to an untransfected negative control. As shown in Figure 11A, the ORF 7-8x.4, ORF 7-8x.31, ORF 7-8x.45, and ORF 7-8x.29 meganucleases were all found to produce GFP-positive cells in cell lines comprising their corresponding recognition sequence at frequencies significantly exceeding the negative control. Figure 11B demonstrates that ORF 9-10x.15, ORF 9-10x.14, ORF 9-10x.61, and ORF 9-10x.77 all produced GFP-positive cells with significant frequencies; ORF 9-10x.14 exceeded the positive control. The ORF 11-12L.1, ORF 11-12L.8, ORF 11-12L.64, and ORF 11-12x.5 all produced GFP-positive cells with frequencies equal to or greater than the positive control (Figures 11C and 11D). ORF 13-14x.40, ORF 13-14x.77, ORF 13-14x.90, and ORF 13-14x.3 all generated GFP-positive cells with frequencies greater than or equal to the positive control (Figure 11E).

### 2. Deletion of hexanucleotide repeats in mammalian cells

To test whether the ORF meganucleases could delete the hexanucleotide repeat, several combinations were tested in HEK 293 cells. Since ORF 7-8 and ORF 9-10 generate compatible overhangs, and ORF 11-12 and ORF 13-14 generate compatible overhangs, these pairs could delete the hexanucleotide repeat in a consistent manner due to direct re-ligation of the overhangs. RNA for ORF meganucleases was generated and HEK 293 cells were electroporated as described above using the following ORF meganuclease pairs: ORF 7-8x.4 and ORF 9-10x.15; ORF 11-12x.5 and ORF 13-14x.40 (Figure 12A). 48 hours post-transfection, DNA was isolated, PCR was performed using primers that flank the hexanucleotide repeat region and PCR products were resolved by agarose gel electrophoresis. In mock-transfected cells, the large band at approximately 850 bp represents the unmodified region. In cells transfected with either ORF 7-8/ORF 9-10 or ORF 11-12/ORF 13-14, the presence of smaller bands are consistent in size with what would be expected if the corresponding nuclease pair deleted a fragment from the *C9ORF72* gene.

To determine whether the ORF meganucleases with compatible ends were able to directly re-ligate upon removal of the intervening sequence, the small PCR bands shown in Figure 12A were gel purified and deep sequenced (MiSeq, Illumina, San Diego, CA). Sequencing reads were aligned to the product predicted if direct re-ligation occurred. As shown in Table 24, both pairs of ORF meganucleases demonstrated direct re-ligation of compatible overhangs in the majority of sequences. In particular, sequences from cells transfected with the ORF 11-12/ORF 13-14 pair directly re-ligated the overhangs in over 80% of the sequences.

**Table 24.**

| Meganuclease pair | Total # sequences assembled | Total # full length sequence | Total aligned with predicted sequence | Percent aligned with predicted sequence |
|---|---|---|---|---|
| ORF 7-8/ORF 9-10 | 1197408 | 1059686 | 610297 | 57.6 |
| ORF 11-12/ORF 13-14 | 1036131 | 952325 | 783452 | 82.3 |

To test whether the ORF meganucleases could delete the hexanucleotide repeat expansion in patient-derived cells, the same ORF combinations were tested in fibroblasts derived from a patient homozygous for the expansion in the *C9ORF72* gene (ND42496, Coriell Institute for Medical Research). RNA for the ORF meganucleases was generated as described above and ND42496 cells were transfected with the same pairs discussed above using the Stemfect RNA transfection reagent (Stemgent) according to the manufacturer's instructions. 48 hours post-transfection, DNA was isolated, PCR was performed using primers that flank the hexanucleotide repeat region and PCR products were resolved by agarose gel electrophoresis (Figure 12B). In contrast to HEK 293 cells, no PCR product is detected in mock-transfected cells. This is likely due to the difficulty of amplifying a region with a repetitive region with 100% GC content. However, in cells transfected with either ORF 7-8/ORF 9-10 or ORF 11-12/ORF 13-14, the presence of smaller bands are consistent in size with what would be expected if the corresponding nuclease pair deleted a fragment from the *C9ORF72* gene.

### 3. Conclusions

These data clearly demonstrate that it is possible to use two engineered meganucleases flanking the hexanucleotide repeat region in the *C9ORF72* gene to delete the repeat region in both HEK 293 cells and from fibroblasts derived from patients. Moreover, engineered meganucleases that generate compatible overhangs show evidence of direct re-ligation with high frequency, resulting in highly predictable, consistent repaired sequence.

### ITEMS

1. A method for treating a subject having a nucleotide repeat expansion disorder, wherein said nucleotide repeat expansion disorder is characterized by expansion of a nucleotide repeat in a gene of interest, said method comprising delivering to target cells in said subject:
   (a) at least a first engineered nuclease protein and a second engineered nuclease protein; or
   (b) at least a first nucleic acid encoding said first engineered nuclease and a second nucleic acid encoding said second engineered nuclease, wherein said first engineered nuclease and said second engineered nuclease are expressed in said target cells *in vivo*;

   wherein said first engineered nuclease recognizes and cleaves a first recognition sequence positioned 5' upstream of said nucleotide repeat in said gene of interest;
   and wherein said second engineered nuclease recognizes and cleaves a second recognition sequence positioned 3' downstream of said nucleotide repeat in said gene of interest;
   and wherein an intervening DNA fragment between said first recognition sequence and said second recognition sequence is excised and the number of said nucleotide repeat is reduced in said gene of interest.
2. The method of item 1, wherein said engineered nuclease is an engineered meganuclease, a compact TALEN, or a CRISPR.
3. The method of item 1 or item 2, wherein said first engineered nuclease and said second engineered nuclease generate complementary overhangs which promote direct re-ligation of said gene of interest.
4. The method of any one of items 1-3, wherein said first recognition sequence and said second recognition sequence are positioned within the same exon, the same intron, or the same untranslated region (UTR) as said nucleotide repeat.
5. The method of any one of items 1-4, wherein said nucleotide repeat is a trinucleotide repeat.
6. The method of item 5, wherein said trinucleotide repeat is selected from the group consisting of CAG, CGG, CCG, GAA, and CTG.
7. The method of item 5 or item 6, wherein said trinucleotide repeat is GAA and said gene of interest is the frataxin (*FXN*) gene, wherein said trinucleotide repeat is positioned within intron 1 of the *FXN* gene.
8. The method of item 7, wherein said first recognition sequence is positioned 5' upstream in said intron 1 (SEQ ID NO: 74) of said trinucleotide repeat.
9. The method of item 7 or item 8, wherein said second recognition sequence is positioned 3' downstream in said intron 1 (SEQ ID NO: 96) of said trinucleotide repeat.
10. The method of any one of items 7-9, wherein said first engineered nuclease is a first engineered meganuclease and said second engineered nuclease is a second engineered meganuclease.
11. The method of item 10, wherein said first recognition sequence comprises any one of SEQ ID NOs: 12-73.
12. The method of item 10 or item 11, wherein said first recognition sequence comprises SEQ ID NO: 34.
13. The method of item 12, wherein said first engineered meganuclease comprises a first subunit and a second subunit, wherein said first subunit binds to a first recognition half-site of said first recognition sequence and comprises a first hypervariable (HVR1) region, and wherein said second subunit binds to a second recognition half-site of said first recognition sequence and comprises a second hypervariable (HVR2) region.
14. The method of item 13, wherein said first subunit comprises an amino acid sequence having at least 80% sequence identity to residues 7-153 of any one of SEQ ID NOs: 155-159, and wherein said second subunit comprises an amino acid sequence having at least 80% sequence identity to residues 198-344 of any one of SEQ ID NOs: 155-159.
15. The method of item 13 or item 14, wherein said HVR1 region comprises residues 24-79 of any one of SEQ ID NOs: 155-159.
16. The method of any one of items 13-15, wherein said HVR2 region comprises residues 215-270 of any one of SEQ ID NOs: 155-159.
17. The method of any one of items 13-16, wherein said first subunit comprises residues 7-153 of any one of SEQ ID NOs: 155-159.
18. The method of any one of items 13-17, wherein said second subunit comprises residues 198-344 of any one of SEQ ID NOs: 155-159.
19. The method of any one of items 13-18, wherein said first engineered meganuclease comprises the amino acid sequence of any one of SEQ ID NOs: 155-159.
20. The method of any one of items 10-19, wherein said second recognition sequence comprises any one of SEQ ID NOs: 75-95.
21. The method of any one of items 10-20, wherein said second recognition sequence comprises SEQ ID NO: 89.
22. The method of item 21, wherein said second engineered meganuclease comprises a first subunit and a second subunit, wherein said first subunit binds to a first recognition half-site of said second recognition sequence and comprises a first hypervariable (HVR1) region, and wherein said second subunit binds to a second recognition half-site of said second recognition sequence and comprises a second hypervariable (HVR2) region.
23. The method of item 22, wherein said first subunit comprises an amino acid sequence having at least 80% sequence identity to residues 198-344 of any one of SEQ ID NOs: 170-172, or residues 7-153 of SEQ ID NO: 173, and wherein said second subunit comprises an amino acid sequence having at least 80% sequence identity to residues 7-153 of any one of SEQ ID NOs: 170-172, or residues 198-344 of SEQ ID NO: 173.
24. The method of item 22 or item 23, wherein said HVR1 region comprises residues 215-270 of any one of SEQ ID NOs: 170-172, or residues 24-79 of SEQ ID NO: 173.
25. The method of any one of items 22-24, wherein said HVR2 region comprises residues 24-79 of any one of SEQ ID NOs: 170-172, or residues 215-270 of SEQ ID NO: 173.
26. The method of any one of items 22-25, wherein said first subunit comprises residues 198-344 of any one of SEQ ID NOs: 170-172, or residues 7-153 of SEQ ID NO: 173.
27. The method of any one of items 22-26, wherein said second subunit comprises residues 7-153 of any one of SEQ ID NOs: 170-172, or residues 198-344 of SEQ ID NO: 173.
28. The method of any one of items 22-27, wherein said second engineered meganuclease comprises the amino acid sequence of any one of SEQ ID NOs: 170-173.
29. The method of any one of items 1-28, wherein said method comprises administering to said subject a pharmaceutical composition comprising a pharmaceutically acceptable carrier and:
   (a) said first nucleic acid encoding said first engineered nuclease of any one of items 1-28 and said second nucleic acid encoding a second engineered nuclease of any one of items 1-28, wherein said first engineered nuclease and said second engineered nuclease are expressed in a target cell *in vivo*; or
   (b) said first engineered nuclease protein of any one of items 1-28 and said second engineered nuclease protein of any one of items 1-28.
30. A pharmaceutical composition for treatment of a subject having a nucleotide repeat expansion disorder, wherein said nucleotide repeat expansion disorder is characterized by expansion of a nucleotide repeat in a gene of interest, said pharmaceutical composition comprising a pharmaceutically acceptable carrier and:
   (a) a first nucleic acid encoding a first engineered nuclease and a second nucleic acid encoding a second engineered nuclease, wherein said first engineered nuclease and said second engineered nuclease are expressed in a target cell *in vivo*; or
   (b) a first engineered nuclease protein and a second engineered nuclease protein;

   wherein said first engineered nuclease recognizes and cleaves a first recognition sequence positioned 5' upstream of said nucleotide repeat in said gene of interest;
   and wherein said second engineered nuclease recognizes and cleaves a second recognition sequence positioned 3' downstream of said nucleotide repeat in said gene of interest.
31. The pharmaceutical composition of item 30, wherein said first engineered nuclease and said second engineered nuclease generate complementary overhangs which promote direct re-ligation of said gene of interest.
32. The pharmaceutical composition of item 30 or item 31, wherein said first recognition sequence and said second recognition sequence are positioned within the same exon, the same intron, or the same untranslated region (UTR) as said nucleotide repeat.
33. The pharmaceutical composition of any one of items 30-32, wherein said first nucleic acid and/or said second nucleic acid is an mRNA.
34. The pharmaceutical composition of any one of items 30-33, wherein said pharmaceutical composition comprises a first recombinant DNA construct comprising said first nucleic acid and/or a second recombinant DNA construct comprising said second nucleic acid.
35. The pharmaceutical composition of any one of items 30-33, wherein said pharmaceutical composition comprises a first viral vector comprising said first nucleic acid and/or a second viral vector comprising said second nucleic acid.
36. The pharmaceutical composition of item 35, wherein said first viral vector and/or said second viral vector is a recombinant AAV vector.
37. The pharmaceutical composition of any one of items 30-36, wherein said first engineered nuclease and/or said second engineered nuclease is an engineered meganuclease, a TALEN, a zinc finger nuclease, a compact TALEN, a CRISPR, or a megaTAL.
38. The pharmaceutical composition of any one of items 30-37, wherein said first engineered nuclease is a first engineered meganuclease and said second engineered nuclease is a second engineered meganuclease.
39. The pharmaceutical composition of item 38, wherein said first recognition sequence comprises any one of SEQ ID NOs: 12-73.
40. The pharmaceutical composition of item 38 or item 39, wherein said first recognition sequence comprises SEQ ID NO: 34.
41. The pharmaceutical composition of item 40, wherein said first engineered meganuclease is said first engineered meganuclease of any one of items 13-19.
42. The pharmaceutical composition of any one of items 38-41, wherein said first engineered meganuclease comprises any one of SEQ ID NOs: 155-159.
43. The pharmaceutical composition of any one of items 38-42, wherein said second recognition sequence comprises any one of SEQ ID NOs: 75-95.
44. The pharmaceutical composition of any one of items 38-43, wherein said second recognition sequence comprises SEQ ID NO: 89.
45. The pharmaceutical composition of item 44, wherein said second engineered meganuclease is said second engineered meganuclease of any one of items 22-28.
46. The pharmaceutical composition of any one of items 38-45, wherein said second engineered meganuclease comprises any one of SEQ ID NOs: 170-173.
47. The pharmaceutical composition of any one of items 38-46, wherein said first recognition sequence comprises SEQ ID NO: 34 and said second recognition sequence comprises SEQ ID NO: 89.
48. An engineered meganuclease that recognizes and cleaves a recognition sequence within intron 1 of the frataxin (*FXN*) gene, wherein said engineered meganuclease comprises a first subunit and a second subunit, wherein said first subunit binds to a first recognition half-site of said recognition sequence and comprises a first hypervariable (HVR1) region, and wherein said second subunit binds to a second recognition half-site of said recognition sequence and comprises a second hypervariable (HVR2) region.
49. The engineered meganuclease of item 48, wherein said recognition sequence comprises SEQ ID NO: 34.
50. The engineered meganuclease of item 49, wherein said first subunit comprises an amino acid sequence having at least 80% sequence identity to residues 7-153 of any one of SEQ ID NOs: 155-159, and wherein said second subunit comprises an amino acid sequence having at least 80% sequence identity to residues 198-344 of any one of SEQ ID NOs: 155-159.
51. The engineered meganuclease of item 49 or item 50, wherein said HVR1 region comprises residues 24-79 of any one of SEQ ID NOs: 155-159.
52. The engineered meganuclease of any one of items 49-51, wherein said HVR2 region comprises residues 215-270 of any one of SEQ ID NOs: 155-159.
53. The engineered meganuclease of any one of items 49-52, wherein said first subunit comprises residues 7-153 of any one of SEQ ID NOs: 155-159.
54. The engineered meganuclease of any one of items 49-53, wherein said second subunit comprises residues 198-344 of any one of SEQ ID NOs: 155-159.
55. The engineered meganuclease of any one of items 49-54, wherein said engineered meganuclease is a single-chain meganuclease comprising a linker, wherein said linker covalently joins said first subunit and said second subunit.
56. The engineered meganuclease of any one of items 49-55, wherein said engineered meganuclease comprises the amino acid sequence of any one of SEQ ID NOs: 155-159.
57. The engineered meganuclease of item 48, wherein said recognition sequence comprises SEQ ID NO: 89.
58. The engineered meganuclease of item 57, wherein said first subunit comprises an amino acid sequence having at least 80% sequence identity to residues 198-344 of any one of SEQ ID NOs: 170-172, or residues 7-153 of SEQ ID NO: 173, and wherein said second subunit comprises an amino acid sequence having at least 80% sequence identity to residues 7-153 of any one of SEQ ID NOs: 170-172, or residues 198-344 of SEQ ID NO: 173.
59. The engineered meganuclease of item 57 or item 58, wherein said HVR1 region comprises residues 215-270 of any one of SEQ ID NOs: 170-172, or residues 24-79 of SEQ ID NO: 173.
60. The engineered meganuclease of any one of items 57-59, wherein said HVR2 region comprises residues 24-79 of any one of SEQ ID NOs: 170-172, or residues 215-270 of SEQ ID NO: 173.
61. The engineered meganuclease of any one of items 57-60, wherein said first subunit comprises residues 198-344 of any one of SEQ ID NOs: 170-172, or residues 7-153 of SEQ ID NO: 173.
62. The engineered meganuclease of any one of items 57-61, wherein said second subunit comprises residues 7-153 of any one of SEQ ID NOs: 170-172, or residues 198-344 of SEQ ID NO: 173.
63. The engineered meganuclease of any one of items 57-62, wherein said engineered meganuclease is a single-chain meganuclease comprising a linker, wherein said linker covalently joins said first subunit and said second subunit.
64. The engineered meganuclease of any one of items 57-63, wherein said engineered meganuclease comprises the amino acid sequence of any one of SEQ ID NOs: 170-173.
65. An isolated polynucleotide comprising a nucleic acid sequence encoding said engineered meganuclease of any one of items 48-64.
66. The isolated polynucleotide of item 65, wherein said isolated polynucleotide is an mRNA.
67. A recombinant DNA construct comprising said nucleic acid sequence of item 65.
68. The recombinant DNA construct of item 67, wherein said recombinant DNA construct encodes a viral vector.
69. The recombinant DNA construct of item 68, wherein said viral vector is a recombinant adeno-associated virus (AAV) vector.
70. A viral vector comprising said nucleic acid sequence of item 65.
71. The viral vector of item 70, wherein said viral vector is a recombinant AAV vector.
72. A method for promoting precise deletion of a locus flanked by a pair of direct repeat sequences in a chromosome in a population of eukaryotic cells, the method comprising:
   (a) introducing into the cells a first engineered nuclease protein and a second engineered nuclease protein; or
   (b) introducing into the cells at least a first nucleic acid encoding the first engineered nuclease and a second nucleic acid encoding the second engineered nuclease, wherein the first engineered nuclease and the second engineered nuclease are expressed in the cells *in vivo*;

   wherein the pair of direct repeat sequences comprises a first direct repeat sequence 5' of the locus on a first DNA strand of the chromosome and a second direct repeat sequence 3' of the locus on the first DNA strand of the chromosome, and each of the first direct repeat sequence and the second direct repeat sequence consists of the same nucleotide sequence of 2-4 basepairs;
   wherein the first engineered nuclease recognizes the first direct repeat sequence and cleaves the first strand of the chromosome at either the 3' or 5' end of the first direct repeat sequence on the first strand, and cleaves the second strand of the chromosome at either the 3' or 5' end of the first direct repeat sequence on the second strand, thereby producing either a first 3' or 5' overhang;
   wherein the second engineered nuclease recognizes the second direct repeat sequence and cleaves the first strand of the chromosome at either the 3' or 5' end of the second direct repeat sequence on the first strand, and cleaves the second strand of the chromosome at either the 3' or 5' end of the second direct repeat sequence on the second strand, thereby producing either a second 3' or 5' overhang which is complementary to the first 3' or 5' overhang; and
   wherein re-ligation of the first 3' or 5' overhang and the second 3' or 5' overhang promotes a precise deletion of the locus between the pair of direct repeats.
73. The method of item 72, wherein the engineered nuclease is an engineered meganuclease, single chain meganuclease, compact TALEN, CRISPR, or zinc finger nuclease.
74. The method of item 72, wherein the engineered nuclease is an engineered LAGLIDADG meganuclease or single chain LAGLIDADG meganuclease and cleavage of the chromosome produces a 4 basepair 3' overhang.
75. The method of item 72, wherein the engineered nuclease is an engineered GIY-YIG meganuclease and cleavage of the chromosome produces a 2 basepair 3' overhang.
76. The method of item 72, wherein the engineered nuclease is an engineered compact TALEN and cleavage of the chromosome produces a 2 basepair 3' overhang.
77 The method of item 72, wherein the engineered nuclease is an engineered CRISPR and cleavage of the chromosome produces a 2-4 basepair 5' overhang.
78. The method of item 73, wherein re-ligation of the first 3' or 5' overhang and the second 3' or 5' overhang promotes a precise deletion of the locus between the pair of direct repeats in at least 20% of the population of cells.
79. The method of item 73, wherein re-ligation of the first 3' or 5' overhang and the second 3' or 5' overhang promotes a precise deletion of the locus between the pair of direct repeats in at least 25% of the population of cells.
80. The method of item 73, wherein re-ligation of the first 3' or 5' overhang and the second 3' or 5' overhang promotes a precise deletion of the locus between the pair of direct repeats in at least 30% of the population of cells.
81. The method of item 73, wherein re-ligation of the first 3' or 5' overhang and the second 3' or 5' overhang promotes a precise deletion of the locus between the pair of direct repeats in at least 35% of the population of cells.
82. The method of item 73, wherein re-ligation of the first 3' or 5' overhang and the second 3' or 5' overhang promotes a precise deletion of the locus between the pair of direct repeats in at least 40% of the population of cells.

## Claims

1. A composition for use in treating a subject having a nucleotide repeat expansion disorder, wherein said nucleotide repeat expansion disorder is **characterized by** expansion of a nucleotide repeat in a gene of interest, said composition comprising:
at least a first nucleic acid encoding a first engineered nuclease and a second nucleic acid encoding a second engineered nuclease, wherein said first engineered nuclease and said second engineered nuclease are expressible in target cells *in vivo*;
wherein said first engineered nuclease recognizes and cleaves a first recognition sequence positioned 5' upstream of said nucleotide repeat in said gene of interest;
and wherein said second engineered nuclease recognizes and cleaves a second recognition sequence positioned 3' downstream of said nucleotide repeat in said gene of interest;
and wherein an intervening DNA fragment between said first recognition sequence and said second recognition sequence is excised and the number of said nucleotide repeat is reduced in said gene of interest.

2. The composition for the use of claim 1, wherein said first engineered nuclease and/or said second engineered nuclease is an engineered meganuclease, a compact TALEN, or a CRISPR.

3. The composition for the use of claim 1 or claim 2, wherein said first recognition sequence and said second recognition sequence are positioned within the same exon, the same intron, or the same untranslated region (UTR) as said nucleotide repeat.

4. The composition for the use of any one of claims 1-3 wherein said nucleotide repeat is a hexanucleotide repeat.

5. The composition for the use of claim 4, wherein said hexanucleotide repeat is GGGGCC and said gene of interest is the *C9ORF72* gene, wherein said hexanucleotide repeat is positioned within intron 1 of the *C9ORF72* gene.

6. The composition for the use of claim 4 or claim 5, wherein said first recognition sequence is positioned 5' upstream of a hexanucleotide repeat positioned within intron 1 of the *C9ORF72* gene.

7. The composition for the use of any one of claims 4-6, wherein said second recognition sequence is positioned 3' downstream of said hexanucleotide repeat in said intron 1.

8. The composition for the use of any one of claims 1-3, wherein said nucleotide repeat is a trinucleotide repeat, preferably wherein said trinucleotide repeat is selected from the group consisting of CAG, CGG, CCG, GAA, and CTG.

9. The composition for the use of claim 8, wherein said trinucleotide repeat is GAA and said gene of interest is the frataxin (*FXN*) gene, wherein said trinucleotide repeat is positioned within intron 1 of the *FXN* gene.

10. The composition for the use of claim 9, wherein said first recognition sequence is positioned 5' upstream of said trinucleotide repeat in said intron 1 (SEQ ID NO: 74), and/or wherein said second recognition sequence is positioned 3' downstream of said trinucleotide repeat in said intron 1 (SEQ ID NO: 96).

11. The composition for the use of claim 9 or claim 10, wherein said first recognition sequence comprises any one of SEQ ID NOs: 13-15, 19, 26-28, 30, 31, 33, 34, 40, 42, 43, 45, 46, 48, 49, 54, 55, 57, 58, 60, 62, 63, 65, 67, 71, and 73, preferably wherein said first recognition sequence comprises SEQ ID NO: 34.

12. The composition for the use of any one of claims 9-11, wherein said second recognition sequence comprises any one of SEQ ID NOs: 76 and 78-95, preferably wherein said second recognition sequence comprises SEQ ID NO: 89.

13. The composition for the use of claim 11, wherein said first recognition sequence comprises SEQ ID NO: 34, and wherein said first engineered nuclease is an engineered meganuclease that comprises a first subunit and a second subunit, wherein:
(a) said first subunit binds to a first recognition half-site of said first recognition sequence, and wherein said first subunit comprises an amino acid sequence having at least 80% sequence identity to residues 7-153 of any one of SEQ ID NOs: 155-159 and comprises a first hypervariable (HVR1) region which comprises residues 24-79 of any one of SEQ ID NOs: 155-159; and
(b) said second subunit binds to a second recognition half-site of said first recognition sequence, and wherein said second subunit comprises an amino acid sequence having at least 80% sequence identity to residues 198-344 of any one of SEQ ID NOs: 155-159 and comprises a second hypervariable (HVR2) region which comprises residues 215-270 of any one of SEQ ID NOs: 155-159.

14. The composition for the use of claim 12, wherein said second recognition sequence comprises SEQ ID NO: 89, and wherein said second engineered nuclease is an engineered meganuclease that comprises a first subunit and a second subunit, wherein:
(a) said first subunit binds to a first recognition half-site of said second recognition sequence, and wherein said first subunit comprises an amino acid sequence having at least 80% sequence identity to residues 198-344 of any one of SEQ ID NOs: 170-172, or residues 7-153 of SEQ ID NO: 173 and comprises a first hypervariable (HVR1) region which comprises residues 215-270 of any one of SEQ ID NOs: 170-172, or residues 24-79 of SEQ ID NO: 173; and
(b) wherein said second subunit binds to a second recognition half-site of said second recognition sequence, and wherein said second subunit comprises an amino acid sequence having at least 80% sequence identity to residues 7-153 of any one of SEQ ID NOs: 170-172, or residues 198-344 of SEQ ID NO: 173 and comprises a second hypervariable (HVR2) region which comprises residues 24-79 of any one of SEQ ID NOs: 170-172, or residues 215-270 of SEQ ID NO: 173.

15. The composition for the use of any one of claims 1-14, wherein said first engineered nuclease is a first engineered meganuclease and said second engineered nuclease is a second engineered meganuclease.
